(19) Europäisches Patentamt
European Patent Office
Office européen des brevets

(11) EP 4 467 154 A1

(12) EUROPEAN PATENT APPLICATION
published in accordance with Art. 153(4) EPC

(43) Date of publication:
**27.11.2024 Bulletin 2024/48**

(21) Application number: **23743322.2**

(22) Date of filing: **19.01.2023**

(51) International Patent Classification (IPC):
***A61K 38/12*** *(2006.01)* ***A61K 31/4184*** *(2006.01)*
***A61K 31/44*** *(2006.01)* ***A61K 31/47*** *(2006.01)*
***A61K 31/497*** *(2006.01)* ***A61K 31/506*** *(2006.01)*
***A61K 31/517*** *(2006.01)* ***A61K 31/519*** *(2006.01)*
***A61K 31/5377*** *(2006.01)* ***A61K 39/395*** *(2006.01)*
***A61K 45/00*** *(2006.01)* ***A61P 35/00*** *(2006.01)*
***A61P 35/02*** *(2006.01)* ***A61P 43/00*** *(2006.01)*
***C07K 7/02*** *(2006.01)* ***C07K 16/28*** *(2006.01)*

(52) Cooperative Patent Classification (CPC):
**A61K 31/4184; A61K 31/44; A61K 31/47; A61K 31/497; A61K 31/506; A61K 31/517; A61K 31/519; A61K 31/5377; A61K 38/12; A61K 39/395; A61K 45/00; A61P 35/00; A61P 35/02; A61P 43/00; C07K 7/02;** (Cont.)

(86) International application number:
**PCT/JP2023/001552**

(87) International publication number:
**WO 2023/140329 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.01.2022 JP 2022008247**
**19.09.2022 EP 22196417**

(71) Applicant: **CHUGAI SEIYAKU KABUSHIKI KAISHA**
**Tokyo 115-8543 (JP)**

(72) Inventors:
- **SASE Hitoshi**
  **Kamakura-shi, Kanagawa 247-8530 (JP)**
- **HIRANO Saki**
  **Kamakura-shi, Kanagawa 247-8530 (JP)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

(54) **MEDICINE FOR TREATING OR PREVENTING CANCER**

(57) A medicine for treating or preventing cancer, the medicine comprising a first active ingredient and being used in combination with a second active ingredient, wherein the first active ingredient is a compound represented by the following formula (1) or a salt thereof, or a solvate thereof, and the second active ingredient is a molecular-targeted agent:

EP 4 467 154 A1

[Formula 1]

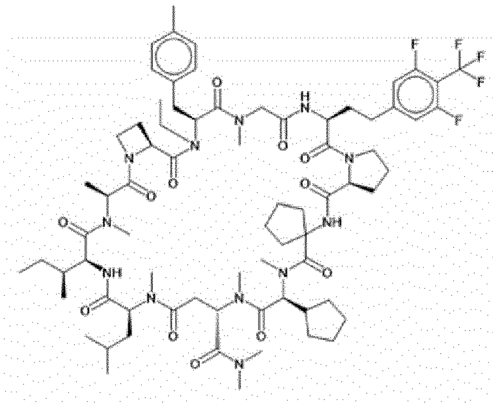

(1)

*Fig.1*

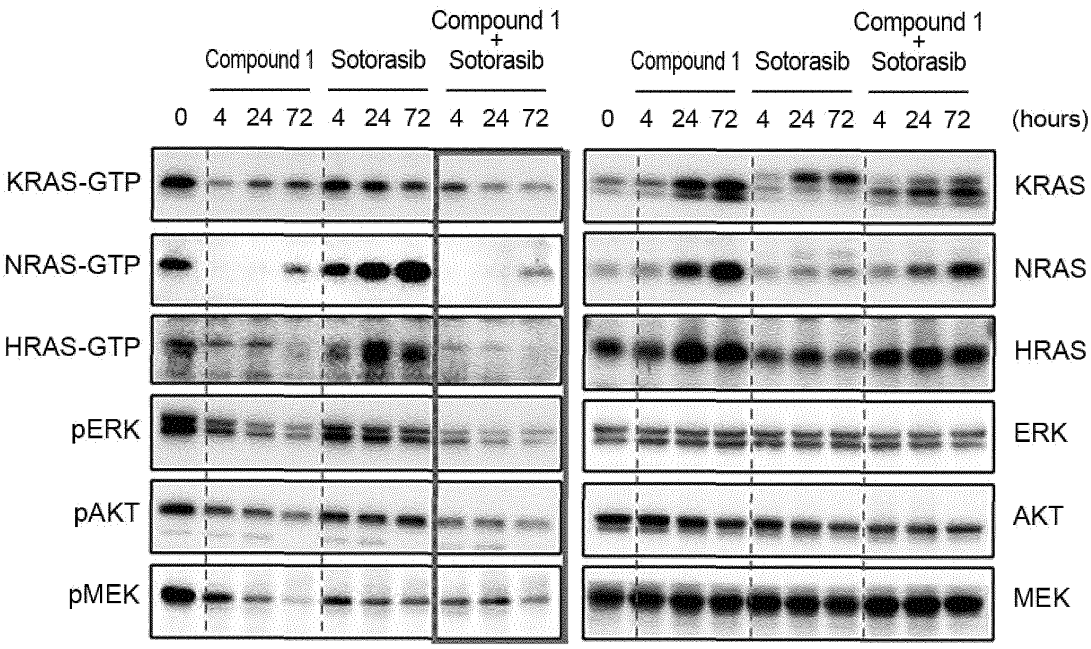


(52) Cooperative Patent Classification (CPC): (Cont.)
**C07K 16/28**

## Description

Technical Field

The present invention relates to a medicine for treating or preventing cancer.

Background Art

Cancer is a disease in which cells undergo uncontrolled proliferation due to the occurrence of abnormalities in genes. Chemotherapy is a medical treatment method for cancer, and a variety of anticancer agents have been used. In recent years, a large number of molecular-targeted agents that specifically act on molecules specifically expressed in certain cancer cells or molecules whose expression is enhanced in cancer cells have been developed as anticancer agents. Known molecular-targeted agents include antibody drugs such as cetuximab, bevacizumab, and panitumumab (Patent Literature 1) and small-molecule drugs such as gefitinib, erlotinib, and afatinib (Patent Literature 2). Furthermore, in recent years, middle-molecule drugs are also known (Patent Literature 3).

Citation List

Patent Literature

Patent Literature 1: Japanese Unexamined Patent Publication No. 2018-076369
Patent Literature 2: Japanese Unexamined Patent Publication No. 2021-063014
Patent Literature 3: International Publication No. WO 2021/090855

Summary of Invention

Technical Problem

Under these circumstances, a medicine with even better efficacy in the treatment or prevention of cancer has been in demand.
Therefore, an object of the present invention is to provide a medicine that exhibits an improved effect in the treatment or prevention of cancer as compared with the prior art.

Solution to Problem

The present invention encompasses the following [1] to [94]:

[1] A medicine for treating or preventing cancer, the medicine comprising a first active ingredient and being used in combination with a second active ingredient, wherein the first active ingredient is a compound represented by the following formula (1) (hereinafter, also referred to as "compound 1") or a salt thereof, or a solvate thereof, and the second active ingredient is a molecular-targeted agent.

[Formula 1]

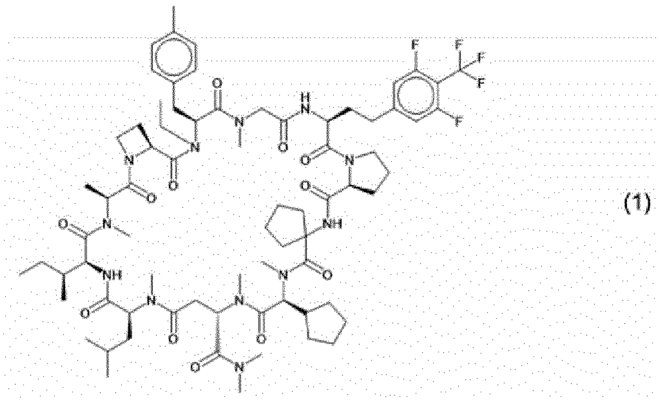

(1)

[2] A medicine for treating or preventing cancer, the medicine comprising a first active ingredient and being used in combination with a second active ingredient, wherein the first active ingredient is a molecular-targeted agent, and the second active ingredient is compound 1 or a salt thereof, or a solvate thereof.
[3] A medicine for treating or preventing cancer, the medicine comprising a first active ingredient and being used in combination with a second active ingredient, wherein the first active ingredient is compound 1 or a salt thereof, or a solvate thereof, and the second active ingredient is a MAPK/ERK-pathway inhibitor.
[4] A medicine for treating or preventing cancer, the medicine comprising a first active ingredient and being used in combination with a second active ingredient, wherein the first active ingredient is a MAPK/ERK-pathway inhibitor, and the second active ingredient is compound 1 or a salt thereof, or a solvate thereof.
[5] A medicine for treating or preventing cancer, the medicine comprising a first active ingredient and being used in combination with a second active ingredient, wherein the first active ingredient is compound 1 or a salt thereof, or a solvate thereof, and the second active ingredient is one selected from the group consisting of an EGFR inhibitor, a VEGF inhibitor, an SHP2 inhibitor, a KRAS-G12C inhibitor, and a MEK inhibitor.
[6] A medicine for treating or preventing cancer, the medicine comprising a first active ingredient and being used in combination with a second active ingredient, wherein the first active ingredient is one selected from the group consisting of an EGFR inhibitor, a VEGF inhibitor, an SHP2 inhibitor, a KRAS-G12C inhibitor, and a MEK inhibitor, and the second active ingredient is compound 1 or a salt thereof, or a solvate thereof.
[7] The medicine according to any one of [1] to [6], wherein the first active ingredient and the second active ingredient are provided as a kit.
[8] A method for treating or preventing cancer, the method comprising administering a first active ingredient and a second active ingredient to a subject, wherein the first active ingredient is compound 1 or a salt thereof, or a solvate thereof, and the second active ingredient is a molecular-targeted agent.
[9] A method for treating or preventing cancer, the method comprising administering a first active ingredient and a second active ingredient to a subject, wherein the first active ingredient is a molecular-targeted agent, and the second active ingredient is compound 1 or a salt thereof, or a solvate thereof.
[10] A method for treating or preventing cancer, the method comprising administering a first active ingredient and a second active ingredient to a subject, wherein the first active ingredient is compound 1 or a salt thereof, or a solvate thereof, and the second active ingredient is a MAPK/ERK-pathway inhibitor.
[11] A method for treating or preventing cancer, the method comprising administering a first active ingredient and a second active ingredient to a subject, wherein the first active ingredient is a MAPK/ERK-pathway inhibitor, and the second active ingredient is compound 1 or a salt thereof, or a solvate thereof.
[12] A method for treating or preventing cancer, the method comprising administering a first active ingredient and a second active ingredient to a subject, wherein the first active ingredient is compound 1 or a salt thereof, or a solvate thereof, and the second active ingredient is one selected from the group consisting of an EGFR inhibitor, a VEGF inhibitor, an SHP2 inhibitor, a KRAS-G12C inhibitor, and a MEK inhibitor.
[13] A method for treating or preventing cancer, the method comprising administering a first active ingredient and a second active ingredient to a subject, wherein the first active ingredient is one selected from the group consisting of an EGFR inhibitor, a VEGF inhibitor, an SHP2 inhibitor, a KRAS-G12C inhibitor, and a MEK inhibitor, and the second active ingredient is compound 1 or a salt thereof, or a solvate thereof.
[14] A first active ingredient for use in treatment or prevention of cancer, the first active ingredient being used in combination with a second active ingredient, wherein the first active ingredient is compound 1 or a salt thereof, or a solvate thereof, and the second active ingredient is a molecular-targeted agent.

[15] A first active ingredient for use in treatment or prevention of cancer, the first active ingredient being used in combination with a second active ingredient, wherein the first active ingredient is a molecular-targeted agent, and the second active ingredient is compound 1 or a salt thereof, or a solvate thereof.
[16] A first active ingredient for use in treatment or prevention of cancer, the first active ingredient being used in combination with a second active ingredient, wherein the first active ingredient is compound 1 or a salt thereof, or a solvate thereof, and the second active ingredient is a MAPK/ERK-pathway inhibitor.
[17] A first active ingredient for use in treatment or prevention of cancer, the first active ingredient being used in combination with a second active ingredient, wherein the first active ingredient is a MAPK/ERK-pathway inhibitor, and the second active ingredient is compound 1 or a salt thereof, or a solvate thereof.
[18] A first active ingredient for use in treatment or prevention of cancer, the first active ingredient being used in combination with a second active ingredient, wherein the first active ingredient is compound 1 or a salt thereof, or a solvate thereof, and the second active ingredient is one selected from the group consisting of an EGFR inhibitor, a VEGF inhibitor, an SHP2 inhibitor, a KRAS-G12C inhibitor, and a MEK inhibitor.
[19] A first active ingredient for use in treatment or prevention of cancer, the first active ingredient being used in combination with a second active ingredient, wherein the first active ingredient is one selected from the group consisting of an EGFR inhibitor, a VEGF inhibitor, an SHP2 inhibitor, a KRAS-G12C inhibitor, and a MEK inhibitor, and the second active ingredient is compound 1 or a salt thereof, or a solvate thereof.
[20] Use of a first active ingredient for production of a medicine for treating or preventing cancer, the first active ingredient being used in combination with a second active ingredient, wherein the first active ingredient is compound 1 or a salt thereof, or a solvate thereof, and the second active ingredient is a molecular-targeted agent.
[21] Use of a first active ingredient for production of a medicine for treating or preventing cancer, the first active ingredient being used in combination with a second active ingredient, wherein the first active ingredient is a molecular-targeted agent, and the second active ingredient is compound 1 or a salt thereof, or a solvate thereof.
[22] Use of a first active ingredient for production of a medicine for treating or preventing cancer, the first active ingredient being used in combination with a second active ingredient, wherein the first active ingredient is compound 1 or a salt thereof, or a solvate thereof, and the second active ingredient is a MAPK/ERK-pathway inhibitor.
[23] Use of a first active ingredient for production of a medicine for treating or preventing cancer, the first active ingredient being used in combination with a second active ingredient, wherein the first active ingredient is a MAPK/ERK-pathway inhibitor, and the second active ingredient is compound 1 or a salt thereof, or a solvate thereof.
[24] Use of a first active ingredient for production of a medicine for treating or preventing cancer, the first active ingredient being used in combination with a second active ingredient, wherein the first active ingredient is compound 1 or a salt thereof, or a solvate thereof, and the second active ingredient is one selected from the group consisting of an EGFR inhibitor, a VEGF inhibitor, an SHP2 inhibitor, a KRAS-G12C inhibitor, and a MEK inhibitor.
[25] Use of a first active ingredient for production of a medicine for treating or preventing cancer, the first active ingredient being used in combination with a second active ingredient, wherein the first active ingredient is one selected from the group consisting of an EGFR inhibitor, a VEGF inhibitor, an SHP2 inhibitor, a KRAS-G12C inhibitor, and a MEK inhibitor, and the second active ingredient is compound 1 or a salt thereof, or a solvate thereof.
[26] The medicine, method, first active ingredient for use, or use according to any one of [1] to [25], wherein the combined use of the first active ingredient and the second active ingredient is simultaneous administration or separate administration (i.e., the first active ingredient and the second active ingredient are used in combination simultaneously or separately).
[27] The medicine, method, first active ingredient for use, or use according to any one of [1] to [26], wherein the first active ingredient and the second active ingredient are used in combination as a combination drug.
[28] The medicine, method, first active ingredient for use, or use according to any one of [1], [2], [7], [8], [9], [14], [15], [20], [21], [26] and [27], wherein the molecular-targeted agent is a MAPK/ERK-pathway inhibitor.
[29] The medicine, method, first active ingredient for use, or use according to any one of [1], [2], [7], [8], [9], [14], [15], [20], [21], [26], [27], and [28], wherein the molecular-targeted agent is at least one selected from the group consisting of an EGFR inhibitor, a VEGF inhibitor, an SHP2 inhibitor, a KRAS-G12C inhibitor, and a MEK inhibitor.
[30] The medicine, method, first active ingredient for use, or use according to any one of [1], [2], [7], [8], [9], [14], [15], [20], [21], [26], [27], [28], and [29], wherein the molecular-targeted agent is an EGFR inhibitor, a VEGF inhibitor, an SHP2 inhibitor, a KRAS-G12C inhibitor, or a MEK inhibitor.
[31] The medicine, method, first active ingredient for use, or use according to any one of [1], [2], [7], [8], [9], [14], [15], [20], [21], [26], [27], [28], [29], and [30], wherein the molecular-targeted agent is an EGFR inhibitor.
[32] The medicine, method, first active ingredient for use, or use according to any one of [1], [2], [7], [8], [9], [14], [15], [20], [21], [26], [27], [28], [29], and [30], wherein the molecular-targeted agent is a VEGF inhibitor.
[33] The medicine, method, first active ingredient for use, or use according to any one of [1], [2], [7], [8], [9], [14], [15], [20], [21], [26], [27], [28], [29], and [30], wherein the molecular-targeted agent is an SHP2 inhibitor.
[34] The medicine, method, first active ingredient for use, or use according to any one of [1], [2], [7], [8], [9], [14], [15],

[20], [21], [26], [27], [28], [29], and [30], wherein the molecular-targeted agent is a KRAS-G12C inhibitor.
[35] The medicine, method, first active ingredient for use, or use according to any one of [1], [2], [7], [8], [9], [14], [15], [20], [21], [26], [27], [28], [29], and [30], wherein the molecular-targeted agent is a MEK inhibitor.
[36] The medicine, method, first active ingredient for use, or use according to any one of [5], [6], [7], [12], [13], [18], [19], [24], [25], [26], [27], [29], [30], and [31], wherein the EGFR inhibitor is at least one selected from the group consisting of gefitinib, erlotinib, afatinib, osimertinib, lapatinib, or a salt thereof, or a solvate thereof, and an anti-EGFR antibody.
[37] The medicine, method, first active ingredient for use, or use according to any one of [5], [6], [7], [12], [13], [18], [19], [24], [25], [26], [27], [29], [30], [31], and [36], wherein the EGFR inhibitor is an anti-EGFR antibody.
[38] The medicine, method, first active ingredient for use, or use according to [36] or [37], wherein the anti-EGFR antibody has a heavy chain variable region comprising an amino acid sequence of SEQ ID NO: 1 and a light chain variable region comprising an amino acid sequence of SEQ ID NO: 2.
[39] The medicine, method, first active ingredient for use, or use according to any one of [36] to [38], wherein the anti-EGFR antibody is cetuximab.
[40] The medicine, method, first active ingredient for use, or use according to any one of [5], [6], [7], [12], [13], [18], [19], [24], [25], [26], [27], and [29] to [39], wherein the VEGF inhibitor is at least one selected from the group consisting of sorafenib, pazopanib, sunitinib, axitinib, regorafenib, or a salt thereof, or a solvate thereof, and an anti-VEGF antibody.
[41] The medicine, method, first active ingredient for use, or use according to any one of [5], [6], [7], [12], [13], [18], [19], [24], [25], [26], [27], and [29] to [40], wherein the VEGF inhibitor is an anti-VEGF antibody.
[42] The medicine, method, first active ingredient for use, or use according to [40] or [41], wherein the anti-VEGF antibody has a heavy chain variable region comprising an amino acid sequence of SEQ ID NO: 3 and a light chain variable region comprising an amino acid sequence of SEQ ID NO: 4.
[43] The medicine, method, first active ingredient for use, or use according to any one of [40] to [42], wherein the anti-VEGF antibody is bevacizumab.
[44] The medicine, method, first active ingredient for use, or use according to any one of [5], [6], [7], [12], [13], [18], [19], [24], [25], [26], [27], and [29] to [43], wherein the SHP2 inhibitor is at least one selected from the group consisting of RMC4550, TNO155, RLY1971, SHP099, NSC-87877, and a salt thereof, and a solvate thereof.
[45] The medicine, method, first active ingredient for use, or use according to any one of [5], [6], [7], [12], [13], [18], [19], [24], [25], [26], [27], and [29] to [44], wherein the SHP2 inhibitor is at least one selected from the group consisting of RMC4550, TNO155, and a salt thereof, and a solvate thereof.
[46] The medicine, method, first active ingredient for use, or use according to any one of [5], [6], [7], [12], [13], [18], [19], [24], [25], [26], [27], and [29] to [45], wherein the SHP2 inhibitor is RMC4550 or a salt thereof, or a solvate thereof.
[46-1] The medicine, method, first active ingredient for use, or use according to any one of [5], [6], [7], [12], [13], [18], [19], [24], [25], [26], [27], and [29] to [45], wherein the SHP2 inhibitor is TNO155 or a salt thereof, or a solvate thereof.
[47] The medicine, method, first active ingredient for use, or use according to any one of [5], [6], [7], [12], [13], [18], [19], [24], [25], [26], [27], and [29] to [46], wherein the KRAS-G12C inhibitor is at least one selected from the group consisting of sotorasib, MRTX849, and a salt thereof, and a solvate thereof.
[48] The medicine, method, first active ingredient for use, or use according to any one of [5], [6], [7], [12], [13], [18], [19], [24], [25], [26], [27], and [29] to [47], wherein the KRAS-G12C inhibitor is sotorasib.
[48-1] The medicine, method, first active ingredient for use, or use according to any one of [5], [6], [7], [12], [13], [18], [19], [24], [25], [26], [27], and [29] to [47], wherein the KRAS-G12C inhibitor is MRTX849.
[49] The medicine, method, first active ingredient for use, or use according to any one of [5], [6], [7], [12], [13], [18], [19], [24], [25], [26], [27], and [29] to [47], wherein the KRAS-G12C inhibitor is sotorasib or a salt thereof, or a solvate thereof.
[49-1] The medicine, method, first active ingredient for use, or use according to any one of [5], [6], [7], [12], [13], [18], [19], [24], [25], [26], [27], and [29] to [47], wherein the KRAS-G12C inhibitor is MRTX849 or a salt thereof, or a solvate thereof.
[50] The medicine, method, first active ingredient for use, or use according to any one of [5], [6], [7], [12], [13], [18], [19], [24], [25], [26], [27], and [29] to [49], wherein the MEK inhibitor is at least one selected from the group consisting of a compound represented by the following formula (2), trametinib, selumetinib, CH4987655, and a salt thereof, and a solvate thereof.

[Formula 2]

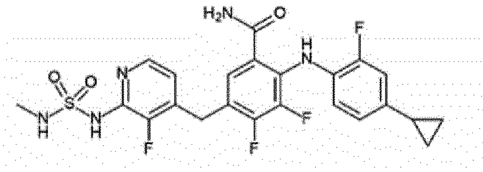

(2)

[51] The medicine, method, first active ingredient for use, or use according to any one of [5], [6], [7], [12], [13], [18], [19], [24], [25], [26], [27], and [29] to [50], wherein the MEK inhibitor is a compound represented by the following formula (2), trametinib, or a salt thereof, or a solvate thereof.

[Formula 3]

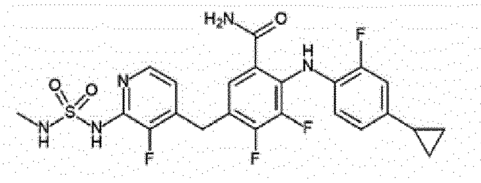

(2)

[52] The medicine, method, first active ingredient for use, or use according to any one of [5], [6], [7], [12], [13], [18], [19], [24], [25], [26], [27], and [29] to [51], wherein the MEK inhibitor is a compound represented by the following formula (2) or a salt thereof, or a solvate thereof.
[53] The medicine, method, first active ingredient for use, or use according to any one of [5], [6], [7], [12], [13], [18], [19], [24], [25], [26], [27], and [29] to [51], wherein the MEK inhibitor is trametinib or a salt thereof, or a solvate thereof.
[54] The medicine, method, first active ingredient for use, or use according to any one of [5], [6], [7], [12], [13], [18], [19], [24], [25], [26], [27], [29] to [51], and [53], wherein the MEK inhibitor is trametinib dimethyl sulfoxide.
[55] The medicine, method, first active ingredient for use, or use according to any one of [1] to [54], wherein compound 1 or the salt thereof, or the solvate thereof is a solvate of compound 1.
[56] The medicine, method, first active ingredient for use, or use according to [55], wherein the solvate is a hydrate.
[57] The medicine, method, first active ingredient for use, or use according to any one of [1] to [54], wherein compound 1 or the salt thereof, or the solvate thereof is compound 1.
[58] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the EGFR inhibitor is cetuximab.
[59] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the VEGF inhibitor is an anti-VEGF antibody.
[60] The medicine, method, first active ingredient for use, or use according to [59], wherein the anti-VEGF antibody is bevacizumab.
[61] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the SHP2 inhibitor is RMC4550 or a salt thereof, or a solvate thereof.
[61-1] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the SHP2 inhibitor is TNO155 or a salt thereof, or a solvate thereof.
[62] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the SHP2 inhibitor is RLY1971 or a salt thereof, or a solvate thereof.
[63] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the KRAS-G12C inhibitor is sotorasib or a salt thereof, or a solvate thereof.
[63-1] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the KRAS-G12C inhibitor is MRTX849 or a salt thereof, or a solvate thereof.
[64] The medicine, method, first active ingredient for use, or use according to any one of [1] to [25], wherein compound 1 or the salt thereof, or the solvate thereof is a hydrate of compound 1, and the molecular-targeted agent, the

MAPK/ERK-pathway inhibitor, or the MEK inhibitor is a compound represented by the following formula (2) or a salt thereof, or a solvate thereof.

[65] The medicine, method, first active ingredient for use, or use according to any one of [1] to [25], wherein compound 1 or the salt thereof, or the solvate thereof is a hydrate of compound 1, and the molecular-targeted agent, the MAPK/ERK-pathway inhibitor, or the MEK inhibitor is trametinib or a salt thereof, or a solvate thereof.

[66] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the EGFR inhibitor is cetuximab.

[67] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the VEGF inhibitor is an anti-VEGF antibody.

[68] The medicine, method, first active ingredient for use, or use according to [67], wherein the anti-VEGF antibody is bevacizumab.

[69] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the SHP2 inhibitor is RMC4550 or a salt thereof, or a solvate thereof.

[69-1] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the SHP2 inhibitor is TNO155 or a salt thereof, or a solvate thereof.

[70] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the SHP2 inhibitor is RLY1971 or a salt thereof, or a solvate thereof.

[71] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the KRAS-G12C inhibitor is sotorasib or a salt thereof, or a solvate thereof.

[71-1] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the KRAS-G12C inhibitor is MRTX849 or a salt thereof, or a solvate thereof.

[72] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the MEK inhibitor is a compound represented by formula (2) or a salt thereof, or a solvate thereof.

[73] The medicine, method, first active ingredient for use, or use according to any one of [1] to [25], wherein compound 1 or the salt thereof, or the solvate thereof is compound 1, and the molecular-targeted agent, the MAPK/ERK-pathway inhibitor, or the MEK inhibitor is trametinib or a salt thereof, or a solvate thereof.

[74] The medicine, method, first active ingredient for use, or use according to any one of [1] to [73], wherein the cancer is a solid cancer or a blood cancer.

[75] The medicine, method, first active ingredient for use, or use according to any one of [1] to [74], wherein the cancer is at least one selected from the group consisting of lung cancer, esophageal cancer, gastric cancer, colorectal cancer, uterine cancer, ovarian cancer, pancreatic cancer, bladder cancer, thyroid cancer, skin cancer, leukemia, malignant lymphoma, and multiple myeloma.

[76] The medicine, method, first active ingredient for use, or use according to any one of [1] to [75], wherein the cancer is cancer associated with an abnormality in a RAS gene or MAPK/ERK-pathway (an abnormality in a protein on the MAPK/ERK-pathway or a gene producing the protein).

[77] The medicine, method, first active ingredient for use, or use according to any one of [1] to [76], wherein the abnormality in the MAPK/ERK-pathway is abnormal activation of the MAPK/ERK-pathway.

[78] The medicine, method, first active ingredient for use, or use according to any one of [1] to [77], wherein the abnormality in the MAPK/ERK-pathway is abnormal activation due to amplification of a protein in the MAPK/ERK-pathway.

[79] The medicine, method, first active ingredient for use, or use according to any one of [1] to [78], wherein the abnormality in the MAPK/ERK-pathway is abnormal activation due to gene mutation in the MAPK/ERK-pathway.

[80] The medicine, method, first active ingredient for use, or use according to any one of [1] to [79], wherein the cancer is a cancer associated with an abnormality in a RAS gene.

[81] The medicine, method, first active ingredient for use, or use according to [80], wherein the abnormality in a RAS gene is a mutation in the coding region of the RAS gene and/or an amplification of the copy number of the RAS gene.

[82] The medicine, method, first active ingredient for use, or use according to any one of [76] to [81], wherein the RAS gene is at least one RAS gene selected from the group consisting of a KRAS gene, a NRAS gene, and an HRAS gene.

[83] The medicine, method, first active ingredient for use, or use according to any one of [76] to [82], wherein the RAS gene is a KRAS gene.

[84] The medicine, method, first active ingredient for use, or use according to any one of [1] to [83], wherein the cancer is associated with a production of a mutated RAS protein and/or an increase in the production of a RAS protein.
[85] The medicine, method, first active ingredient for use, or use according to any one of [1] to [84], wherein the cancer is associated with a production of a mutated RAS protein.
[86] The medicine, method, first active ingredient for use, or use according to [84] or [85], wherein the mutant RAS protein is one or more mutant RAS protein selected from the group consisting of a mutant KRAS protein, a mutant NRAS protein, and a mutant HRAS protein.
[87] The medicine, method, first active ingredient for use, or use according to any one of [84] to [86], wherein the mutant RAS protein has a mutation at at least one amino acid position selected from the group consisting of G12, G13, and Q61.
[88] The medicine, method, first active ingredient for use, or use according to any one of [84] to [87], wherein the mutant RAS protein has a mutation at at least one amino acid position selected from the group consisting of G12, G13, and Q61 (but excluding the mutation of G12D).
[89] The medicine, method, first active ingredient for use, or use according to any one of [84] to [88], wherein the mutant RAS protein is a mutant KRAS protein.
[90] The medicine, method, first active ingredient for use, or use according to any one of [84] to [89], wherein the mutant RAS protein is a mutant KRAS protein, and has at least one amino acid mutation selected from the group consisting of G12A, G12C, G12D, G12S, G12V, G13D, Q61H, and Q61K.
[91] The medicine, method, first active ingredient for use, or use according to any one of [84] to [90], wherein the mutant RAS protein is a mutant KRAS protein, and has at least one amino acid mutation selected from the group consisting of G12A, G12C, G12S, G12V, G13D, Q61H, and Q61K.
[92] The medicine, method, first active ingredient for use, or use according to any one of [84] to [88], wherein the mutant RAS protein is a mutant NRAS protein, and has at least one amino acid mutation selected from the group consisting of G12C, G12D, G13D, G13V, Q61K, and Q61L.
[93] The medicine, method, first active ingredient for use, or use according to any one of [84] to [88] and [92], wherein the mutant RAS protein is a mutant NRAS protein, and has at least one amino acid mutation selected from the group consisting of G12C, G13D, G13V, Q61K, and Q61L.
[94] The medicine, method, first active ingredient for use, or use according to any one of [84] to [88], wherein the mutant RAS protein is a mutant HRAS protein, and has a G13R amino acid mutation.

Advantageous Effect of Invention

According to the present invention, there is provided a medicine that exhibits an improved effect in the treatment or prevention of cancer as compared with the prior art.

Brief Description of Drawings

[Figure 1] Figure 1 is a diagram showing results of evaluation of inhibitory activity due to combined use of compound 1 and sotorasib in RAS signaling using NCI-H358 in Example 1. More specifically, Figure 1 is an electrophoresis image showing results of Western blotting of proteins (KRAS, NRAS, HRAS, KRAS-GTP, NRAS-GTP, HRAS-GTP, ERK, AKT, MEK, pERK, pAKT, and pMEK) extracted from NCI-H358.
[Figure 2] Figure 2 is a diagram showing results of evaluation of inhibitory activity due to combined use of compound 1 and sotorasib in RAS signaling using NCI-H1373 in Example 1. More specifically, Figure 2 is an electrophoresis image showing results of Western blotting of proteins (KRAS, NRAS, HRAS, KRAS-GTP, NRAS-GTP, HRAS-GTP, ERK, AKT, MEK, pERK, pAKT, and pMEK) extracted from NCI-H1373.
[Figure 3] Figure 3 is a diagram showing results of evaluation of inhibitory activity due to combined use of compound 1 and RMC-4550 (RMC) in RAS signaling using NCI-H441 in Example 1. More specifically, Figure 3 is an electrophoresis image showing results of Western blotting of proteins (KRAS, NRAS, HRAS, KRAS-GTP, NRAS-GTP, HRAS-GTP, ERK, AKT, MEK, pERK, pAKT, and pMEK) extracted from NCI-H441.
[Figure 4] Figure 4 is a diagram showing results of evaluation of inhibitory activity due to combined use of compound 1 and trametinib (trame) in RAS signaling using NCI-H441 in Example 1. More specifically, Figure 4 is an electrophoresis image showing results of Western blotting of proteins (KRAS, NRAS, HRAS, KRAS-GTP, NRAS-GTP, HRAS-GTP, ERK, AKT, MEK, pERK, pAKT, and pMEK) extracted from NCI-H441.
[Figure 5] Figure 5 is a diagram showing results of evaluation of inhibitory activity due to combined use of compound 1 and compound 2 in RAS signaling using NCI-H358 in Example 1. More specifically, Figure 5 is an electrophoresis image showing results of Western blotting of proteins (KRAS, KRAS-GTP, ERK, AKT, MEK, pERK, pAKT, and pMEK)

extracted from NCI-H358.

[Figure 6] Figure 6 is a diagram showing results of evaluation of inhibitory activity due to combined use of compound 1 and compound 2 in RAS signaling using NCI-H441 in Example 1. More specifically, Figure 6 is an electrophoresis image showing results of Western blotting of proteins (KRAS, KRAS-GTP, ERK, AKT, MEK, pERK, pAKT, and pMEK) extracted from NCI-H441.

[Figure 7] Figure 7 is a diagram showing results of evaluation of cell growth inhibitory activity due to combined use of compound 1 and RMC-4550 using NCI-H358 in Example 2. In Figure 7a, the abscissa represents the concentration of RMC-4550, and the ordinate represents the concentration of compound 1. Each cell in Figure 7a shows a growth inhibition (GI, growth inhibition rate (%)) at each concentration. In Figure 7b, the abscissa represents a value obtained by dividing the concentration of RMC-4550 by the concentration (0.55 μM) of RMC-4550 as a single drug giving GI 155, and the ordinate represents a value obtained by dividing the concentration of compound 1 by the concentration (0.033 μM) of compound 1 as a single drug giving GI 155. Concentrations of RMC-4550 and compound 1 used in combination that give GI 155 are determined and plotted on the graph of Figure 7b.

[Figure 8] Figure 8 is a diagram showing results of evaluation of cell growth inhibitory activity due to combined use of compound 1 and RMC-4550 using NCI-H441 in Example 2. In Figure 8a, the abscissa represents the concentration of RMC-4550, and the ordinate represents the concentration of compound 1. Each cell in Figure 8a shows GI (%) at each concentration. In Figure 8b, the abscissa represents a value obtained by dividing the concentration of RMC-4550 by the concentration (0.35 μM) of RMC-4550 as a single drug giving GI 100, and the ordinate represents a value obtained by dividing the concentration of compound 1 by the concentration (0.0055 μM) of compound 1 as a single drug giving GI 100. Concentration of RMC-4550 and compound 1 used in combination that give GI 100 are determined and plotted on the graph of Figure 8b.

[Figure 9] Figure 9 is a diagram showing results of evaluation of cell growth inhibitory activity due to combined use of compound 1 and trametinib using NCI-H358 in Example 2. In Figure 9a, the abscissa represents the concentration of trametinib, and the ordinate represents the concentration of compound 1. Each cell in Figure 9a shows GI (%) at each concentration. In Figure 9b, the abscissa represents a value obtained by dividing the concentration of trametinib by the concentration (0.00035 μM) of trametinib as a single drug giving GI 50, and the ordinate represents a value obtained by dividing the concentration of compound 1 by the concentration (0.0046 μM) of compound 1 as a single drug giving GI 50. Concentrations of trametinib and compound 1 used in combination that give GI 50 are determined and plotted on the graph of Figure 9b.

[Figure 10] Figure 10 is a diagram showing results of evaluation of in vivo antitumor activity due to combined use of compound 1 and cetuximab using LoVo in Example 3. The ordinate represents a tumor volume, and the abscissa represents the number of days after cell transplantation.

[Figure 11] Figure 11 is a diagram showing results of evaluation of in vivo inhibitory activity in RAS signaling in Example 4. "Cetu" in the figure means cetuximab. More specifically, Figure 11 is an electrophoresis image showing results of Western blotting of proteins (KRAS, KRAS-GTP, ERK, AKT, EGFR, pERK, pAKT, and pEGFR) extracted from tumor cells of Example 4.

[Figure 12] Figure 12 is a diagram showing results of evaluation of in vivo antitumor activity due to combined use of compound 1 and bevacizumab using AsPC-1 (pancreatic cancer: KRAS G12D variant) in Example 5. The ordinate represents a tumor volume, and the abscissa represents the number of days after cell transplantation.

[Figure 13] Figure 13 is a diagram showing results of evaluation of cell growth inhibitory activity due to combined use of compound 1 and sotorasib using SW837 in Example 6.

[Figure 14] Figure 14 is a diagram showing results of evaluation of cell growth inhibitory activity due to combined use of compound 1 and MRTX849 using HCC-1171 and NCI-H1373 in Example 7.

[Figure 15] Figure 15 is a diagram showing results of evaluation of cell growth inhibitory activity due to combined use of compound 1 and TNO155 using HCC-1171, NCI-H23, NCI-H1373, and UM-UC-3 in Example 7.

[Figure 16] Figure 16 is a diagram showing results of evaluation of in vivo antitumor activity due to combined use of compound 1 and sotorasib using CTG-2579 (lung cancer: KRAS G12C variant) in Example 8. The ordinate represents a tumor volume, and the abscissa represents the number of days from the start of administration, with the start date of administration as day 0.

[Figure 17] Figure 17 is a diagram showing results of evaluation of in vivo inhibitory activity in RAS signaling in Example 9. More specifically, Figure 17 is an electrophoresis image showing results of Western blotting of proteins (KRAS, KRAS-GTP, ERK, pERK, AKT, and pAKT) extracted from tumor cells of Example 9.

[Figure 18] Figure 18 is a diagram showing results of evaluation of in vivo antitumor activity due to combined use of compound 1 and MRTX849 using LU65 (lung cancer: KRAS G12C variant) and UM-UC-3 (bladder cancer: KRAS G12C variant) in Example 10. The ordinate represents a tumor volume, and the abscissa represents the number of days after cell transplantation.

[Figure 19] Figure 19 is a diagram showing results of evaluation of in vivo antitumor activity due to combined use of compound 1 and TNO155 using NCI-H1373 (lung cancer: KRAS G12C variant) in Example 11. The ordinate

represents a tumor volume, and the abscissa represents the number of days from the start of administration, with the start date of administration as day 0.

[Figure 20] Figure 20 is a diagram showing results of evaluation of in vivo antitumor activity due to combined use of compound 1 and compound 2 using NCI-H441 (lung cancer: KRAS G12V variant) in Example 12. The ordinate represents a tumor volume, and the abscissa represents the number of days after cell transplantation.

[Figure 21] Figure 21 is a diagram showing results of evaluation of in vivo antitumor activity due to combined use of compound 1 and compound 2 using NCI-H1373 (lung cancer: KRAS G12C variant) in Example 12. The ordinate represents a tumor volume, and the abscissa represents the number of days from the start of administration, with the start date of administration as day 0.

[Figure 22] Figure 22 is a diagram showing results of evaluation of in vivo inhibitory activity in RAS signaling in Example 13. More specifically, Figure 22 is an electrophoresis image showing results of Western blotting of proteins (KRAS, KRAS-GTP, ERK, pERK, AKT, pAKT, MEK and pMEK) extracted from tumor cells of Example 13.

Description of Embodiments

Embodiments of the present invention will be described. Note that the present invention is not limited to the following embodiments. The medicine for the treatment or prevention, and the method for the treatment or prevention of the present invention may be administered or applied to humans. As used herein, "to" indicating a range means that values at both ends thereof are included, and for example, "A to B" means a range of A or more and B or less. As used herein, the term "about" when used in combination with a numerical value means a range of +10% and -10% of the numerical value. In the present invention, the term "and/or" is meant to include every combination of the terms "and" and "or" appropriately combined. Specifically, for example, the term "A, B, and/or C" includes the following seven variations; (i) A, (ii) B, (iii) C, (iv) A and B, (v) A and C, (vi) B and C, and (vii) A, B, and C.

One embodiment of the present invention is a medicine for treating or preventing cancer, the medicine comprising a first active ingredient and being used in combination with a second active ingredient, wherein the first active ingredient is a compound represented by the following formula (1) (hereinafter, also referred to as "compound 1") or a salt thereof, or a solvate thereof, and the second active ingredient is a molecular-targeted agent.

[Formula 4]

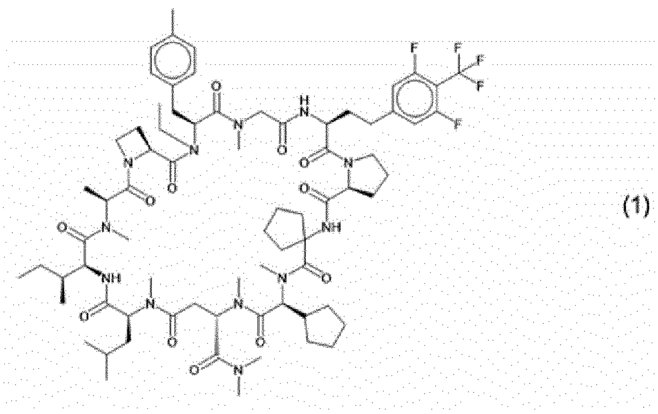

(1)

Another embodiment of the present invention is a medicine for treating or preventing cancer, the medicine comprising a first active ingredient and being used in combination with a second active ingredient, wherein the first active ingredient is a molecular-targeted agent, and the second active ingredient is compound 1 or a salt thereof, or a solvate thereof.

In order for compound 1 or a salt thereof, or a solvate thereof to be used in combination with a molecular-targeted agent, compound 1 of the present invention or a salt thereof, or a solvate thereof can be used as either the first active ingredient or the second active ingredient, and the molecular-targeted agent of the present invention can be used as either the first active ingredient or the second active ingredient. Preferably, the molecular-targeted agent is a MAPK/ERK-pathway inhibitor. More preferably, the molecular-targeted agent is one selected from the group consisting of an EGFR inhibitor, a VEGF inhibitor, an SHP2 inhibitor, a KRAS-G12C inhibitor, and a MEK inhibitor.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is a MAPK/ERK-pathway inhibitor.

Another embodiment of the present invention is a medicine for treating or preventing cancer, the medicine comprising a first active ingredient and being used in combination with a second active ingredient, wherein the first active ingredient is a MAPK/ERK-pathway inhibitor, and the second active ingredient is compound 1 or a salt thereof, or a solvate thereof.

In order for compound 1 or a salt thereof, or a solvate thereof to be used in combination with a MAPK/ERK-pathway inhibitor, compound 1 according to the present embodiment or a salt thereof, or a solvate thereof can be used as either the first active ingredient or the second active ingredient, and the MAPK/ERK-pathway inhibitor according to the present embodiment can be used as either the first active ingredient or the second active ingredient.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is an EGFR inhibitor.

Another embodiment of the present invention is a medicine for treating or preventing cancer, the medicine comprising a first active ingredient and being used in combination with a second active ingredient, wherein the first active ingredient is an EGFR inhibitor, and the second active ingredient is compound 1 or a salt thereof, or a solvate thereof.

In order for compound 1 or a salt thereof, or a solvate thereof to be used in combination with an EGFR inhibitor, compound 1 according to the present embodiment or a salt thereof, or a solvate thereof can be used as either the first active ingredient or the second active ingredient, and the EGFR inhibitor according to the present embodiment can be used as either the first active ingredient or the second active ingredient. The EGFR inhibitor may be at least one selected from the group consisting of gefitinib, erlotinib, afatinib, osimertinib, lapatinib, or a salt thereof, or a solvate thereof, and an anti-EGFR antibody and may be an anti-EGFR antibody. Preferably, the EGFR inhibitor may be an anti-EGFR antibody. The anti-EGFR antibody may have a heavy chain variable region comprising an amino acid sequence of SEQ ID NO: 1 and a light chain variable region comprising an amino acid sequence of SEQ ID NO: 2 and may be cetuximab. More preferably, the EGFR inhibitor may be cetuximab.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is a VEGF inhibitor.

Another embodiment of the present invention is a medicine for treating or preventing cancer, the medicine comprising a first active ingredient and being used in combination with a second active ingredient, wherein the first active ingredient is a VEGF inhibitor, and the second active ingredient is compound 1 or a salt thereof, or a solvate thereof.

In order for compound 1 or a salt thereof, or a solvate thereof to be used in combination with a VEGF inhibitor, compound 1 according to the present embodiment or a salt thereof, or a solvate thereof can be used as either the first active ingredient or the second active ingredient, and the VEGF inhibitor according to the present embodiment can be used as either the first active ingredient or the second active ingredient. The VEGF inhibitor may be at least one selected from the group consisting of sorafenib, pazopanib, sunitinib, axitinib, regorafenib, or a salt thereof, or a solvate thereof, and an anti-VEGF antibody, may be an anti VEGF antibody, may have a heavy chain variable region comprising an amino acid sequence of SEQ ID NO: 3 and a light chain variable region comprising an amino acid sequence of SEQ ID NO: 4, and may be bevacizumab, ramucirumab, or aflibercept. The VEGF inhibitor is preferably an anti-VEGF antibody, and more preferably bevacizumab.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is an SHP2 inhibitor.

Another embodiment of the present invention is a medicine for treating or preventing cancer, the medicine comprising a first active ingredient and being used in combination with a second active ingredient, wherein the first active ingredient is an SHP2 inhibitor, and the second active ingredient is compound 1 or a salt thereof, or a solvate thereof.

In order for compound 1 or a salt thereof, or a solvate thereof to be used in combination with an SHP2 inhibitor, compound 1 according to the present embodiment or a salt thereof, or a solvate thereof can be used as either the first active ingredient or the second active ingredient, and the SHP2 inhibitor according to the present embodiment can be used as either the first active ingredient or the second active ingredient. The SHP2 inhibitor may be at least one selected from the group consisting of RMC4550, TNO155, RLY1971, SHP099, NSC-87877, and a salt thereof, and a solvate thereof, and preferred examples of the SHP2 inhibitor include RMC4550, RLY1971, TNO155, salts thereof, and solvates thereof. More preferably, the SHP2 inhibitor is RMC4550, TNO155, or a salt thereof, or a solvate thereof.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is a KRAS-G12C inhibitor.

Another embodiment of the present invention is a medicine for treating or preventing cancer, the medicine comprising a first active ingredient and being used in combination with a second active ingredient, wherein the first active ingredient is a KRAS-G12C inhibitor, and the second active ingredient is compound 1 or a salt thereof, or a solvate thereof.

In order for compound 1 or a salt thereof, or a solvate thereof to be used in combination with a KRAS-G12C inhibitor, compound 1 according to the present embodiment or a salt thereof, or a solvate thereof can be used as either the first active ingredient or the second active ingredient, and the KRAS-G12C inhibitor according to the present embodiment can be used as either the first active ingredient or the second active ingredient. The KRAS-G12C inhibitor is at least one selected from the group consisting of sotorasib, MRTX849, and a salt thereof, and a solvate thereof and is preferably sotorasib, MRTX849, or a salt thereof, or a solvate thereof.

One embodiment of the present invention is a medicine for treating or preventing cancer, the medicine comprising a first active ingredient and being used in combination with a second active ingredient, wherein the first active ingredient is compound 1 or a salt thereof, or a solvate thereof, and the second active ingredient is a MEK inhibitor.

Another embodiment of the present invention is a medicine for treating or preventing cancer, the medicine comprising a first active ingredient and being used in combination with a second active ingredient, wherein the first active ingredient is a MEK inhibitor, and the second active ingredient is compound 1 or a salt thereof, or a solvate thereof.

In order for compound 1 or a salt thereof, or a solvate thereof to be used in combination with a MEK inhibitor, compound 1 according to the present embodiment or a salt thereof, or a solvate thereof can be used as either the first active ingredient or the second active ingredient, and the MEK inhibitor according to the present embodiment can be used as either the first active ingredient or the second active ingredient. Examples of the MEK inhibitor include at least one selected from the group consisting of a compound represented by the following formula (2), trametinib, selumetinib, CH4987655, and a salt thereof, and a solvate thereof and preferred examples include a compound represented by formula (2), trametinib, or a salt thereof, or a solvate thereof. More preferably, examples of the MEK inhibitor include a compound represented by formula (2), a salt thereof, and a solvate thereof.

The first active ingredient and the second active ingredient may be provided as a kit. Another embodiment of the present invention can be a kit comprising the first active ingredient and the second active ingredient. The medicine of the present invention may be provided as a kit. Another embodiment of the present invention may be a kit comprising the medicine.

The term "use in combination" or "combined use" refers to use of active ingredients in combination. For example, the combined use of a first active ingredient and a second active ingredient includes "an aspect in which the first active ingredient and the second active ingredient are administered as a single formulation containing the first active ingredient and the second active ingredient" (i.e., an aspect in which the first active ingredient and the second active ingredient are used in combination as combination drug (complex drug)) and "an aspect in which the first active ingredient and the second active ingredient are administered simultaneously or separately as separate formulations." In the latter aspect, the formulation containing the first active ingredient may be administered first, or the formulation containing the second active ingredient may be administered first. The latter aspect may be any one of "an aspect in which the first active ingredient and the second active ingredient are separately formulated and simultaneously administered via the same administration route," "an aspect in which the first active ingredient and the second active ingredient are separately formulated and separately administered at different times via the same administration route," "an aspect in which the first active ingredient and the second active ingredient are separately formulated and simultaneously administered via different administration routes (administered from different sites of the same patient)," and "an aspect in which the first active ingredient and the second active ingredient are separately formulated and separately administered at different times via different administration routes." In the case of the "aspect in which the first active ingredient and the second active ingredient are separately formulated and simultaneously administered via the same administration route," both of the agents (agent containing the first active ingredient and agent containing the second active ingredient) may be mixed immediately before the administration. The term "separately" means that one formulation is administered before or after administration of another formulation.

In other words, the term "use in combination" or "combined use" can also refer to usage in which one active ingredient is present in the body of a patient with the other active ingredient present in the body of the patient. That is, an aspect in which the first active ingredient and the second active ingredient are administered so as to be simultaneously present in the body of a patient, for example, in blood, is preferable, and an aspect in which certain agents containing the first active ingredient and the second active ingredient, respectively, are simultaneously administered to a patient, or in which a certain agent containing the first active ingredient or the second active ingredient is administered and then another agent containing the other active ingredient is administered within 48 hours is preferable.

Compound 1 or a salt thereof, or a solvate thereof may be compound 1, a solvate of compound 1, or a hydrate of compound 1.

Examples of the salt of compound 1 include pharmaceutically acceptable salts, and specific examples thereof include: salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; and salts with amino acids such as arginine. These salts are produced by, for example, a contact of the compound with an

acid or a base. The solvate as used herein is not particularly limited as long as it forms a single molecular population together with a solvent, but it is a solvate formed by a solvent that can be taken (ingested) with the administration of a drug. Examples of the solvate include hydrates, alcohol hydrates (such as ethanol hydrates, methanol hydrates, 1-propanol hydrates, and 2-propanol hydrates), and not only solvates formed with a single solvent such as dimethyl sulfoxide, but also solvates formed with a plurality of molecules of solvent per one molecule of compound, or solvates formed with a plurality of types of solvents per one molecule of compound. If a solvent is water, it is called a hydrate. A solvate is produced by, for example, a contact of the compound with a solvent, and a solvate of a salt is produced by, for example, a contact of a compound salt with a solvent. The salt of the compound or the solvate of the compound or salt of the compound are all active in the same manner as the compound (free form). As used herein, the term "solvate (thereof)" includes a solvate of compound 1, a solvate of the molecular-targeted agent, a solvate of a salt of compound 1, and a solvate of a salt of the molecular-targeted agent.

Compound 1 or the salt thereof, or the solvate thereof may include crystal polymorphism, and the crystal polymorphism may be either a single substance or a mixture of any crystal forms. Compound 1 or the salt thereof, or the solvate thereof also includes an amorphous substance.

A dose of compound 1 or the salt thereof, or the solvate thereof is preferably 0.001 to 100 mg per administration per kg of the body weight of a subject to be administered (0.001 to 100 mg/kg/day), more preferably 0.003 to 20 mg/kg/day, still more preferably 0.003 to 10 mg/kg/day. When the dose of compound 1 falls within the above range, the effect of treating or preventing cancer will be further enhanced. A frequency of administration of compound 1 can be, for example, once a day, twice a day, or three times a day, but once a day is more preferred. The same dosages and/or administration can be applied to the molecular-targeted agent.

Compound 1 can be produced according to, for example, the method described in International Publication No. WO 2021/090855, the Fmoc-based peptide synthesis method, or solid phase peptide synthesis (published by Bachem, [search on December 24, 2021], Internet URL:https://www.bachem.com/wp-admin/admin-ajax.php?juwpfisadmin=false&action=wpfd&task=file.download&wpfd_category_id=180&wpf d_file_id=213455&token=&preview=1, or Peptide Guide, No: 2004505, published by Global Marketing Bachem AG, 2020.).

As used herein, the term "molecular-targeted agent" refers to an agent which has an antitumor effect and which specifically inhibits a particular protein. In the present specification, compound 1 or the salt thereof, or the solvate thereof is not included in the molecular-targeted agent. Examples of the molecular-targeted agent include a MAPK/ERK-pathway inhibitor, which can be used as a medicine for treating or preventing cancer. In the present specification, the term "MAPK/ERK-pathway inhibitor" refers to an inhibitor of a target involved in MAPK/ERK signaling and includes inhibitors of growth factors and their receptors, as well as kinase inhibitors. Example of the MAPK/ERK-pathway inhibitor include an EGFR inhibitor, a VEGF inhibitor, an SHP2 inhibitor, a KRAS inhibitor, a NRAS inhibitor, an HRAS inhibitor, a KRAS-GTP inhibitor, an NRAS-GTP inhibitor, an HRAS-GTP inhibitor, a KRAS-G12C inhibitor, an ERK inhibitor, an AKT inhibitor and a MEK inhibitor. The molecular-targeted agent may be at least one selected from the group consisting of an EGFR inhibitor, a VEGF inhibitor, an SHP2 inhibitor, a KRAS-G12C inhibitor, and a MEK inhibitor and may be an EGFR inhibitor, a VEGF inhibitor, an SHP2 inhibitor, a KRAS-G12C inhibitor, or a MEK inhibitor. Preferably, the molecular-targeted agent is an EGFR inhibitor. The EGFR inhibitor may be at least one selected from the group consisting of gefitinib, erlotinib, afatinib, osimertinib, lapatinib, or a salt thereof, or a solvate thereof, and an anti-EGFR antibody and may be an anti-EGFR antibody. Preferably, the EGFR inhibitor is an anti-EGFR antibody. The anti-EGFR antibody may have a heavy chain variable region comprising an amino acid sequence of SEQ ID NO: 1 and a light chain variable region comprising an amino acid sequence of SEQ ID NO: 2 and may be cetuximab. More preferably, the EGFR inhibitor is cetuximab. The VEGF inhibitor may be at least one selected from the group consisting of sorafenib, pazopanib, sunitinib, axitinib, regorafenib, or a salt thereof, or a solvate thereof, and an anti-VEGF antibody, may be an anti VEGF antibody, may have a heavy chain variable region comprising an amino acid sequence of SEQ ID NO: 3 and a light chain variable region comprising an amino acid sequence of SEQ ID NO: 4, and may be bevacizumab, ramucirumab, or aflibercept. The VEGF inhibitor is preferably an anti-VEGF antibody, and more preferably bevacizumab. The SHP2 inhibitor may be at least one selected from the group consisting of RMC4550, TNO155, RLY1971, SHP099, NSC-87877, and a salt thereof, and a solvate thereof and may be RMC4550, TNO155, and a salt thereof, and a solvate thereof. More preferably, examples of the SHP2 inhibitor include one selected from the group consisting of RMC4550, TNO155, RLY1971, salts thereof, and solvates thereof. More preferably, examples of the SHP2 inhibitor include RMC4550, TNO155, a salt thereof, and a solvate thereof. The KRAS-G12C inhibitor may be at least one selected from the group consisting of sotorasib, MRTX849, and a salt thereof, and a solvate thereof and may preferably be sotorasib or a salt thereof, or a solvate thereof, or MRTX849 or a salt thereof, or a solvate thereof. The MEK inhibitor may be at least one selected from the group consisting of a compound represented by the following formula (2), trametinib, selumetinib, CH4987655, and a salt thereof, and a solvate thereof and may be preferably a compound represented by the following formula (2), trametinib, or a salt thereof, or a solvate thereof. More preferably, examples of the MEK inhibitor include a compound represented by the following formula (2), a salt thereof, and a solvate thereof.

[Formula 5]

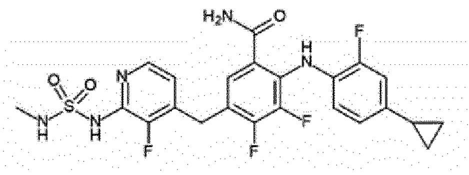

(2)

The molecular-targeted agent can be obtained from commercial suppliers or produced according to known methods.

Gefitinib (CAS No.: 184475-35-2, N-(3-chloro-4-fluoro-phenyl)-7-methoxy-6-(3-morpholine-4-ylpropoxy)quinazoline-4-amine) is a compound represented by the following formula (3). Gefitinib is used as Iressa.

[Formula 6]

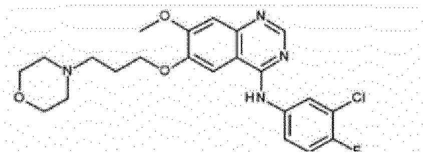

(3)

Erlotinib (CAS No.: 183321-74-6, N-(3-ethynylphenyl)-6,7-bis(2-methoxyethoxy)quinazoline-4-amine) is a compound represented by the following formula (4). Erlotinib is used as erlotinib hydrochloride or Tarceva.

[Formula 7]

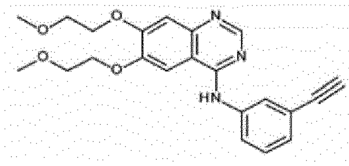

(4)

Afatinib (CAS No.: 439081-18-2, N-[4-[(3-chloro-4-fluorophenyl)amino]-7-[[(3S)-tetrahydro-3-furanyl]oxy]-6-quinazolinyl]-4(dimethylamino)-2-butenamide) is a compound represented by the following formula (5). Afatinib is used as afatinib maleate or Giotrif.

[Formula 8]

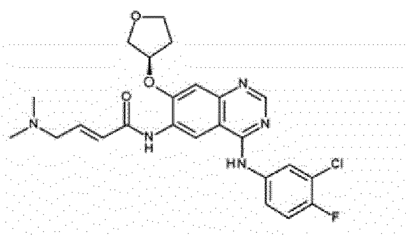

(5)

Osimertinib (CAS No.: 1421373-65-0, N-(2-{2-dimethylaminoethyl-methylamino}-4-methoxy-5-{[4-(1-methylindole-3-yl)pyrimidine-2-yl]amino}phenyl)prop-2-enamide) is a compound represented by the following formula (6). Osimertinib is used as osimertinib mesylate or Tagrisso.

[Formula 9]

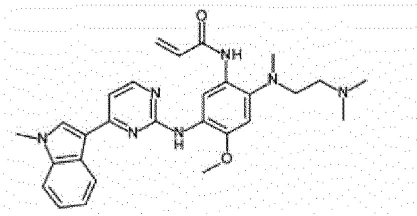

(6)

Lapatinib (CAS No.: 231277-92-2, N-[3-chloro-4-[(3-fluorophenyl)methoxy]phenyl]-6-[5-[(2-methylsulfonylethylamino)methyl]-2-furyl]quinazoline-4-amine) is a compound represented by the following formula (7). Lapatinib is used as lapatinib tosilate hydrate or Tykerb.

[Formula 10]

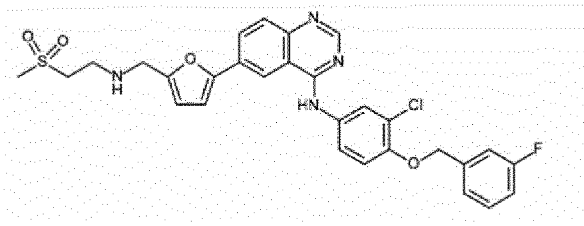

(7)

RMC-4550 (CAS No.: 2172651-73-7, 3-[(3S,4S)-4-amino-3-methyl-2-oxa-8-azaspiro[4.5]deca-8-yl]-6-(2,3-dichlorophenyl)-5-methyl-2-pyrazinemethanol) is a compound represented by the following formula (8).

[Formula 11]

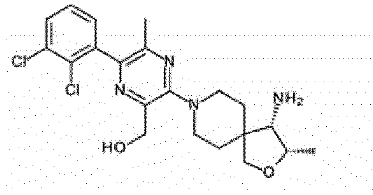

(8)

TNO155 (CAS No.: 1801765-04-7, (3S,4S)-8-(6-amino-5-((2-amino-3-chloropyridine-4-yl)thio)pyrazine-2-yl)-3-methyl-2-oxa-8-azaspiro[4.5]decane-4-amine) is a compound represented by the following formula (9).

[Formula 12]

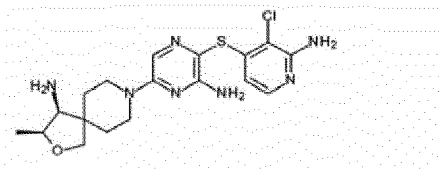

(9)

RLY1971 is a compound represented by CAS No.: 2668298-71-1.

SHP099 (CAS No.: 1801747-42-1, 6-(4-amino-4-methyl-1-piperidinyl)-3-(2,3-dichlorophenyl)-2-pyrazineamine) is a compound represented by the following formula (10).

[Formula 13]

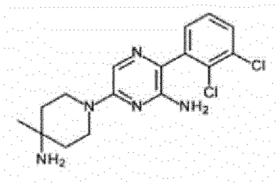

(10)

NSC-87877 (CAS No.: 56990-57-9, 8-hydroxy-7-[2-(6-suflo-2-naphthalenyl)diazenyl]-5-quinolinesulfonic acid) is a compound represented by the following formula (II).

[Formula 14]

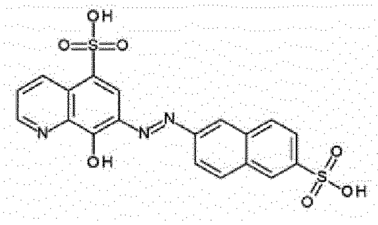

(11)

Sotorasib (CAS No.: 2296729-00-3, 4-((S)-4-acryloyl-2-methylpiperazine-1-yl)-6-fluoro-7-(2-fluoro-6-hydroxyphenyl)-1-(2-isopropyl-4-methylpyridine-3-yl)pyrido[2,3-d]pyrimidine-2(1H)-one) is a compound represented by the following formula (12). Sotorasib is used as AMG510 or Lumakras.

[Formula 15]

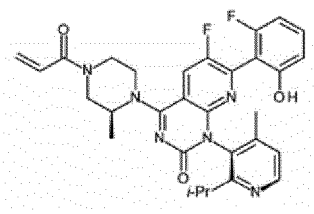

(12)

MRTX849 (CAS No.: 2326521-71-3, 2-((S)-4-(7-(8-chloronaphthalene-1-yl)-2-(((S)-1-methylpyrrolidine-2-yl) methoxy)-5, 6,7, 8-tetrahydropyrido[3,4-d]pyrimidine-4-yl)-1-(2-fluoroacryloyl)piperazine-2-yl)acetonitrile) is a compound represented by the following formula (13). MRTX849 is used as Adagrasib.

[Formula 16]

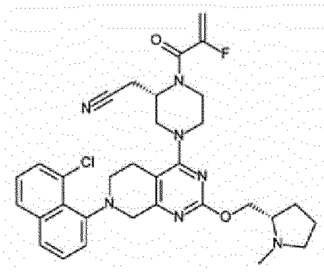

(13)

Trametinib (CAS No.: 871700-17-3, N-[3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidine-1-yl]phenyl]acetamide is a compound represented by the following formula (14). Trametinib is used as trametinib dimethyl sulfoxide or Mekinist.

[Formula 17]

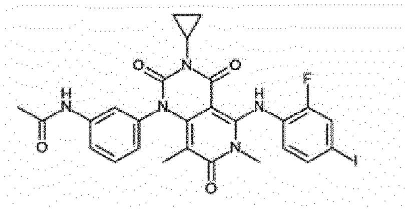

(14)

Compound 2 (CAS No.: 2677856-11-8, 2-(4-cyclopropyl-2-fluoro-anilino)-3,4-difluoro-5-[[3-fluoro-2-(methylsulfamoylamino)-4-pyridyl]methyl]benzamide, 2-(4-cyclopropyl-2-fluoro-anilino)-3,4-difluoro-5-[[3-fluoro-2-(methylsulfamoylamino)-4-pyridyl]methyl]benzamide) is a compound represented by the following formula (2). Compound 2 is used, for example, as a sodium salt.

[Formula 18]

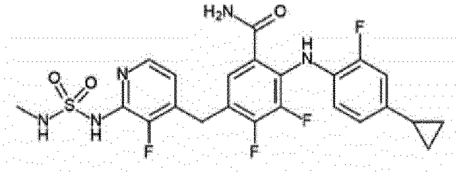

(2)

CH4987655 (CAS No.: 874101-00-5, 3,4-difluoro-2-[(2-fluoro-4-iodophenyl)amino]-N-(2-hydroxyethoxy)-5-[(tetrahydro-3-oxo-2H-1,2-oxazine-2-yl)methyl]benzamide, RO4987655) is a compound represented by the following formula (15).

[Formula 19]

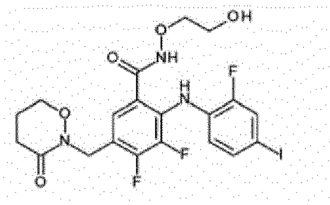

(15)

Selumetinib (CAS No.: 606143-52-6, 6-(4-bromo-2-chloroanilino)-7-fluoro-N-(2-hydroxyethoxy)-3-methylbenzimidazole-5-carboxamide) is a compound represented by the following formula (16). Selumetinib is used as selumetinib sulfate or KOSELUGO.

[Formula 20]

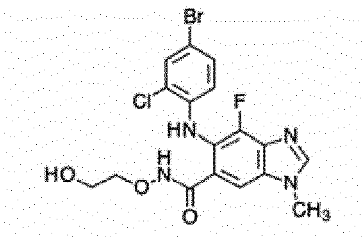

(16)

Sorafenib (CAS No.: 284461-73-0, 4-[4-[[4-chloro-3-(trifluoromethyl)phenyl]carbamoylamino]phenoxy]-N-methyl-pyridine-2-carboxamide) is a compound represented by the following formula (17). Sorafenib is used as sorafenib tosilate or Nexavar.

[Formula 21]

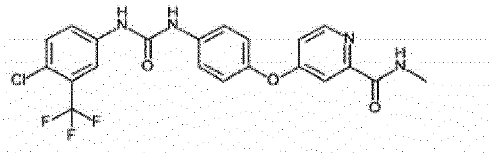

(17)

Pazopanib (CAS No.: 444731-52-6, 5-[[4-[(2,3-dimethylindazole-6-yl)-methyl-amino]pyrimidine-2-yl]amino]-2-methyl-benzenesulfonamide) is a compound represented by the following formula (18). Pazopanib is used as pazopanib hydrochloride or Votrient.

[Formula 22]

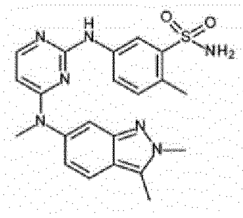

(18)

Sunitinib (CAS No.: 557795-19-4, N-[2-(diethylamino)ethyl]-5-[(Z)-(5-fluoro-2-oxo-indoline-3-ylidene) methyl]-2,4-dimethyl-1H-pyrrole-3-carboxamide) is a compound represented by the following formula (19). Sunitinib is used as sunitinib malate or Sutent.

[Formula 23]

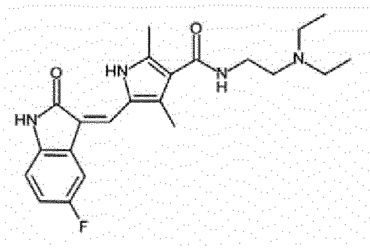

(19)

Axitinib (CAS No.: 319460-85-0, N-methyl-2-[[3-[(E)-2-(2-pyridyl)vinyl]-1H-indazole-6-yl]sulfanyl]benzamide) is a compound represented by the following formula (20). Axitinib is used as Inlta.

[Formula 24]

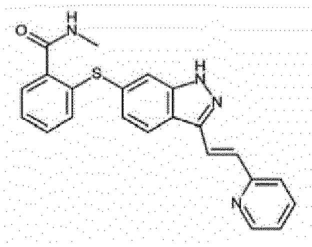

(20)

Regorafenib (CAS No.: 755037-03-7, 4-[4-[[4-chloro-3-(trifluoromethyl)phenyl]carbamoylamino]-3-fluoro-phenoxy]-N-methyl-pyridine-2-carboxamide) is a compound represented by the following formula (21). Regorafenib is used as regorafenib hydrate or Stivarga.

[Formula 25]

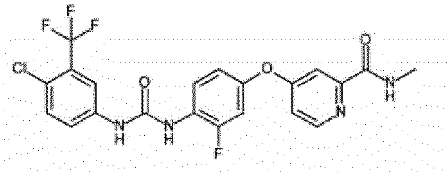

(21)

A dose of the molecular-targeted agent is preferably 0.005 to 100 mg per day per kg of the body weight of a subject to be administered (0.005 to 100 mg/kg/day), more preferably 0.01 to 75 mg/kg/day, still more preferably 0.02 to 50 mg/kg/day, most preferably 5 to 40 mg/kg/day. When the dose of the molecular-targeted agent falls within the above range, the effect of treating or preventing cancer will be further enhanced. A frequency of administration of the molecular-targeted agent can be, for example, one or more times per week, and can be twice per week. The same dosages and/or administration can be applied to compound 1 or salts thereof, or solvates thereof.

Doses of compound 1 or the salts thereof, or the solvates thereof and of the molecular-targeted agent are preferably 0.001 to 100 mg/kg/day for compound 1 or the salts thereof, or the solvates thereof and 0.005 to 100 mg/kg/day for the molecular-targeted agent, more preferably 0.003 to 20 mg/kg/day for compound 1 or the salts thereof, or the solvates thereof and 0.01 to 75 mg/kg/day for the molecular-targeted agent, still more preferably 0.003 to 10 mg/kg/day for compound 1 or the salts thereof, or the solvates thereof and 0.02 to 30 mg/kg/day for the molecular-targeted agent, respectively, per kg of the body weight of a subject to be administered. When the doses of compound 1 or the salts thereof, or the solvates thereof and of the molecular-targeted agent fall within the above ranges, the effect of treating or preventing cancer will be further enhanced. The dosage and administration can be adjusted as appropriate while monitoring blood drug concentrations such as AUC and Cmax and the patient's condition. For the appropriate dose and number of doses (frequency of administration), for example, the package insert of the molecular-targeted agent can also be referred to. The administration of compound 1 or the salt thereof, or the solvate thereof may be, for example, once daily or more, or twice daily. The administration of compound 1 or the salt thereof, or the solvate thereof is preferably once daily.

In the present invention, examples of the administration method include oral, transrectal, parenteral (intravenous, intramuscular, subcutaneous, percutaneous absorption), intracisternal, intravaginal, intraperitoneal, intravesical, or topical (injection, drip, powder, ointment, gel, or cream) administration, and (intraoral or nasal spray) inhalation. Examples of the dosage form therefor include tablets, capsules, granules, powders, pills, aqueous and non-aqueous oral solutions and suspensions, and parenteral solutions filled into containers adapted for subdivision into individual doses. The dosage form can also be adapted for various administration methods, encompassing controlled release formulations such as subcutaneous transplantation. The administration method is equally applicable to the first active ingredient and the second active ingredient. The first active ingredient and the second active ingredient can be used as agents and can be administered as a formulation or a combination drug.

To the first active ingredient, for example, any of the administration methods described above can be applied, and to the second active ingredient, for example, an administration method the same as or different from that for the first active ingredient can be applied. An interval between administration of the first active ingredient and the second active ingredient can be, for example, an interval of 0 days to 14 days, an interval of 0 days to 10 days, or an interval of 0 days to 7 days. The administration interval can be determined based on an index such as AUC, Cmax, Tmax, elimination half-life, or health status of a subject. For example, compound 1 or the salt thereof, or the solvate thereof can be administered orally (tablets, capsules, etc.) and the molecular-targeted agent can be administered by infusion, respectively. For example, both of compound 1 or the salt thereof, or the solvate thereof and the molecular-targeted agent can be administered orally (tablets, capsules, etc.). For example, compound 1 or the salt thereof, or the solvate thereof can be administered by infusion and the molecular-targeted agent can be administered orally (tablets, capsules, etc.). For example, both of compound 1 or the salt thereof, or the solvate thereof and the molecular-targeted agent can be administered by infusion. In such a case, compound 1 or the salt thereof, or the solvate thereof and the molecular-targeted agent may be administered at any interval, such as twice a day, once a day, once a week, or once every two weeks. More specifically, both of compound 1 or the salt thereof, or the solvate thereof and the molecular-targeted agent may be administered once a day; in this case, they may be administered before meal, between meals, or after meal. The meal as in before meal, between meals, or after meal may be any of the following meals: breakfast, lunch, dinner, evening meal, or snack. If the interval between the administration of compound 1 or the salt thereof, or the solvate thereof and the administration of the molecular-targeted agent is within 24 hours, it can be said that both are administered once a day. If necessary, compound 1 or the salt thereof, or the solvate thereof and the molecular-targeted agent may be administered twice a day and once a day, once a day and once a day, once a day and twice a day, once a day and once every 2 days, once a day and once every 3 days, once a day and once every 7 days, and twice a day and once every 7 days, respectively. If necessary, the interval of administration of the molecular-targeted agent alone can be increased or decreased, or the interval of administration of compound 1 or the

salt thereof, or the solvate thereof alone can be increased or decreased based on the AUC, Cmax, Tmax, or elimination half-life of compound 1 and/or the molecular-targeted agent, or the patient's health status. The administration of the molecular-targeted agent may be started on the day of starting the administration of compound 1 or the salt thereof, or the solvate thereof, or the day of starting the administration of compound 1 or the salt thereof, or the solvate thereof may be different from the day of starting the administration of the molecular-targeted agent. For the duration of administration, the total number of courses may be one course or more, or two courses or more, with 7 days as one course. Each course may be continuously carried out, or a rest period may be provided. A rest period may be provided in a course. If necessary, during the course, only compound 1 or the salt thereof, or the solvate thereof can be continuously administered with the molecular-targeted agent withdrawn, or only compound 1 or the salt thereof, or the solvate thereof can be withdrawn with the molecular-targeted agent continuously administered. Compound 1 or the salt thereof, or the solvate thereof and the molecular-targeted agent can also be included in the same tablet, capsule or the like.

The pharmaceutical formulation according to the present invention can be formulated by known methods by introducing a pharmaceutically acceptable carrier in addition to the active ingredients such as compound 1 or the salt thereof, or the solvate thereof and the molecular-targeted agent. The pharmaceutical formulation according to the present invention can be formulated with conventional excipients, binders, lubricants, colorants, or flavoring agents, and if necessary, stabilizers, emulsifiers, absorption enhancers, surfactants, pH adjusters, preservatives, antioxidants, or the like, and can be formulated by conventional methods by mixing ingredients generally used as raw materials for pharmaceutical formulations. In the formulation, the active ingredient used in the pharmaceutical can be processed into an optimal form or shape, that is, dosage form according to the use method or use purpose by known methods. Commonly used dosage forms include, but are not limited to, liquid pharmaceutical preparations (liquids) such as injections, suspensions, emulsions, and ophthalmic solutions, and solid pharmaceutical preparations (solid preparations) such as tablets, powders, fine granules, granules, coated tablets, capsules, dry syrup, lozenges, and suppositories. For example, formulations containing the active ingredients exemplified by compound 1 or the salt thereof, or the solvate thereof and the molecular-targeted agent can be used as the medicine of the present invention.

In the therapeutic or preventive medicine of the present invention, either or both of the first active ingredient and the second active ingredient may be used in admixture with a pharmaceutically acceptable additive. As the pharmaceutically acceptable additive, those skilled in the art can appropriately select well-known additives, and a plurality of additives can be appropriately used if necessary. The additive is appropriately selected depending on an oral preparation or an injection and may be, for example, a mixture with water, ethanol, physiological saline, liquid paraffin, a surfactant, sucrose, or the like; and the obtained mixture may be encapsulated.

Examples of the cancer include a solid cancer and a blood cancer, both types of which are composed of abnormal cells that grow uncontrollably. Solid tumors form one or many masses, while blood cancers circulate throughout the body via the bloodstream. The cancer may be, for example, at least one selected from the group consisting of lung cancer, esophageal cancer, gastric cancer, colorectal cancer, uterine cancer, ovarian cancer, pancreatic cancer, bladder cancer, thyroid cancer, skin cancer, leukemia, malignant lymphoma, and multiple myeloma. The cancer may be a cancer associated with an abnormality in a RAS gene. Preferably, the cancer is a lung cancer, a pancreatic cancer, or a colorectal cancer.

As used herein, "abnormal" refers to a state in which various chromosomes, genes, proteins, and signaling cascades within the cell deviate from their normal function and regulation, usually triggered by their homeostatic activation.

As used herein, "abnormal activation" refers to a state in which various chromosomes, genes, proteins and signaling cascades within the cell are more activated than in normal cells, which may be due in part, for example, to genetic mutations (Cancer Metastasis Rev, 2013, 32, 147-162), and cell activation can be measured by, for example, LC/MS-MS-based assays to directly quantitatively measure the involvement of mutants in cells (Cancer Discov., 2016, 6, 316 -329).

The term "protein amplification" refers to the synthesis and amplification of proteins in living cells, such as the amplification of proteins involved in the MAPK/ERK-pathway (e.g., KRAS, NRAS, HRAS, KRAS-GTP, NRAS-GTP, HRAS-GTP, ERK, AKT, MEK, pERK, pAKT, and pMEK). Protein amplification can be confirmed by measuring it by Western blotting, comparative genomic hybridization (CGH), or the like, and comparing it to the proteins in normal cells (Nat. Rev. Drug Discov., 2020, 19, 533-552, Molecular Cytogenetics, 2015, 8:103).

In one aspect, the cancer includes a cancer associated with an abnormality in a RAS gene. A cancer associated with an abnormality in a RAS gene refers to a cancer in which, for example, cancer cells have mutations in the coding region of a RAS gene and/or amplification of the copy number of a RAS gene and show increased RAS activity (including increased production of RAS proteins). A mutation in the coding region of a RAS gene refers to, for example, one or more base deletions, additions or substitutions in the coding region of the RAS gene. Amplification of the copy number of a RAS gene means that there are more copies of the RAS gene than the number of copies of the RAS gene normally present on the chromosome. More copies means more compared to normal cells, and a copy number of 4 or more can be considered as an amplified copy number.

Whether a cancer is associated with an abnormality in a RAS gene can be determined by checking whether the subject suffering from the cancer has an abnormality in a RAS gene. For example, it is possible to determine that the cancer

is a cancer associated with an abnormality in a RAS gene by identifying the presence of a mutation in the nucleotide sequence of the coding region of the RAS gene in a biological sample obtained from a subject suffering from cancer, and further confirming that the mutated RAS protein is expressed.

Whether a cancer is associated with an abnormality in a RAS gene can be determined by checking whether the subject suffering from the cancer has amplification of the copy number of the RAS gene. For example, it is possible to determine that the cancer is a cancer associated with an abnormality in a RAS gene by confirming that the copy number of the RAS gene is amplified, and/or that the production of RAS protein is increased in a biological sample obtained from a subject suffering from cancer. Increased production of RAS protein can also be referred to as overexpression of RAS protein. The increased production of RAS protein can be confirmed, for example, by comparing the expression levels of RAS protein in general, the expression levels of RAS protein in subjects who do not suffer from the cancer, or the expression levels of RAS protein in subjects before they suffer from the cancer.

If there is a mutation in the nucleotide sequence of the coding region of a RAS gene in a subject suffering from cancer, and the copy number of the RAS gene is amplified, it can also be considered as a cancer associated with an abnormality in a RAS gene.

Any method can be used as long as it is a method that can detect mutations in the coding region of a RAS gene or amplification of the copy number of the RAS gene in a biological sample of a subject suffering from cancer, and can be performed by a common method by those skilled in the art. For example, the RAS gene may be isolated from a biological sample obtained from a subject suffering from cancer, amplified by PCR or the like, and its nucleotide sequence determined directly by a sequencer or the like. In addition, commercially available in vitro diagnostics that can detect mutations in a RAS gene may be used, for example.

Three types of RAS protein isoforms are known: KRAS protein, NRAS protein, and HRAS protein. In one aspect, the RAS gene is one or more RAS genes selected from the group consisting of a KRAS gene, a NRAS gene, and an HRAS gene. Preferably, the RAS gene is a KRAS gene.

In one aspect, the cancer includes a cancer associated with the production of mutated RAS proteins and/or increased production of RAS proteins. The production of mutant RAS proteins and/or increased production of RAS proteins is usually due to the abnormality in a RAS gene described above.

In one aspect, the mutant RAS protein is at least one mutant RAS protein selected from the group consisting of a mutant KRAS protein, a mutant NRAS protein, and a mutant HRAS protein. Preferably, the mutant RAS protein is a mutant KRAS protein.

In one aspect, the mutant RAS protein has a mutation at at least one amino acid position selected from the group consisting of G12, G13, and Q61 in the amino acid sequence of the human wild-type KRAS protein, the amino acid sequence of the human wild-type NRAS protein, or the amino acid sequence of the human wild-type HRAS protein. In the amino acid sequences of the human wild-type KRAS protein, NRAS protein and HRAS protein, the 12th and 13th amino acids are glycine and the 61st amino acid is glutamine.

In one aspect, the mutant RAS protein is a mutant KRAS protein, and has at least one amino acid mutation selected from the group consisting of G12A, G12C, G12D, G12S, G12V, G13D, Q61H, and Q61K, compared to the amino acid sequence of the wild-type KRAS protein.

In one aspect, the mutant RAS protein is a mutant NRAS protein, and has at least one amino acid mutation selected from the group consisting of G12C, G12D, G13D, G13V, Q61K, and Q61L, compared to the amino acid sequence of the wild-type NRAS protein.

In one aspect, the mutant RAS protein is a mutant HRAS protein, and has a G13R amino acid mutation, compared to the amino acid sequence of the wild-type HRAS protein.

Examples

The subject matter of the present invention is further described in the following Examples but is not limited thereto. All compounds and reagents were obtained from commercial suppliers or synthesized using known methods. In the present Examples, a molecular-targeted agent may be referred to as a compound for combined use.

Compound 1 ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontyne-21,1'-cyclopentane]-15-carboxamide) was produced according to the peptide synthesis method by the Fmoc method described in International Publication No. WO 2021/090855.

That is, compound 1 was produced in the following four steps of:

1) Fmoc-based peptide elongation from the N-terminal of the amino acid according to the sequence of compound 1 with carboxyl groups of (3 S)-4-(dimethylamino)-3-[9H-fluoren-9-ylmethoxycarbonyl(methyl)amino]-4-oxo-butanoic

acid supported on 2-chlorotrityl resin;
2) Peptide cleavage from the 2-chlorotrityl resin;
3) Amide cyclization through condensation of a carboxyl group released from the 2-chlorotrityl resin by the cleavage with the amino group at the peptide chain N-terminal (N-methylleucine); and 4) Purification of the compound by Preparative HPLC.

Compound 2 (2-(4-cyclopropyl-2-fluoro-anilino)-3,4-difluoro-5-[[3-fluoro-2-(methylsulfamoylamino)-4-pyridyl]methyl]benzamide) was produced according to the method described in International Publication No. WO 2021/149776. That is,

1) 2,3,4-Trifluorobenzoic acid, available from commercial suppliers, was reacted with iodine to obtain 2,3,4-trifluoro-5-iodo-benzoic acid.
2) The compound obtained in the above reaction was coupled with potassium vinyltrifluoroborate in the presence of a palladium catalyst to obtain 2,3,4-trifluoro-5-vinylbenzoic acid.
3) The compound obtained in the above reaction was coupled with 2-fluoro-4-iodoaniline to obtain 3,4-difluoro-2-(2-fluoro-4-iodo-anilino)-5-vinyl-benzoic acid.
4) The compound obtained in the above reaction was treated with sodium periodate and osmium tetroxide to obtain 3,4-difluoro-2-(2-fluoro-4-iodo-anilino)-5-formyl-benzoic acid.
5) The compound obtained in the above reaction was reacted with a diazomethyl trimethylsilane hexane solution in methanol to obtain methyl 3,4-difluoro-2-(2-fluoro-4-iodoanilino)-5-formylbenzoate.
6) The compound obtained in the above reaction was reacted with 4-methylbenzenesulfonyl hydrazide to obtain methyl 3,4-difluoro-2-(2-fluoro-4-iodoanilino)-5-[(E)-[(4-methylphenyl)sulfonylhydrazinylidene]methyl]benzoate.
7) The compound obtained in the above reaction was reacted with [2-[(2,4-dimethoxyphenyl)methylamino]-3-fluoropyridine-4-yl]boronic acid to obtain methyl 5-[[2-[(2,4-dimethoxyphenyl)methylamino]-3-fluoropyridine-4-yl]methyl]-3,4-difluoro-2-(2-fluoro-4-iodoanilino)benzoate.
8) The compound obtained in the above reaction is reacted with trifluoroacetic acid to obtain methyl 5-[(2-amino-3-fluoropyridine-4-yl)methyl]-3,4-difluoro-2-(2-fluoro-4-iodoanilino)benzoate.
9) The compound obtained in the above reaction was treated with hydrochloric acid after hydrolysis of the ester group with lithium hydroxide to obtain 5-((2-amino-3-fluoropyridine-4-yl)methyl)-3,4-difluoro-2-((2-fluoro-4-iodophenyl)amino)benzoic acid hydrochloride.
10) The compound obtained in the above reaction was reacted with EDC·HCl, HOOBt, and ammonia solution in methanol to obtain 5-((2-amino-3-fluoropyridine-4-yl)methyl)-3,4-difluoro-2-((2-fluoro-4-iodophenyl)amino)benzamide. The resultant compound was then reacted with cyclopropylzinc bromide to obtain 5-((2-amino-3-fluoropyridine-4-yl)methyl)-2-((4-cyclopropyl-2-fluorophenyl)amino)-3,4-difluorobenzamide.
11) The compound obtained in the above reaction (2.47 g, 5.74 mmol) was dissolved in anhydrous DMF (28.7 mL), pyridine (2.78 mL, 34.4 mmol) and 4-nitrophenyl methylsulfamate (4.00 g, 17.2 mmol) were added, and the mixture was stirred at 40°C for 2.5 hours. The reaction mixture was cooled to room temperature and water (24.7 mL) was added. Acetonitrile (3 mL) and water (19.8 mL) were added, followed by stirring for 10 minutes, and then the solid was collected by filtration. The obtained solid was washed with water/acetonitrile (1/1, 49.4 mL) to obtain compound 2 (2.56 g, 85%) as a colorless solid. Molecular-targeted agents (sotorasib, RMC-4550, trametinib, and cetuximab) other than compound 2 were supplied by commercial suppliers or manufacturers.

<Pharmacological Test Example>

(Example 1 In Vitro Evaluation of Inhibitory Activity in RAS Signaling)

The effects of compound 1, compound 2, RMC-4550, trametinib, and sotorasib, as well as combinations of these compounds on phosphorylation of ERK, AKT, and MEK and on binding of KRAS, NRAS, and HRAS to GTP in human cancer cells were examined by Western blotting as follows. A human cancer cell line was seeded on a three-dimensional culture plate, Prime Surface (Sumitomo Bakelite Co., Ltd.) and cultured at 37°C for 24 hours. Then, compound 1 and a compound for combined use (molecular-targeted agent) were added to the cells. The cells were cultured in a 5% carbon gas incubator at 37°C, and the cells were collected at the treatment times shown in Table 1. Table 1 shows the name of the human cancer cell line, the medium, the number of cells seeded, the concentration of compound 1, the compound for combined use, the concentration of the compound for combined use, and the treatment time. The collected cells were lysed with a lysis buffer (Cell Signaling Technology, Inc.) containing a protease inhibitor (Sigma-Aldrich) and a phosphatase inhibitor (Sigma-Aldrich). The protein concentration was determined using a BCA protein assay kit (Thermo Fisher Scientific Inc.), and protein solution 1 was prepared such that the protein will be 10 to 20 μg per sample.

RAS-GTP as an activated form was recovered from 300 to 1000 μg of the obtained protein solution 1 with Active

Ras Pull-Down and Detection Kit (Thermo Fisher Scientific Inc.) to prepare a protein solution 2. SDS-PAGE was performed using the above protein solutions 1 and 2 and a gradient gel having an acrylamide concentration of 5 to 20%, and the electrophoresed protein was transcribed to a PVDF membrane with Trans-Blot Turbo Transfer System (Bio-Rad Laboratories, Inc.). After blocking, the PVDF membrane was reacted with a primary antibody and a secondary antibody in this order. Chemiluminescence was performed with Chemi-lumi-One Super (NACALAI TESQUE, INC.), and bands were detected with ChemiDoc Touch Imaging System (Bio-Rad Laboratories, Inc.). The primary antibody and the secondary antibody used, the dilution concentration of each antibody, the reaction temperature, and the reaction time are shown in Tables 2 and 3. The results of electrophoresis images showing the results of Western blotting in which the inhibitory activity in RAS signaling was evaluated are shown in Figures 1 to 6. The terms "pERK," "pAKT," and "pMEK" refer to phosphorylated ERK, AKT, and MEK, respectively. The terms "KRAS-GTP," "NRAS-GTP," and "HRAS-GTP" refer to KRAS, NRAS, and HRAS that are bound to GTP, respectively. In Figures 1 to 6, it was shown that combined use of compound 1 and the compound (molecular-targeted agent) for combined use enhanced the inhibitory effect on the RAS signaling as compared with solo use of compound 1 or the compound (molecular-targeted agent) for combined use as a single drug.

[Table 1]

| **Cell name** | **Medium** | **Number of Cells Seeded** | **Concentration of Compound 1 (nM)** | **Compound for Combined Use** | **Concentration of Compound for Combined Use (nM)** | **Treatment Time (hour)** |
|---|---|---|---|---|---|---|
| NCI-H358 | RPMI-1640 +10% serum | $4X10^6$/Dish | 20 | Sotorasib | 30 | 4, 24, or 72 |
| NCI-H1373 | RPMI-1640 +10% serum +1mM Sodium Pyru vate +10mM HEPES +25mM D-glucose | $4X10^6$/Dish | 20 | Sotorasib | 30 | 4, 24, or 72 |
| NCI-H441 | RPMI-1640 +10% serum +1mM Sodium Pyru vate +10mM HEPES +25mM D-glucose | $3.5X10^6$/Dish | 20 | RMC-4550 | 3000 | 4, 24, or 72 |
| NCI-H441 | RPMI-1640 +10% serum +1mM Sodium Pyru vate +10mM HEPES +25mM D-glucose | $4X10^6$/Dish | 20 | Trametinib | 4 | 4, 24, or 72 |
| NCI-H358 | RPMI-1640 +10% serum | $4X10^6$/Dish | 20 | Compound 2 | 10 | 4, 24, or 72 |
| NCI-H441 | RPMI-1640 +10% serum +1mM Sodium Pyruvate +10mM HEPES +25mM D-glucose | $4X10^5$/Dish | 20 | Compound 2 | 10 | 4, 24, or 72 |

[Table 2]

| Primary Antibody | Catalog Number | Dilution **Concentration** | **Reaction Temperature** | **Reaction Time** |
|---|---|---|---|---|
| Anti-KRAS antibody | Sigma, WH0003845M1 | 1/1000 | 4°C | Overnight |
| Anti-NRAS antibody | Abcam, ab77392 | 1/1000 | 4°C | Overnight |
| Anti-HRAS antibody | Proteintech, 18295-1-AP | 1/1000 | 4°C | Overnight |

(continued)

| Primary Antibody | Catalog Number | Dilution **Concentration** | **Reaction Temperature** | **Reaction Time** |
|---|---|---|---|---|
| Anti-phospho-p44/42 MAPK (Erk1/2) (Thr202/Tyr204) Antibody | Cell Signaling Technology, #9101 | 1/1000 | 4°C | Overnight |
| Anti-p44/42 MAPK (Erk1/2) Antibody | Cell Signaling Technology, #9102 | 1/2000 | Room Temperature | 1 hour |
| Anti-phospho-Akt (Ser473) Antibody | Cell Signaling Technology, #4060 | 1/1000 | 4°C | Overnight |
| Anti-Akt Antibody | Cell Signaling Technology, #9272 | 1/2000 | Room Temperature | 1 hour |
| Anti-phospho-MEK1/2 (Ser2 17/221) Antibody | Cell Signaling Technology, #9121 | 1/1000 | 4°C | Overnight |
| Anti-MEK1/2 Antibody | Cell Signaling Technology, #9122 | 1/2000 | Room Temperature | 1 hour |

[Table 3]

| Secondary Antibody | Catalog Number | Dilution Concentration | Reaction Temperature | Reaction Time |
|---|---|---|---|---|
| Anti-rabbit IgG, HRP-linked Antibody | Cell Signaling Technology, #7074 | 1/10000 | Room Temperature | 1 hour |
| Anti-mouse IgG, HRP-linked Antibody | Cell Signaling Technology, #7076 | 1/10000 | Room Temperature | 1 hour |
| Goat TrueBlot Anti-Goat IgG HRP | Rockland, #18-8814-33 | 1/10000 | Room Temperature | 1 hour |

(Example 2 Evaluation of Cell Growth Inhibitory Activity of Compound 1 and Compound for Combined Use (molecular-targeted agent))

Human cancer cell lines were seeded on a 384-well round bottom ultra-low attachment plate at a concentration of 750 cells per well and cultured in a 5% carbon gas incubator at 37°C. After 24 hours, CellTiter-Glo 2.0 (Promega Corporation) was added to part of the wells (3 wells), and the amount of ATP ($V_0$) was determined using Envision plate readers (PerkinElmer Co., Ltd.). $V_0$ was taken as the amount of ATP at time 0. DMSO alone or compound 1 and/or the compound for combined use each were added to another three wells. Compound 1 and the compound for combined use were used by serially diluting in 8 steps at a common ratio of 3 with 300 nM and 10 or 3000 nM taken as their respective highest concentrations. In Table 4, for example, "0.14 to 300" means that the highest concentration is "300" nM, and indicates that a dilution of "300, about 100, about 33, about 11, about 3.7, about 1.2, about 0.41, or about 0.14 nM" was used. After culturing for 144 hours after addition of DMSO alone or compound 1 and/or the compound for combined use, the amount of ATP (T and V) was determined for the cells to which DMSO alone was added (DMSO-treated group) or the cells to which compound 1 and/or the compound for combined use was added (compound 1 and/or compound for combined use-treated group). Table 4 shows the name of the cancer cell line, the medium, the concentration of compound 1, the compound for combined use, and the concentration of the compound for combined use. The growth inhibition rate (Growth Inhibition %: GI%) was calculated using the following formulas. As shown in Figure 7a, Figure 8a and Figure 9a, compound 1 and the compound for combined use were shown to have a concentration-dependent growth inhibitory effect.

$$(1 - (T - V_0)/V_0) \times 100$$

(when $T < V_0$)

$$(1 - (T - V_0)/(V - V_0)) \times 100$$

(when T ≥ $V_0$)

T: ATP signal value at 144 hours for compound 1 and/or compound for combined use-treated group
V: ATP signal value at 144 hours for DMSO-treated group
$V_0$: ATP signal value for untreated group

Using the obtained GI value, analysis based on the isobologram method was performed according to the following procedure. The GI values used in evaluating the combined effect were as follows:

(1) Calculation of GI% (set GI%) for evaluating the combined effect of compound 1 and the compound for combined use, and of a compound concentration (set GI concentration) that gives the set GI%

- Test on combined use of compound 1 and RMC-4550 (NCI-H358): As the set GI%, 155% (also referred to as "GI 155%" or "GI 155") was used (the set GI concentration of compound 1 was 0.033 μM, and the set GI concentration of RMC-4550 was 0.55 μM).
- Test on combined use of compound 1 and RMC-4550 (NCI-H441): As the set GI%, 100% (also referred to as "GI 100%" or "GI 100") was used (the set GI concentration of compound 1 was 0.0055 μM, and the set GI concentration of RMC-4550 was 0.35 μM).
- Test on combined use of compound 1 and trametinib (NCI-H358): As the set GI%, 50% (also referred to as "GI 50%" or "GI 50") was used (the set GI concentration of compound 1 was 0.0046 μM, and the set GI concentration of trametinib was 0.00035 μM).

(2) Study of combinations of concentrations of compound 1 and the compound for combined use that give each set GI% in (1) and graphical visualization of the combined effect

- The abscissa represents a value obtained by dividing the concentration of the compound for combined use by the set GI concentration, and the ordinate represents a value obtained by dividing the concentration of compound 1 by the set GI concentration.
- The concentrations of the combination of compound 1 and the compound for combined use that give the set GI% in various cell lines were determined and plotted in a graph according to the values of the abscissa and the ordinate.

The results of the evaluation of cell growth inhibitory activity of compound 1 and the compound for combined use are shown in Figure 7 to Figure 9. Each of Figure 7b, Figure 8b, and Figure 9b is a graph called an isobologram. The broken line connecting " 1.0" on the ordinate and "1.0" on the abscissa of the isobologram indicates the case where the growth inhibitory effect of combined use of compound 1 and the compound for combined use was a simple addition of the growth inhibitory effects of solo use of each compound (the effects were additive). A plot below the broken line indicates that a synergistic growth inhibitory effect was exhibited when compound 1 and the compound for combined use were used in combination. The further the plot is away from the broken line, the stronger the synergistic effect is. As shown in Figure 7b, Figure 8b, and Figure 9b, it was found that the combined use of compound 1 and the compound for combined use exhibits a synergistic growth inhibitory effect because the plot is away from the broken line in each figure.

[Table 4]

| Cell name | Medium | Concentration of Compound 1 (nM) | Compound for Combined Use | Concentration of Compound for Combined Use (nM) |
|---|---|---|---|---|
| NCI-H358 | RPMI-1640+10% serum | 0.14 - 300 | RMC-4550 | 1.4 - 3000 |
| NCI-H441 | RPMI-1640+10% serum | 0.14 - 300 | RMC-4550 | 1.4 - 3000 |
| NCI-H358 | RPMI-1640+10% serum | 0.14 - 300 | Trametinib | 0.0046 - 10 |

(Example 3 Evaluation of In Vivo Antitumor Activity due to Combined Use of Compound 1 and Cetuximab)

A xenograft model to which colorectal cancer cell line LoVo (KRAS-G13D) was transplanted was used to evaluate in vivo antitumor activity due to combined use of compound 1 and cetuximab. Nude mice were subcutaneously transplanted with $5 \times 10^6$ cells per mouse, and when tumor volumes reached 200 to 300 $mm^3$, the nude mice were randomized and divided into 4 groups of 8 mice each. Compound 1 or cetuximab was each administered to a monotherapy group (8 mice per group), and compound 1 and cetuximab were administered to a two-drug administration group (8 mice

per group). The administration period was 10 days, and compound 1 was orally administered once a day, and cetuximab was intravenously administered at a rate of twice a week. Compound 1 was administered at 10 mg/kg per dose, and cetuximab was administered at 40 mg/kg per dose. Compound 1 was dissolved in 10% DMSO/5% Cremphor EL/85% distilled water and administered. Cetuximab was diluted with physiological saline and administered. The tumor growth inhibition rate (Tumor Growth Inhibition %: TGI%) was calculated using the following formulas. The results of the evaluation of in vivo antitumor activity are shown in Figure 10. As compared with a vehicle group, the agent administration groups (compound 1 monotherapy group, cetuximab monotherapy group, and compound 1 and cetuximab two-drug administration group) had the increase in tumor volume suppressed. Further, in the comparison between the monotherapy group and the two-drug administration group (combined use group), the two-drug administration group had the tumor volume decreased, which shows the effect of enhancing in vivo antitumor activity due to the combined use of compound 1 and cetuximab.

$$\text{Tumor growth inhibition rate (TGI\%)} = (1 - TV/CV) \times 100$$

$$\text{Tumor volume (mm}^3\text{)} = 1/2 \times \text{major axis (mm)} \times \text{minor axis (mm)} \times \text{minor axis (mm)}$$

Amount of change in tumor volume ($mm^3$) = tumor volume on Day 32, 36, or 39 - tumor volume on Day 29

TV: Each mean of the amount of change in tumor volume in the compound 1 monotherapy group, the cetuximab monotherapy group, or the combined use group
CV: Mean of the amount of change in tumor volume in the vehicle group

(Example 4 Evaluation of In Vivo Inhibitory Activity in RAS Signaling)

After completion of Example 3, compound 1 (10 mg/kg) and cetuximab (40 mg/kg) were re-administered to each nude mouse (n = 3); after 4 hours, the tumors were collected, frozen with liquid nitrogen and stored at -80°C. Thereafter, under cooling with liquid nitrogen, the collected tumors were crushed with Multi-beads Shocker (Yasui Kikai Corporation), and the inhibitory activity of compound 1 and cetuximab in RAS signaling in the xenograft model was evaluated based on the same protocol as in Example 1 (in vitro evaluation of inhibitory activity in RAS signaling. The primary antibody and the secondary antibody used, the dilution concentration of each antibody, the reaction time, and the reaction temperature are shown in Tables 5 and 6. The results of electrophoresis images showing the results of Western blotting in which in vivo inhibitory activity in RAS signaling was evaluated are shown in Figure 11. The term "KRAS-GTP" refers to "KRAS" bound to GTP, and the terms "pERK," "pAKT," and "pEGFR" refer to phosphorylated "ERK," "AKT," and "EGFR," respectively. Combined use of compound 1 and cetuximab was shown to decrease KRAS-GTP and pERK as compared with solo use of compound 1 or cetuximab as a single drug, indicating that inhibition of RAS signaling was enhanced.

[Table 5]

| Primary Antibody | Catalog Number | Dilution Concentration | Reaction Temperature | Reaction Time |
|---|---|---|---|---|
| Anti-KRAS antibody | Sigma, WH0003845M1 | 1/500 | 4°C | Overnight |
| Anti-phospho-p44/42 MAPK (Erk1/2) (Thr202/Tyr204) Antibody | Cell Signaling Technology, #9101 | 1/500 | 4°C | Overnight |
| Anti-p44/42 MAPK (Erk1/2) Antibody | Cell Signaling Technology, #9102 | 1/2000 | Room Temperature | 1 hour |
| Anti-phospho-Akt (Ser473) Antibody | Cell Signaling Technology, #4060 | 1/200 | 4°C | Overnight |
| Anti-Akt Antibody | Cell Signaling Technology, #9272 | 1/2000 | Room Temperature | 1 hour |
| Anti-phospho-EGF Receptor (Tyr1068) Antibody | Cell Signaling Technology, #3777 | 1/1000 | 4°C | Overnight |

(continued)

| Primary Antibody | Catalog Number | Dilution Concentration | Reaction Temperature | Reaction Time |
|---|---|---|---|---|
| Anti-EGF Receptor Antibody | Cell Signaling Technology, #4267 | 1/2000 | Room Temperature | 1 hour |

[Table 6]

| Secondary Antibody | Catalog Number | Dilution concentration | Reaction Temperature | Reaction Time |
|---|---|---|---|---|
| Anti-rabbit IgG, HRP-linked Antibody | Cell Signaling Technology, #7074 | 1/10000 | Room Temperature | 1 hour |
| Anti-mouse IgG, HRP-linked Antibody | Cell Signaling Technology. #7076 | 1/10000 | Room Temperature | 1 hour |

(Example 5 Evaluation of In Vivo Antitumor Activity due to Combined Use of Compound 1 and Bevacizumab)

A xenograft model to which pancreatic cancer line AsPC-1 (KRAS-G12D) was transplanted was used to evaluate in vivo antitumor activity due to combined use of compound 1 and bevacizumab. Nude mice were subcutaneously transplanted with $5 \times 10^6$ cells per mouse, and when tumor volumes reached 200 to 300 $mm^3$, the nude mice were randomized and divided into 4 groups of 8 mice each. Compound 1 or bevacizumab was each administered to a monotherapy group (8 mice per group), and compound 1 and bevacizumab were administered to a two-drug administration group (8 mice per group). The administration period was 17 days, and compound 1 was orally administered once a day, and bevacizumab was administered by intraperitoneal injection at a rate of twice a week. Compound 1 was administered at 10 mg/kg per dose, and bevacizumab was administered at 2.5 mg/kg per dose. Compound 1 was dissolved in 10% DMSO/5% Cremphor EL/85% distilled water and administered. Bevacizumab was diluted with physiological saline and administered. The tumor growth inhibition rate (Tumor Growth Inhibition %: TGI%) was calculated using the following formulas. The results of the evaluation of in vivo antitumor activity are shown in Figure 12. As compared with the vehicle group, the compound 1 monotherapy group and the compound 1 and bevacizumab two-drug administration group had the increase in tumor volume suppressed. On the other hand, the bevacizumab monotherapy group did not have the increase in tumor volume significantly suppressed. Further, in the comparison between the monotherapy group and the two-drug administration group (combined use group), the two-drug administration group had the tumor volume decreased, which shows the effect of enhancing in vivo antitumor activity due to the combined use of compound 1 and bevacizumab.

$$\text{Tumor growth inhibition rate (TGI\%)} = (1 - TV/CV) \times 100$$

$$\text{Tumor volume } (mm^3) = 1/2 \times \text{major axis (mm)} \times \text{minor axis (mm)} \times \text{minor axis (mm)}$$

Tumor volume change ($mm^3$) = tumor volume on or after Day 25, 29, 32, 36, or 39 - tumor volume on Day 22

TV: Mean of the amount of change in tumor volume in the compound 1 monotherapy group, the bevacizumab monotherapy group, or the combined use group
CV: Mean of the amount of change in tumor volume in the vehicle group

(Example 6 Evaluation of Cell Growth Inhibitory Activity due to Combined Use of Compound 1 and Sotorasib)

Human colorectal cancer cell line SW837 was seeded on a 96-well two-dimensional culture plate (Corning) at 10,000 cells per well (n = 3) and cultured in a 5% carbon gas incubator at 37°C. The medium used was Leibovitz's L-15 + 10% serum. Twenty-four hours after seeding, the medium was changed to a medium containing DMSO/Compound 1 30 nM/sotorasib 300 nM/Compound 1 30 nM + sotorasib 300 nM, and measurements of cell density were started every 24 hours by Icucyte ZOOM. The medium was changed to a medium containing the compound about twice a week, and

measurements were performed for 52 days. The cell growth inhibitory activity due to combined use of compound 1 and sotorasib is shown in Figure 13. In Figure 13, the ordinate represents the percentage of cultured cells covering the culture vessel's adhesive surface (cell density, cell occupancy area ratio, confluency), and the abscissa represents the number of days after starting measuring the cell density. As shown in Figure 13, the combination of compound 1 and sotorasib enhanced the growth inhibitory effect in the human colorectal cancer cell line SW837.

(Example 7 Evaluation of Cell Growth Inhibitory Activity of Compound 1 and Compound for Combined Use)

Human cancer cell lines were seeded in low adsorption U-bottom 384 plates (Sumitomo Bakelite) at 1000 cells (HCC-1171), 500 cells (NCI-H1373), 625 cells (NCI-H23), and 250 cells (UM-UC-3) per well (n=1), and cultured in a 5% carbon gas incubator at 37°C. The medium containing compound 1 and/or the compound for combined use was prepared so that when compound 1 and MRTX849 were used in combination, compound 1 was serially diluted in 9 steps at a common ratio of 3 and MRTX849 in 5 steps at a common ratio of 5, and when compound 1 and TNO155 were used in combination, compound 1 was serially diluted in 9 steps at a common ratio of 3 and TNO155 in 5 steps at a common ratio of 5, and all the set concentrations of the two agents were mixed in combination to test the combined effect (10 concentrations and 6 combinations of concentrations, including the DMSO group). After culturing for 144 hours after the addition of compound 1 and/or the compound for combined use, the amount of ATP in the compound-treated groups was measured using CellTiter Glo 2.0. The cancer cell line name, medium, compound name, and final test concentration of the compound are shown in Table 7. From the signal values obtained by CellTiter Glo 2.0, the cell growth inhibition rate (CGI%: Cell Growth Inhibition%) was calculated using the following formulas.

$$\text{Cell growth inhibition rate (CGI\%)} = (1-(TV-V0)/(CV-V0)) \times 100$$

TV: ATP signal value at 144 hours for compound-treated group
CV: ATP signal value at 144 hours for DMSO-treated group
V0: ATP signal value at 144 hours for medium only (no cell seeding)

Using the obtained CGI values, analysis based on the isobologram method was performed according to the following procedure.
The results of the evaluation of cell growth inhibitory activity of compound 1 and the compound for combined use are shown in Figure 14 and Figure 15. The x axis (abscissa) and y axis (ordinate) were the concentrations of compound 1 and the compound for combined use, respectively, and initially, the concentration of compound 1 as a single drug and the concentration of the compound for combined use as a single drug required to exert a CGI 50% value (HCC-1171, NCI-H1373, and NCI-H23) or CGI 70% value (UM-UC-3) were plotted on the x axis or y axis. The straight line connecting the plot on the x axis and the plot on the y axis indicates the case where the growth inhibitory effect of combined use of compound 1 and the compound for combined use was a simple addition of the growth inhibitory effects of solo use of each compound (the effects were additive). Next were plotted the concentrations of compound 1 and the compound for combined use required to exert the CGI values when the agents were used in combination. A plot for the combined use below the straight line connecting the plot on the x axis and the plot on the y axis means that the combination of the agents exhibited a synergistic growth inhibitory effect, and the further the plot is away from the straight line, the stronger the synergistic effect is. As shown in Figure 14 and Figure 15, it was found that the combined use of compound 1 and the compound for combined use exhibits a synergistic growth inhibitory effect because the plot is away from the straight line in each figure.

[Table 7]

| Cell name | Medium | Concentration of Compound 1 (nM) | Compound for Combined Use | Concentration of Compound for Combined Use (nM) |
|---|---|---|---|---|
| HCC-1171 | RPMI-1640 + 10 % serum + 25 mM HEPES | 0.15 - 1000 | MRTX849 | 0.48 - 300 |
| NCI-H1373 | RPMI-1640 + 10 % serum + 1 mM sodium pyruvate + 10 mM HEPES + 25 mM D-glucose | 0.15 - 1000 | MRTX849 | 0.48 - 300 |
| HCC-1171 | RPMI-1640 + 10 % serum + 25 mM HEPES | 0.15 - 1000 | TNO155 | 16 - 10000 |

(continued)

| Cell name | Medium | Concentration of Compound 1 (nM) | Compound for Combined Use | Concentration of Compound for Combined Use (nM) |
|---|---|---|---|---|
| NCI-H23 | RPMI-1640 + 10 % serum + 1 mM sodium pyruvate + 10 mM HEPES + 25 mM D-glucose | 0.15 - 1000 | TNO155 | 16 - 10000 |
| NCI-H1373 | RPMI-1640 + 10 % serum + 1 mM sodium pyruvate + 10 mM HEPES + 25 mM D-glucose | 0.15 - 1000 | TNO155 | 16 - 10000 |
| UM-UC-3 | E-MEM + 10 % serum + 0.1 m M non-essential amino acid + 1 mM sodium pyruvate | 0.15 - 1000 | TNO155 | 16 - 10000 |

(Example 8 Evaluation of In Vivo Antitumor Activity due to Combined Use of Compound 1 and Sotorasib)

The in vivo antitumor activity due to combined use of compound 1 and sotorasib was evaluated using nude mice transplanted with CTG-2579 (KRAS-G12C), a PDX (patient-derived xenograft) model of lung cancer. Nude mice were subcutaneously transplanted with tumor fragments, and when the tumor volume reached approximately 150 to 300 mm$^3$, randomization was performed and administration of compound 1 and/or the compound for combined use was started (n = 8). Compound 1 and sotorasib were orally administered once a day for an administration period of 21 days. The doses of the agents were 7.5 mg/kg for compound 1 and 100 mg/kg for sotorasib. Compound 1 was dissolved in 10% DMSO/5% Cremphor EL/85% Distilled water and sotorasib was dissolved in 10% DMSO/10% Cremphor EL/15% PEG400/2.64% HPBCD/62.36% distilled water, and administered. From the tumor volume data on the last measurement day, the tumor growth inhibition rate (Tumor Growth Inhibition %: TGI%) was calculated using the following formulas. The results of the evaluation of in vivo antitumor activity are shown in Figure 16. As compared with the vehicle group, the compound 1 monotherapy group, the sotorasib monotherapy group, and the compound 1 and sotorasib two-drug administration group had the increase in tumor volume suppressed. Further, in the comparison between the monotherapy group and the two-drug administration group (combined use group), the two-drug administration group had the tumor volume decreased, which shows the effect of enhancing in vivo antitumor activity due to the combined use of compound 1 and sotorasib.

$$\text{Tumor growth inhibition rate (TGI\%)} = (1 - TV/CV) \times 100$$

$$\text{Tumor volume (mm}^3\text{)} = 0.52 \times \text{major axis (mm)} \times \text{minor axis (mm)} \times \text{minor axis (mm)}$$

Amount of change in tumor volume (mm$^3$) = tumor volume after Day 0 - tumor volume on Day 0

TV: Mean of the amount of change in tumor volume in the compound 1 monotherapy group, the sotorasib monotherapy group, or the combined use group
CV: Mean of the amount of change in tumor volume in the vehicle group

(Example 9 Evaluation of In Vivo Inhibitory Activity in RAS Signaling due to Combined Use of Compound 1 and Sotorasib)

After completion of Example 8, compound 1 and sotorasib were re-administered at 7.5 mg/kg and 100 mg/kg, respectively, to each nude mouse (n = 3); after 4 hours, the tumors were collected, frozen with liquid nitrogen and stored at -80°C. Thereafter, under cooling with liquid nitrogen, the collected tumors (PDX samples) were crushed with Multi-beads Shocker (Yasui Kikai Corporation), and the inhibitory activity of compound 1 and sotorasib in RAS signaling in the PDX was evaluated based on the same protocol as the in vitro evaluation of inhibitory activity in RAS signaling of Example 1. The primary antibody and the secondary antibody used, the dilution concentration, the reaction time, and the reaction temperature are shown in Tables 8 and 9. The results of electrophoresis images showing the results of Western blotting in which in vivo inhibitory activity in RAS signaling was evaluated are shown in Figure 17. The term "KRAS-GTP" refers to

"KRAS" bound to GTP, and the terms "pERK" and "pAKT" refer to phosphorylated "ERK" and "AKT," respectively. Combined use of compound 1 and sotorasib was shown to decrease pERK as compared with solo use of compound 1 or sotorasib as a single drug, indicating that inhibition of MAPK signaling was enhanced.

[Table 8]

| Primary Antibody | Catalog Number | Dilution Concentration | Reaction Temperature | Reaction Time |
|---|---|---|---|---|
| Anti-KRAS antibody | Sigma, WH0003845M1 | 1/1000 | 4 °C | Overnight |
| Anti-phospho-p44/42 MAPK (Erk1/2) (Thr202/Tyr204) Antibody | Cell Signaling Technology, #9101 | 1/1000 | 4 °C | Overnight |
| Anti-p44/42 MAPK (Erk1/2) Antibody | Cell Signaling Technology, #9 102 | 1/2000 | Room Temperature | 1 hour |
| Anti-phospho-Akt (Ser473) Antibody | Cell Signaling Technology, #4060 | 1/1000 | 4 °C | Overnight |
| Anti-Akt Antibody | Cell Signaling Technology, # 9 2 7 2 | 1/2000 | Room Temperature | 1 hour |

[Table 9]

| Secondary Antibody | Catalog Number | Dilution Concentration | Reaction Temperature | Reaction Time |
|---|---|---|---|---|
| Anti-rabbit IgG, HRP-linked Antibody | Cell Signaling Technology, #7074 | 1/10000 | Room Temperature | 1 hour |
| Anti-mouse IgG, HRP-linked Antibody | Cell Signaling Technology, #7076 | 1/10000 | Room Temperature | 1 hour |

(Example 10 Evaluation of In Vivo Antitumor Activity due to Combined Use of Compound 1 and MRTX849)

A xenograft model to which lung cancer line LU65 (KRAS-G12C) or bladder cancer line UM-UC-3 (KRAS-G12C) was transplanted was used to evaluate in vivo antitumor activity due to combined use of compound 1 and MRTX849. Nude mice were subcutaneously transplanted with $5 \times 10^6$ cells per mouse, and when the tumor volume reached approximately 200 to 300 $mm^3$, the nude mice were randomized and administration of compound 1 and/or the compound for combined use was started (n = 6). Compound 1 and MRTX849 were orally administered once a day for an administration period of 45 days. The doses of the agents were 3 mg/kg for compound 1 and 100 mg/kg for MRTX849. Compound 1 was dissolved in 10% DMSO/5% Cremphor EL/85% Distilled water and MRTX849 was dissolved in 50 mM citrate buffer (pH 5.0)/10% HPBCD, and administered. From the tumor volume data on the last measurement day, the tumor growth inhibition rate (Tumor Growth Inhibition %: TGI%) was calculated using the following formulas. The results of the evaluation of in vivo antitumor activity are shown in Figure 18. In the lung cancer model (LU65) and the bladder cancer line model (UM-UC-3), as compared with the vehicle group, the MRTX849 monotherapy group and the compound 1 and MRTX849 two-drug administration group had the increase in tumor volume suppressed. The compound 1 monotherapy group did not have the increase in tumor volume suppressed. For the MRTX849 monotherapy group, although the increase in tumor volume was suppressed immediately after the start of administration, tumor regrowth was observed in a plurality of mice with continued administration. The two-drug administration group (combined use group) had a long-term inhibition of the tumor regrowth observed in the MRTX849 monotherapy group, which shows the effect of enhancing in vivo antitumor activity due to the combined use of compound 1 and MRTX849.

$$\text{Tumor growth inhibition rate (TGI\%)} = (1 - \text{TV/CV}) \times 100$$

$$\text{Tumor volume (mm}^3) = 1/2 \times \text{major axis (mm)} \times \text{minor axis (mm)} \times \text{minor axis (mm)}$$

Amount of change in tumor volume ($mm^3$) = tumor volume after Day 0 - tumor volume on Day 0

TV: Mean of the amount of change in tumor volume in the compound 1 monotherapy group, the MRTX849 monotherapy group, or the combined use group
CV: Mean of the amount of change in tumor volume in the vehicle group

(Example 11 Evaluation of In Vivo Antitumor Activity due to Combined Use of Compound 1 and TNO155)

A xenograft model to which lung cancer line NCI-H1373 (KRAS-G12C) was transplanted was used to evaluate in vivo antitumor activity due to combined use of compound 1 and TNO155. Nude mice were subcutaneously transplanted with $5 \times 10^6$ cells per mouse, and when the tumor volume reached 200 to 300 $mm^3$, the nude mice were randomized and administration of compound 1 and/or the compound for combined use was started (n = 8). Compound 1 was orally administered once a day and TNO155 was orally administered twice a day for an administration period of 35 days. The doses of the agents were 5 mg/kg for compound 1 and 5 mg/kg for TNO155. Compound 1 was dissolved in 10% DMSO/5% Cremphor EL/85% Distilled water and TNO155 was dissolved in 0.5% Tween 80/0.5% methylcellulose/99% distilled water, and administered. From the tumor volume data on the last measurement day, the tumor growth inhibition rate (Tumor Growth Inhibition %: TGI%) was calculated using the following formulas. The results of the evaluation of in vivo antitumor activity are shown in Figure 19. As compared with the vehicle group, the compound 1 monotherapy group, the TNO155 monotherapy group, and the compound 1 and TNO155 two-drug administration group had the increase in tumor volume suppressed. Further, in the comparison between the monotherapy group and the two-drug administration group (combined use group), the two-drug administration group had the tumor volume further decreased, which shows the effect of enhancing in vivo antitumor activity due to the combined use of compound 1 and TNO155.

$$\text{Tumor growth inhibition rate (TGI\%)} = (1 - TV/CV) \times 100$$

$$\text{Tumor volume (mm}^3\text{)} = 1/2 \times \text{major axis (mm)} \times \text{minor axis (mm)} \times \text{minor axis (mm)}$$

Amount of change in tumor volume ($mm^3$) = tumor volume after Day 0 - tumor volume on Day 0

TV: Mean of the amount of change in tumor volume in the compound 1 monotherapy group, the TNO155 monotherapy group, or the combined use group
CV: Mean of the amount of change in tumor volume in the vehicle group

(Example 12 Evaluation of In Vivo Antitumor Activity due to Combined Use of Compound 1 and Compound 2)

A xenograft model to which lung cancer line NCI-H441 (KRAS-G12C) or NCI-H1373 (KRAS-G12C) was transplanted was used to evaluate in vivo antitumor activity due to combined use of compound 1 and compound 2. Nude mice were subcutaneously transplanted with $5 \times 10^6$ cells per mouse, and when the tumor volume reached 200 to 300 $mm^3$, randomization was performed and administration of compound 1 and/or the compound for combined use was started (n = 5). Compound 1 and compound 2 were orally administered once a day for an administration period of 14 days. The doses of the agents were 3 mg/kg or 5 mg/kg for compound 1 and 0.25 mg/kg for compound 2. Compound 1 was dissolved in 10% DMSO/5% Cremphor EL/85% Distilled water and compound 2 was dissolved in 10% DMSO/10% Cremphor EL/15% PEG400/15% HPBCD/50% distilled water, and administered. From the tumor volume data on the last measurement day, the tumor growth inhibition rate (Tumor Growth Inhibition %: TGI%) was calculated using the following formulas. The results of the evaluation of in vivo antitumor activity are shown in Figures 20 and 21. As compared with the vehicle group, the compound 1 monotherapy group, the compound 2 monotherapy group, and the compound 1 and compound 2 two-drug administration group had the increase in tumor volume suppressed. Further, in the comparison between the monotherapy group and the two-drug administration group (combined use group), the two-drug administration group had the tumor volume further decreased, which shows the effect of enhancing in vivo antitumor activity due to the combined use of compound 1 and compound 2.

$$\text{Tumor growth inhibition rate (TGI\%)} = (1 - TV/CV) \times 100$$

$$\text{Tumor volume (mm}^3\text{)} = 1/2 \times \text{major axis (mm)} \times \text{minor axis (mm)} \times \text{minor axis (mm)}$$

Amount of change in tumor volume ($mm^3$) = tumor volume after Day 0 - tumor volume on Day 0

TV: Mean of the amount of change in tumor volume in the compound 1 monotherapy group, the compound 2 monotherapy group, or the combined use group
CV: Mean of the amount of change in tumor volume in the vehicle group

(Example 13 Evaluation of In Vivo Inhibitory Activity in RAS Signaling due to Combined Use of Compound 1 and Compound 2)

After completion of the evaluation of in vivo antitumor activity in NCI-H441 of Example 12, compound 1 and compound 2 were re-administered at 5 mg/kg and 0.25 mg/kg, respectively, to each nude mouse (n = 3); after 4 hours, the tumors were collected, frozen with liquid nitrogen and stored at -80°C. Thereafter, under cooling with liquid nitrogen, the collected xenograft samples were crushed with Multi-beads Shocker (Yasui Kikai Corporation), and the inhibitory activity of compound 1 and compound 2 in RAS signaling in the xenografts was evaluated based on the same protocol as in Example 1 (in vitro evaluation of inhibitory activity in RAS signaling). The primary antibody and the secondary antibody used, the dilution concentration, the reaction time, and the reaction temperature are shown in Tables 10 and 11. The results of electrophoresis images showing the results of Western blotting in which in vivo inhibitory activity in RAS signaling was evaluated are shown in Figure 22. The term "KRAS-GTP" refers to "KRAS" bound to GTP, and the terms "pERK," "pAKT," and "pMEK" refer to phosphorylated "ERK," "AKT," and "MEK," respectively. Combined use of compound 1 and compound 2 was shown to decrease pERK and pMEK as compared with solo use of compound 1 or compound 2 as a single drug, indicating that inhibition of MAPK signaling was enhanced.

[Table 10]

| Primary Antibody | Catalog Number | Dilution Concentration | Reaction Temperature | Reaction Time |
|---|---|---|---|---|
| Anti-KRAS antibody | Sigma, WH0003845M1 | 1/1000 | 4 °C | Overnight |
| Anti-phospho-p44/42 MAPK (Erk1/2) (Thr202/Tyr204) Antibody | Cell Signaling Technology, #9101 | 1/1000 | 4 °C | Overnight |
| Anti-p44/42 MAPK (Erk1/2) Antibody | Cell Signaling Technology, #9102 | 1/2000 | Room Temperature | 1 hour |
| Anti-phospho-Akt (Ser473) Antibody | Cell Signaling Technology, #4060 | 1/1000 | 4 °C | Overnight |
| Anti-Akt Antibody | Cell Signaling Technology, #9272 | 1/2000 | Room Temperature | 1 hour |
| Anti-phospho-MEK1/2 (Ser217/221) Antibody | Cell Signaling Technology, # 9121 | 1/1000 | 4 °C | Overnight |
| Anti-MEK1/2 Antibody | Cell Signaling Technology, #9122 | 1/2000 | Room Temperature | 1 hour |

[Table 11]

| Secondary Antibody | Catalog Number | Dilution Concentration | Reaction Temperature | Reaction Time |
|---|---|---|---|---|
| Anti-rabbit IgG, HRP-linked Antibody | Cell Signaling Technology, #7074 | 1 / 10000 | Room Temperature | 1 hour |
| Anti-mouse IgG, HRP-linked Antibody | Cell Signaling Technology, #7076 | 1 / 10000 | Room Temperature | 1 hour |

**Claims**

1. A medicine for treating or preventing cancer, the medicine comprising a first active ingredient and being used in combination with a second active ingredient, wherein the first active ingredient is a compound represented by the following formula (1) or a salt thereof, or a solvate thereof, and the second active ingredient is a molecular-targeted agent:

[Formula 1]

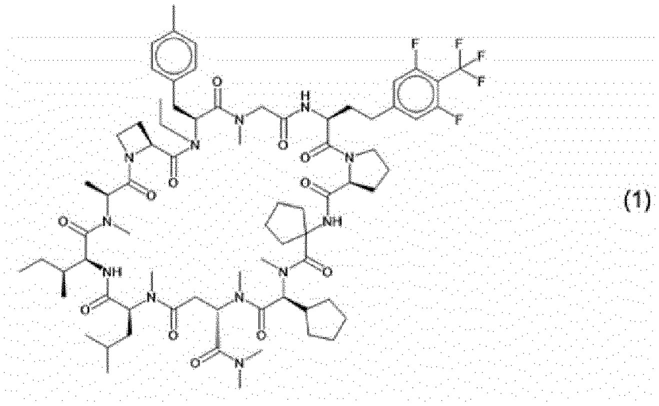

(1)

2. A medicine for treating or preventing cancer, the medicine comprising a first active ingredient and being used in combination with a second active ingredient, wherein the first active ingredient is a molecular-targeted agent, and the second active ingredient is a compound represented by the following formula (1) or a salt thereof, or a solvate thereof:

[Formula 2]

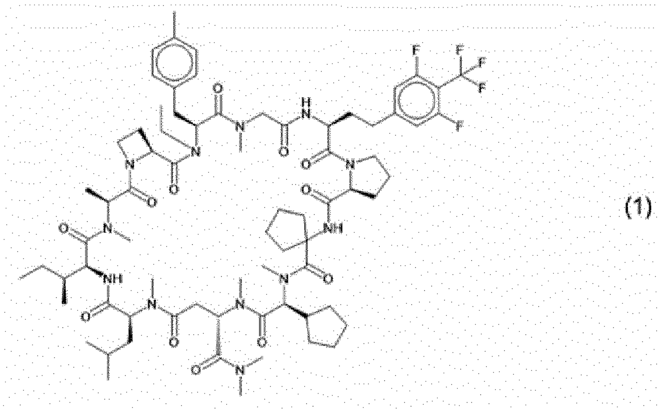

(1)

3. The medicine according to claim 1 or 2, wherein the first active ingredient and the second active ingredient are provided as a kit.

4. The medicine according to any one of claims 1 to 3, wherein the combined use of the first active ingredient and the second active ingredient is simultaneous administration or separate administration.

5. The medicine according to any one of claims 1 to 4, wherein the first active ingredient and the second active ingredient are used in combination as a combination drug.

6. The medicine according to any one of claims 1 to 5, wherein the molecular-targeted agent is a MAPK/ERK-pathway inhibitor.

7. The medicine according to any one of claims 1 to 6, wherein the molecular-targeted agent is at least one selected from the group consisting of an EGFR inhibitor, a VEGF inhibitor, an SHP2 inhibitor, a KRAS-G12C inhibitor, and a MEK

inhibitor.

**8.** The medicine according to any one of claims 1 to 7, wherein the molecular-targeted agent is an EGFR inhibitor, a VEGF inhibitor, an SHP2 inhibitor, a KRAS-G12C inhibitor, or a MEK inhibitor.

**9.** The medicine according to claim 7 or 8, wherein the EGFR inhibitor is at least one selected from the group consisting of gefitinib, erlotinib, afatinib, osimertinib, lapatinib, or a salt thereof, or a solvate thereof, and an anti-EGFR antibody.

**10.** The medicine according to any one of claims 7 to 9, wherein the EGFR inhibitor is an anti-EGFR antibody.

**11.** The medicine according to claim 9 or 10, wherein the anti-EGFR antibody has a heavy chain variable region comprising an amino acid sequence of SEQ ID NO: 1 and a light chain variable region comprising an amino acid sequence of SEQ ID NO: 2.

**12.** The medicine according to any one of claims 9 to 11, wherein the anti-EGFR antibody is cetuximab.

**13.** The medicine according to any one of claims 7 to 12, wherein the VEGF inhibitor is an anti-VEGF antibody.

**14.** The medicine according to claim 13, wherein the anti-VEGF antibody has a heavy chain variable region comprising an amino acid sequence of SEQ ID NO: 3 and a light chain variable region comprising an amino acid sequence of SEQ ID NO: 4.

**15.** The medicine according to claim 13 or 14, wherein the anti-VEGF antibody is bevacizumab.

**16.** The medicine according to any one of claims 7 to 15, wherein the SHP2 inhibitor is at least one selected from the group consisting of RMC4550, TNO155, RLY1971, SHP099, NSC-87877, and a salt thereof, and a solvate thereof.

**17.** The medicine according to any one of claims 7 to 16, wherein the SHP2 inhibitor is at least one selected from the group consisting of RMC4550, TNO155, and a salt thereof, and a solvate thereof.

**18.** The medicine according to any one of claims 7 to 17, wherein the KRAS-G12C inhibitor is at least one selected from the group consisting of sotorasib, MRTX849, and a salt thereof, and a solvate thereof.

**19.** The medicine according to any one of claims 7 to 18, wherein the MEK inhibitor is at least one selected from the group consisting of a compound represented by the following formula (2), trametinib, selumetinib, CH4987655, and a salt thereof, and a solvate thereof:

[Formula 3]

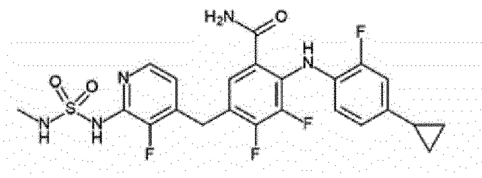

(2)

**20.** The medicine according to any one of claims 7 to 19, wherein the MEK inhibitor is a compound represented by the following formula (2), trametinib, or a salt thereof, or a solvate thereof:

[Formula 4]

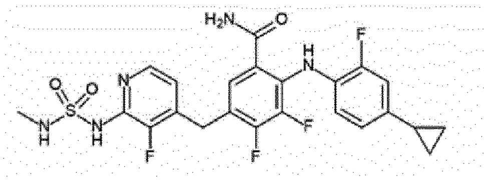

(2)

**21.** The medicine according to any one of claims 1 to 20, wherein the compound represented by the formula (1) or the salt

thereof, or the solvate thereof is a solvate of the compound represented by the formula (1).

**22.** The medicine according to claim 21, wherein the solvate is a hydrate.

**23.** The medicine according to any one of claims 1 to 20, wherein the compound represented by the formula (1) or the salt thereof, or the solvate thereof is a compound represented by the formula (1).

**24.** The medicine according to any one of claims 1 to 23, wherein the cancer is a solid cancer or a blood cancer.

**25.** The medicine according to any one of claims 1 to 24, wherein the cancer is at least one selected from the group consisting of lung cancer, esophageal cancer, gastric cancer, colorectal cancer, uterine cancer, ovarian cancer, pancreatic cancer, bladder cancer, thyroid cancer, skin cancer, leukemia, malignant lymphoma, and multiple myeloma.

**26.** The medicine according to any one of claims 1 to 25, wherein the cancer is a cancer associated with an abnormality in a RAS gene or MAPK/ERK-pathway.

# Fig.1

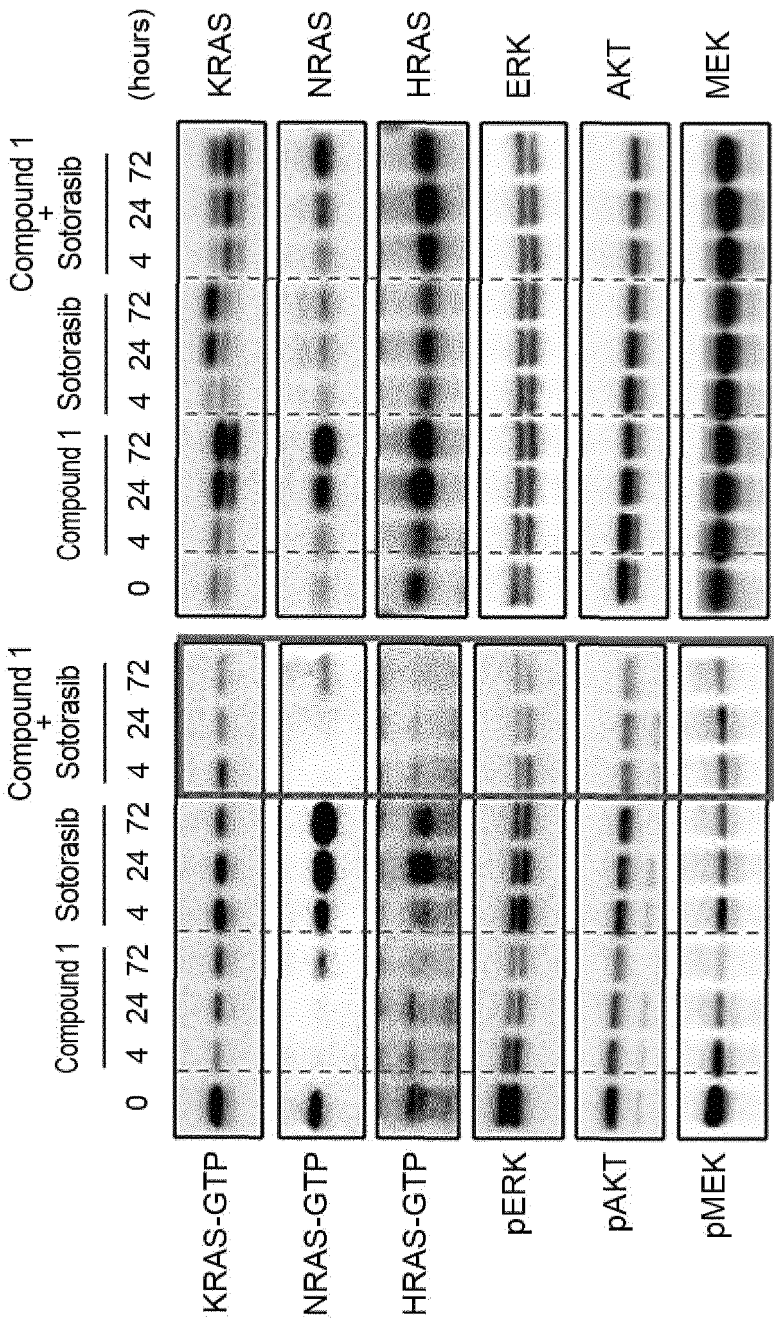

Compound 1
Sotorasib
Compound 1 + Sotorasib
0
4
24
72
(hours)
KRAS-GTP
NRAS-GTP
HRAS-GTP
pERK
pAKT
pMEK
KRAS
NRAS
HRAS
ERK
AKT
MEK

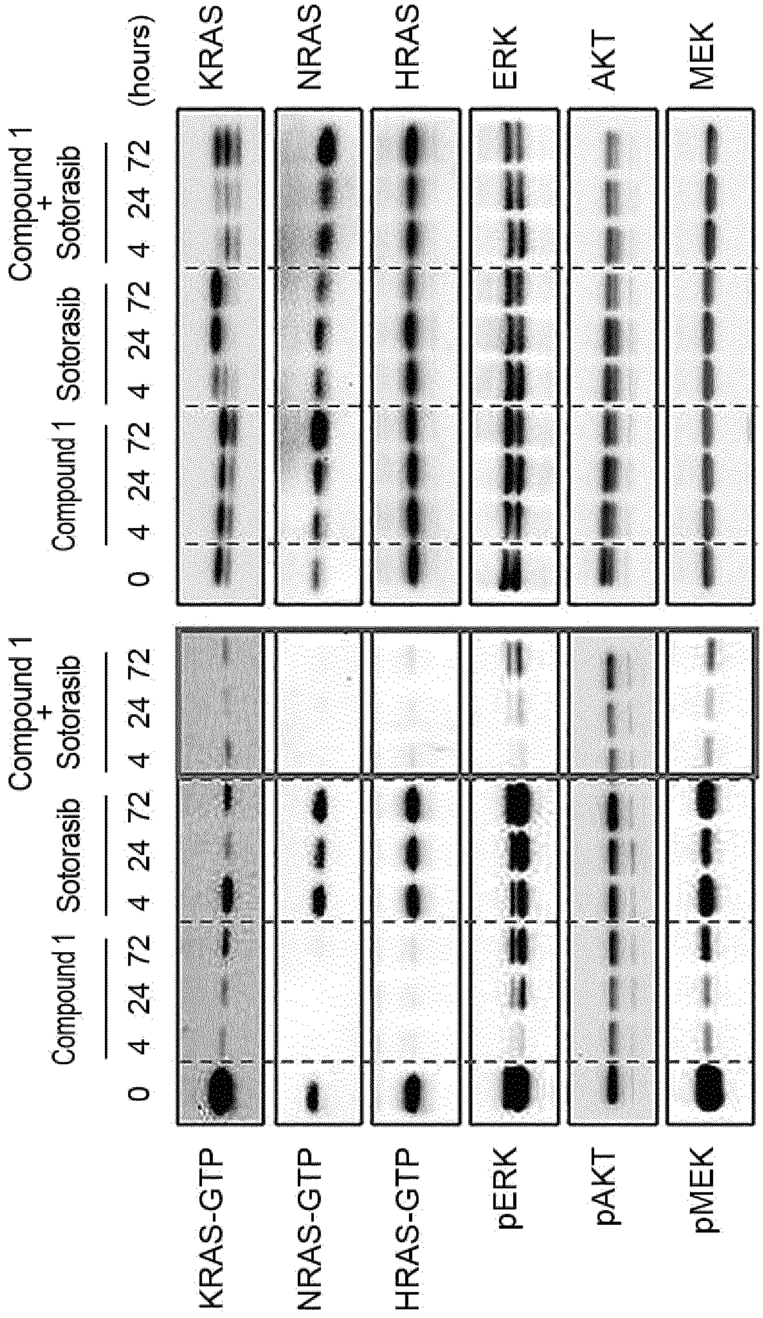
Compound 1
Sotorasib
Compound 1 + Sotorasib
0 4 24 72 4 24 72 4 24 72
KRAS-GTP
NRAS-GTP
HRAS-GTP
pERK
pAKT
pMEK
Compound 1
Sotorasib
Compound 1 + Sotorasib
0 4 24 72 4 24 72 4 24 72 (hours)
KRAS
NRAS
HRAS
ERK
AKT
MEK

*Fig.2*

Fig.3

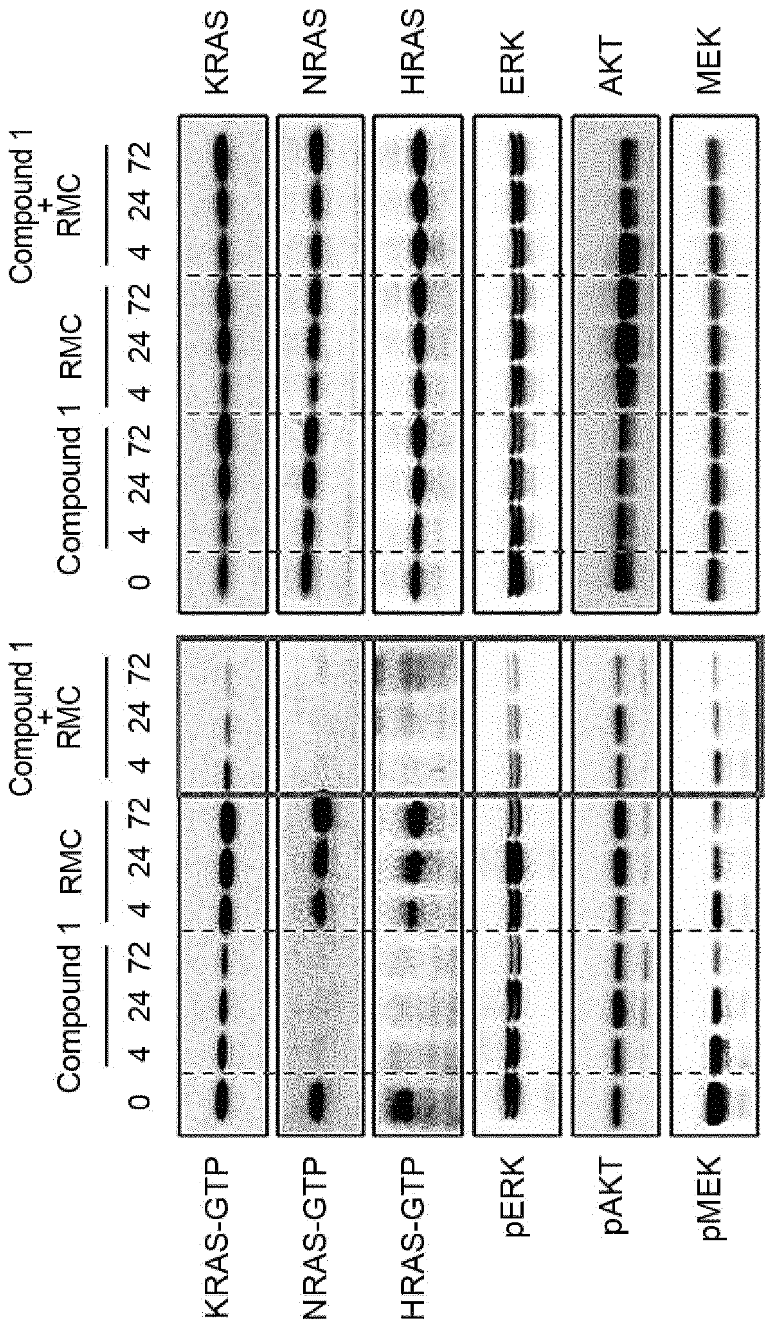

0
4
24
72
Compound 1
RMC
Compound 1 + RMC
KRAS-GTP
NRAS-GTP
HRAS-GTP
pERK
pAKT
pMEK
KRAS
NRAS
HRAS
ERK
AKT
MEK

Fig.4

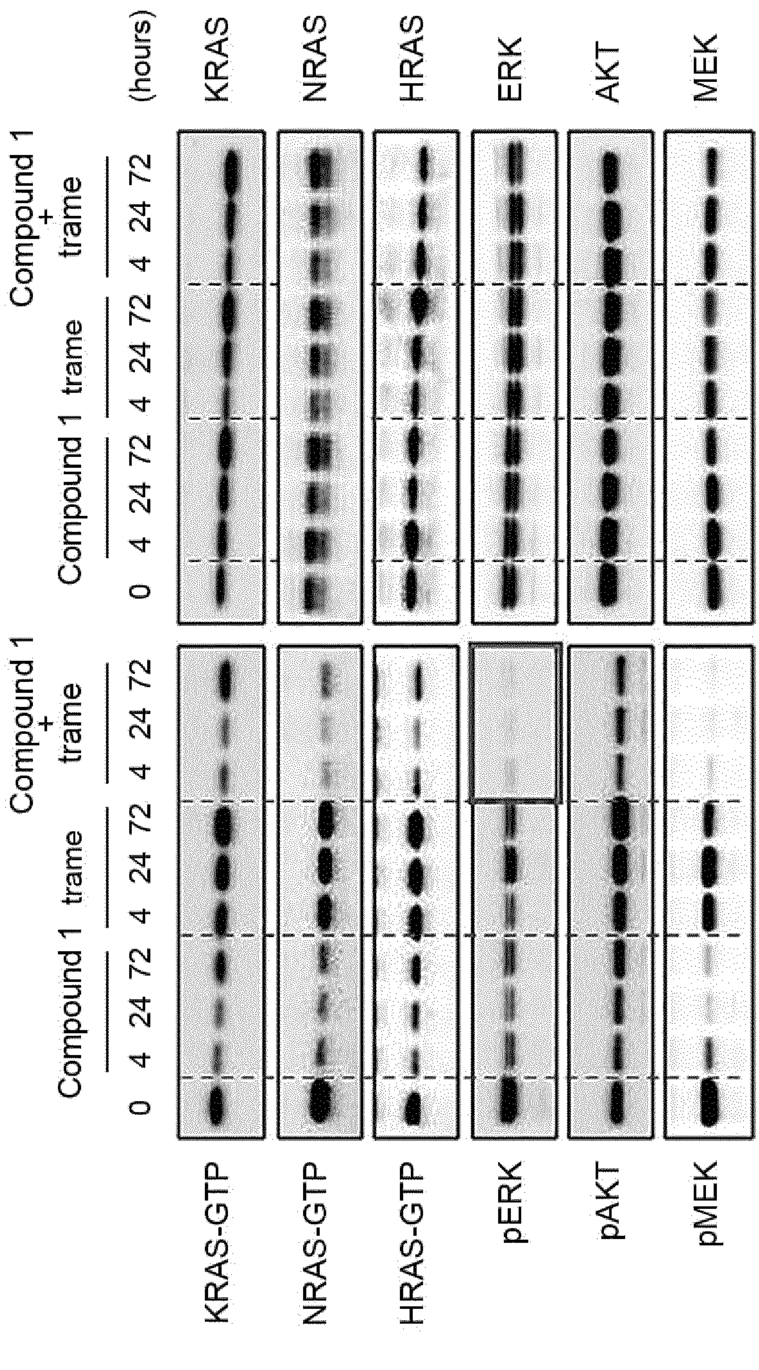

0
4
24
72
Compound 1
trame
Compound 1 + trame
(hours)
KRAS-GTP
NRAS-GTP
HRAS-GTP
pERK
pAKT
pMEK
KRAS
NRAS
HRAS
ERK
AKT
MEK

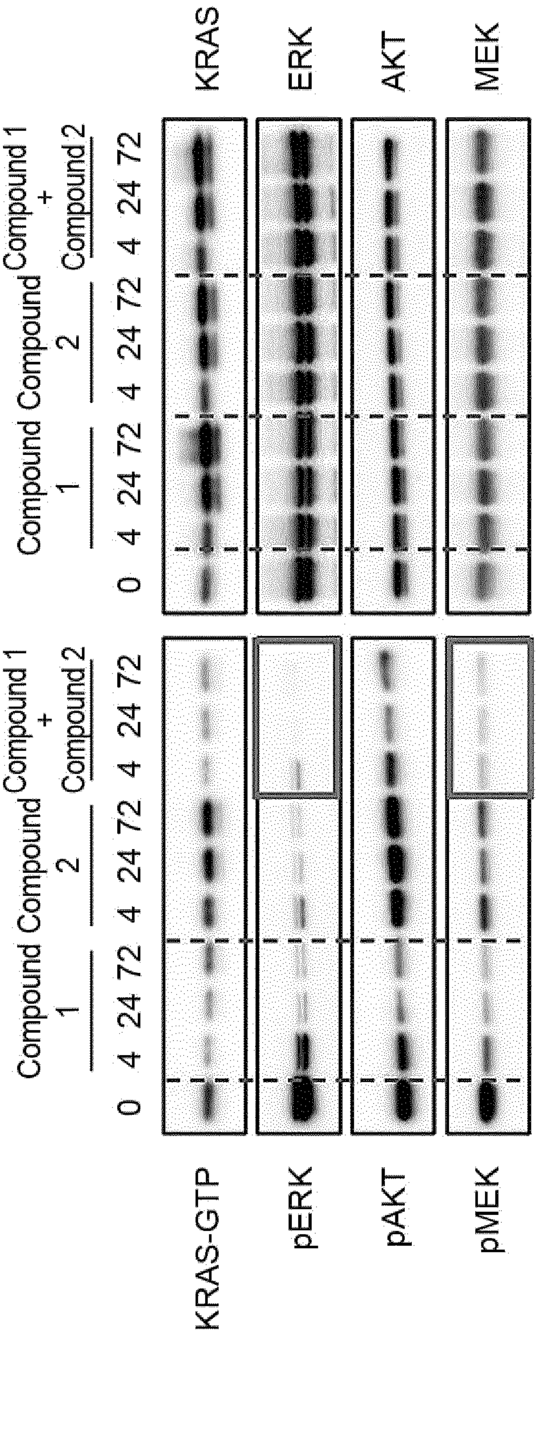
Compound 1
Compound 2
Compound 1 + Compound 2
0 4 24 72 4 24 72 4 24 72
KRAS-GTP
pERK
pAKT
pMEK
KRAS
ERK
AKT
MEK

*Fig.5*

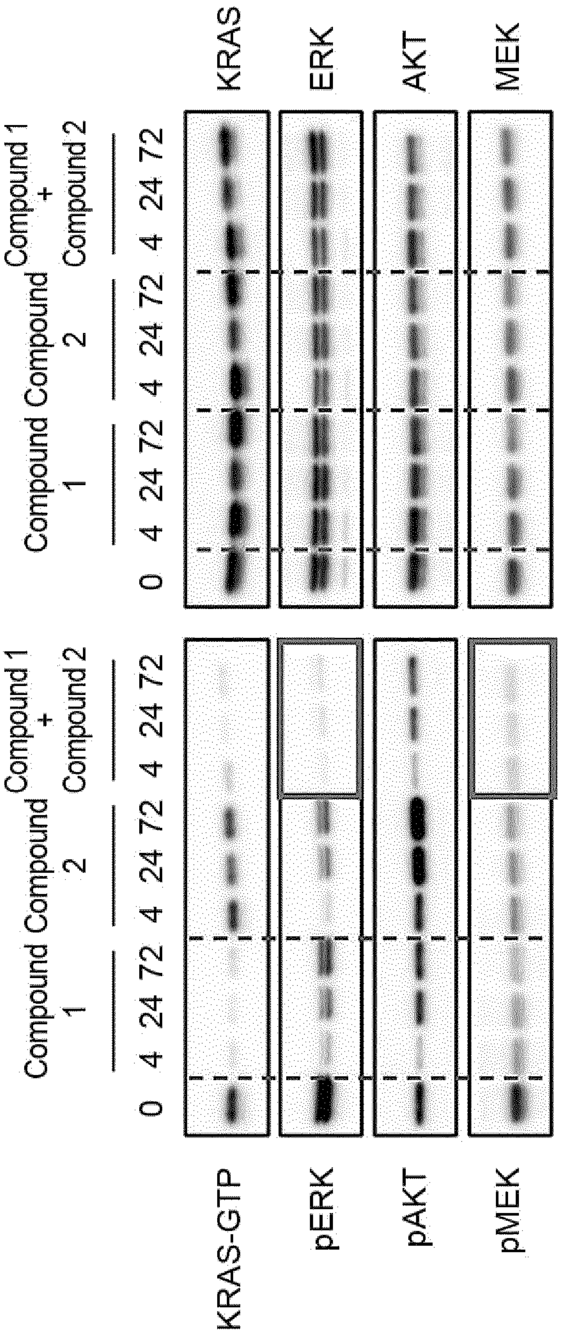
Compound 1
Compound 2
Compound 1 + Compound 2
0 4 24 72 4 24 72 4 24 72
KRAS-GTP
pERK
pAKT
pMEK
KRAS
ERK
AKT
MEK

Fig.6

## Fig.7

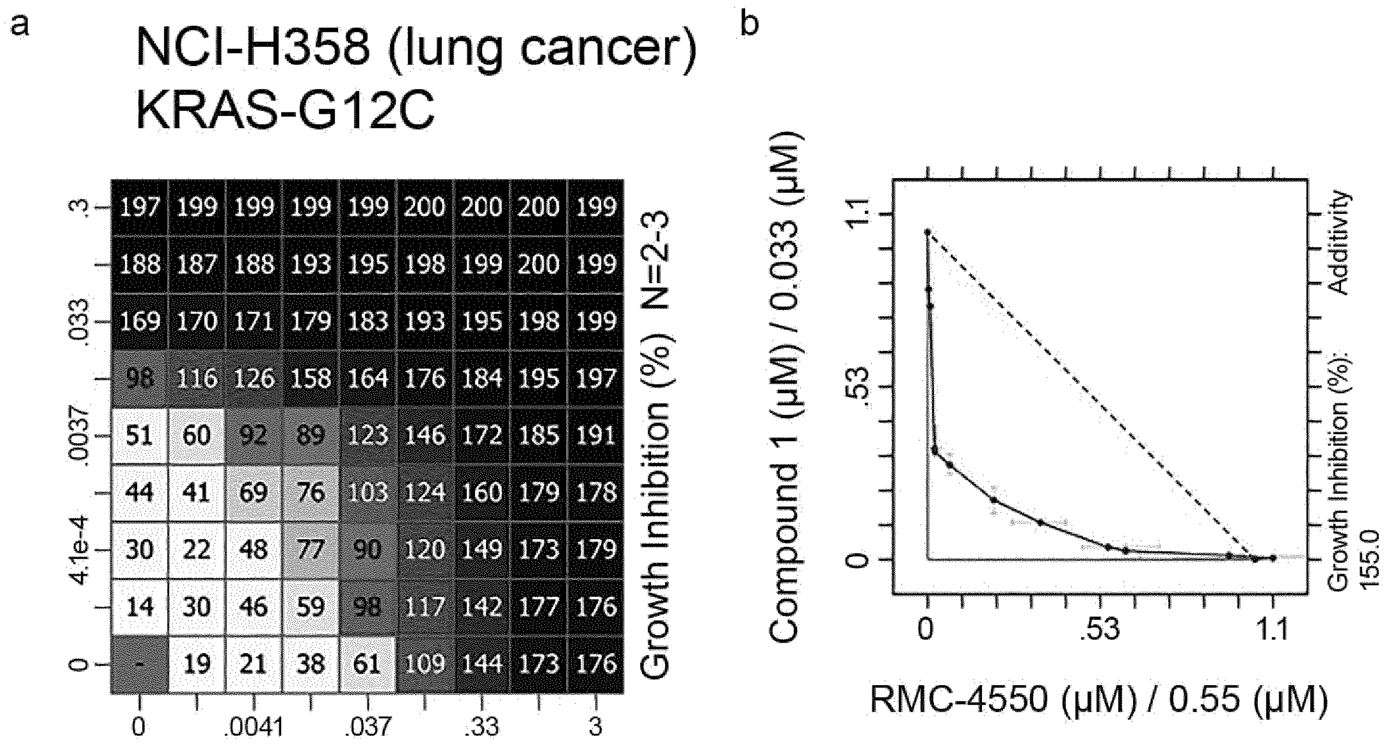
a
NCI-H358 (lung cancer)
KRAS-G12C
197 199 199 199 199 200 200 200 199
188 187 188 193 195 198 199 200 199
169 170 171 179 183 193 195 198 199
98 116 126 158 164 176 184 195 197
51 60 92 89 123 146 172 185 191
44 41 69 76 103 124 160 179 178
30 22 48 77 90 120 149 173 179
14 30 46 59 98 117 142 177 176
- 19 21 38 61 109 144 173 176
.3
.033
.0037
4.1e-4
0
0
.0041
.037
.33
3
Growth Inhibition (%) N=2-3
b
Compound 1 (μM) / 0.033 (μM)
1.1
.53
0
RMC-4550 (μM) / 0.55 (μM)
Additivity
Growth Inhibition (%):
155.0

**Fig.8**

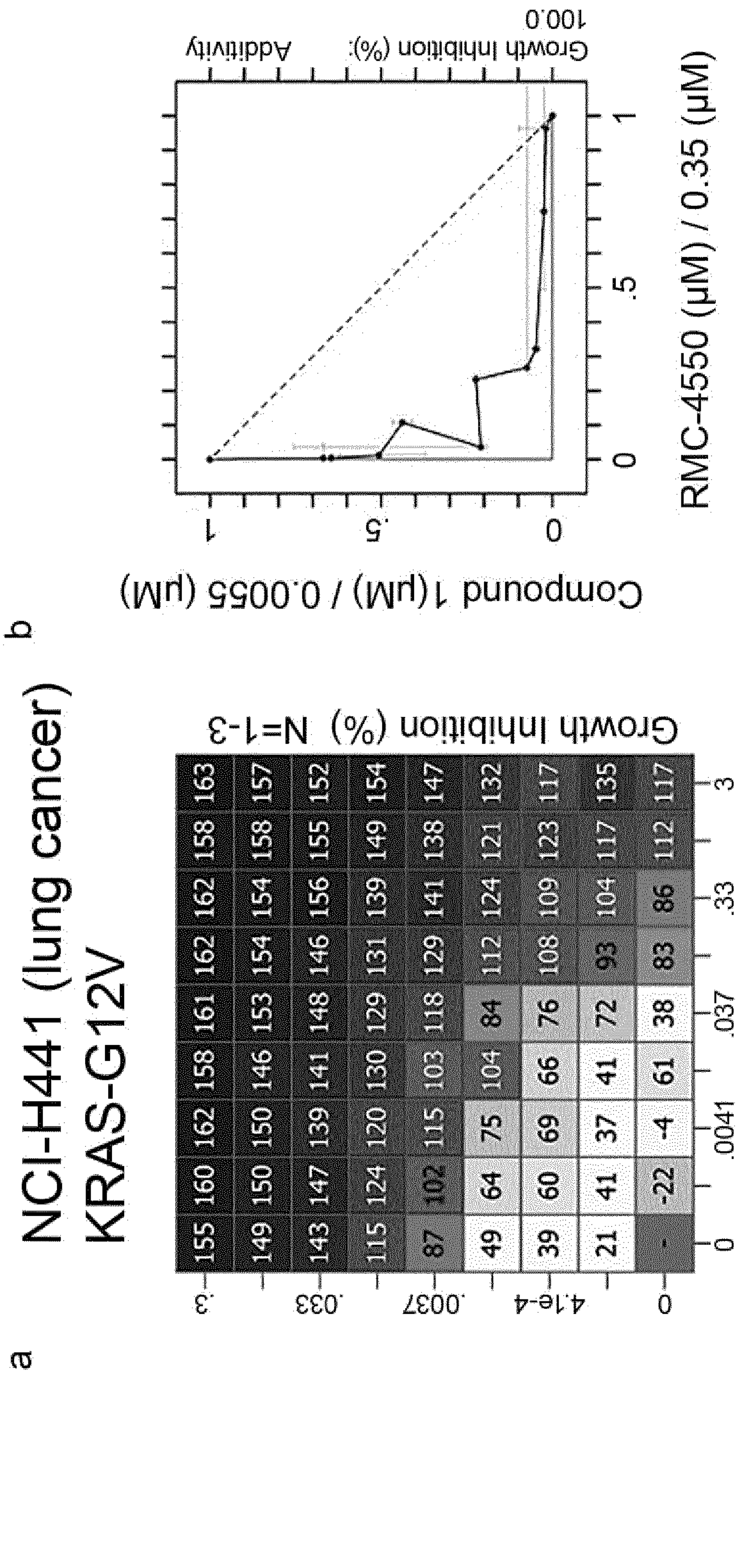
a
NCI-H441 (lung cancer)
KRAS-G12V
.3
155 160 162 158 161 162 162 158 163
149 150 150 146 153 154 154 158 157
.033
143 147 139 141 148 146 156 155 152
115 124 120 130 129 131 139 149 154
.0037
87 102 115 103 118 129 141 138 147
49 64 75 104 84 112 124 121 132
4.1e-4
39 60 69 66 76 108 109 123 117
21 41 37 41 72 93 104 117 135
0
- -22 -4 61 38 83 86 112 117
0
.0041
.037
.33
3
Growth Inhibition (%) N=1-3
b
Compound 1(μM) / 0.0055 (μM)
1
.5
0
RMC-4550 (μM) / 0.35 (μM)
0
.5
1
Growth Inhibition (%):
100.0
Additivity

# Fig.9

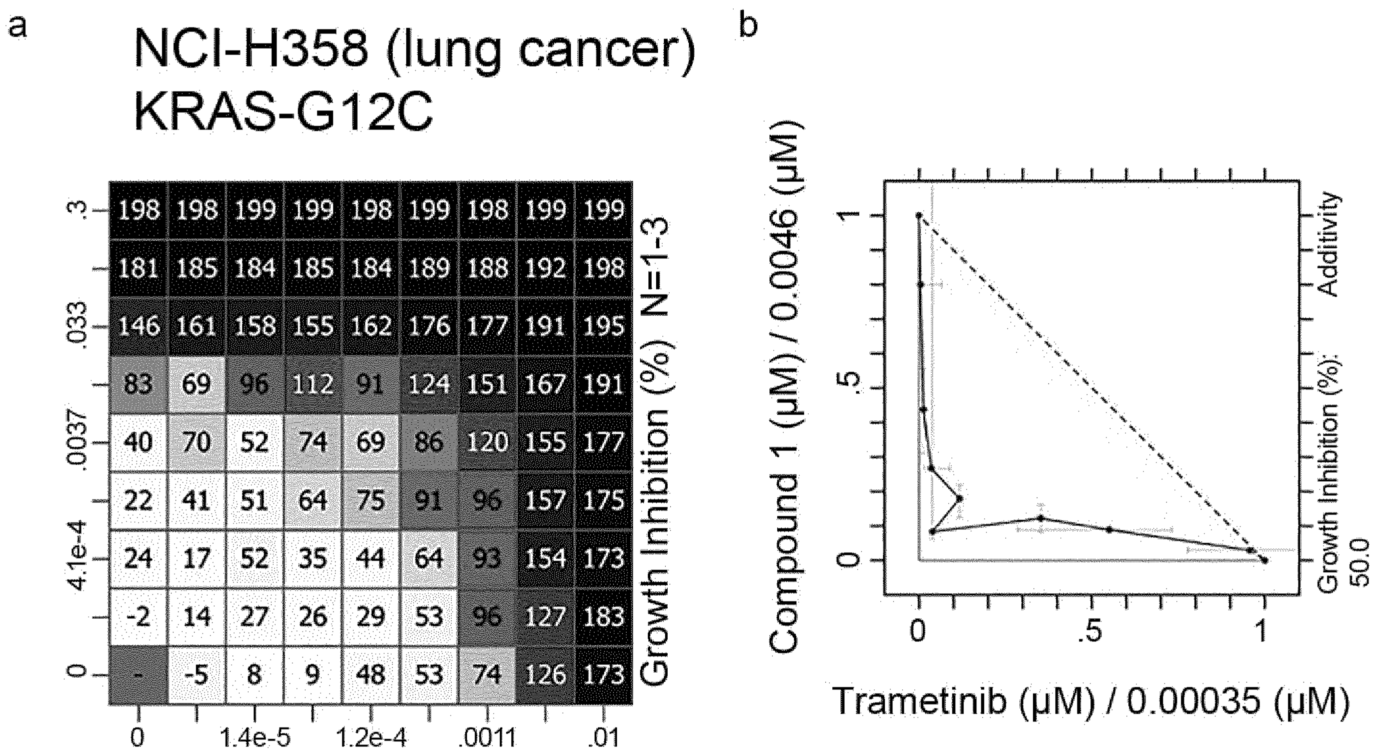
a
NCI-H358 (lung cancer)
KRAS-G12C
198 198 199 199 198 199 198 199 199
181 185 184 185 184 189 188 192 198
146 161 158 155 162 176 177 191 195
83 69 96 112 91 124 151 167 191
40 70 52 74 69 86 120 155 177
22 41 51 64 75 91 96 157 175
24 17 52 35 44 64 93 154 173
-2 14 27 26 29 53 96 127 183
- -5 8 9 48 53 74 126 173
.3
.033
.0037
4.1e-4
0
1.4e-5
1.2e-4
.0011
.01
Growth Inhibition (%) N=1-3
b
Compound 1 (μM) / 0.0046 (μM)
1
.5
0
Trametinib (μM) / 0.00035 (μM)
Additivity
Growth Inhibition (%):
50.0

*Fig.10*

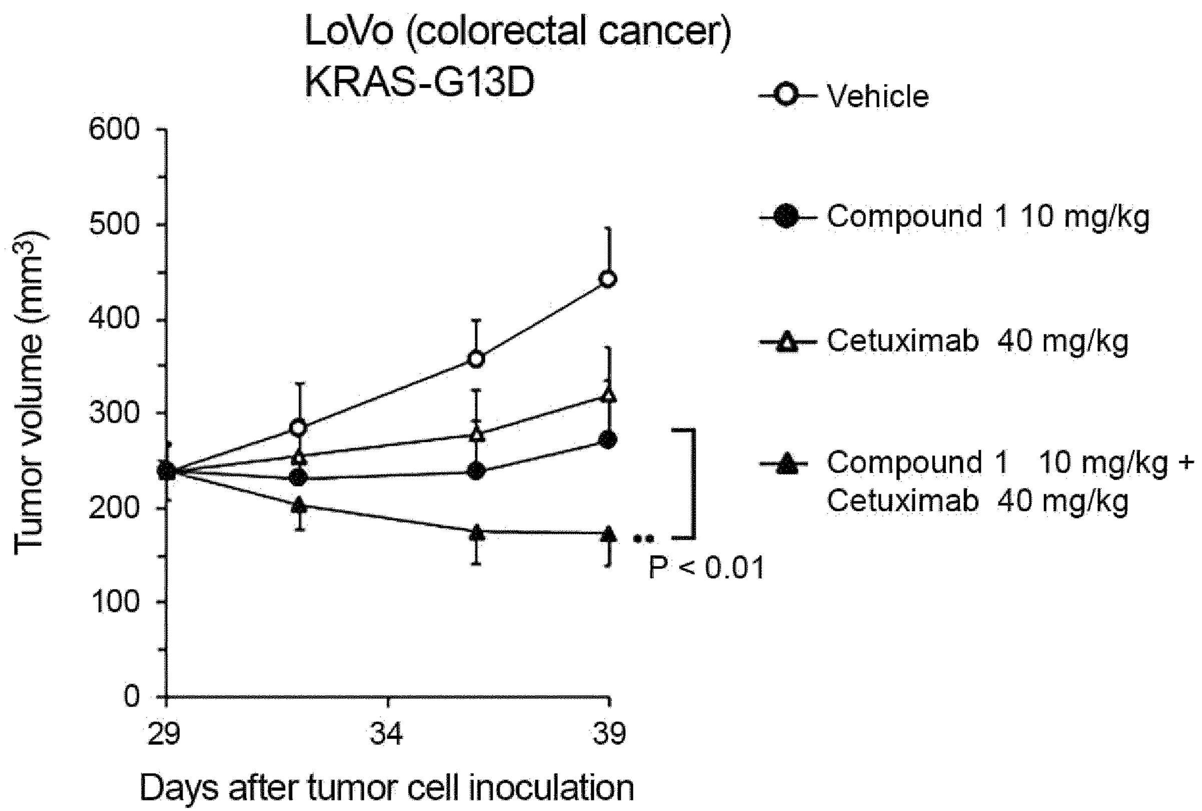
LoVo (colorectal cancer)
KRAS-G13D
Tumor volume (mm³)
600
500
400
300
200
100
0
29
34
39
Days after tumor cell inoculation
P < 0.01
Vehicle
Compound 1 10 mg/kg
Cetuximab 40 mg/kg
Compound 1 10 mg/kg +
Cetuximab 40 mg/kg

Fig. 11

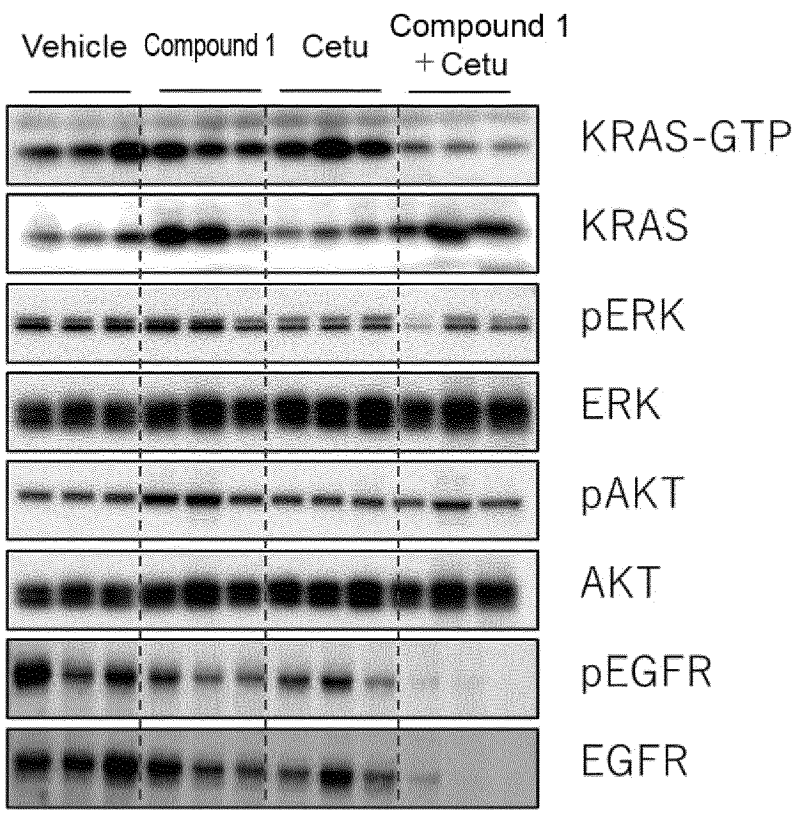


The agents were re-administered on the last day of measurement, and after 4 hours, the tumors were collected

Fig.12

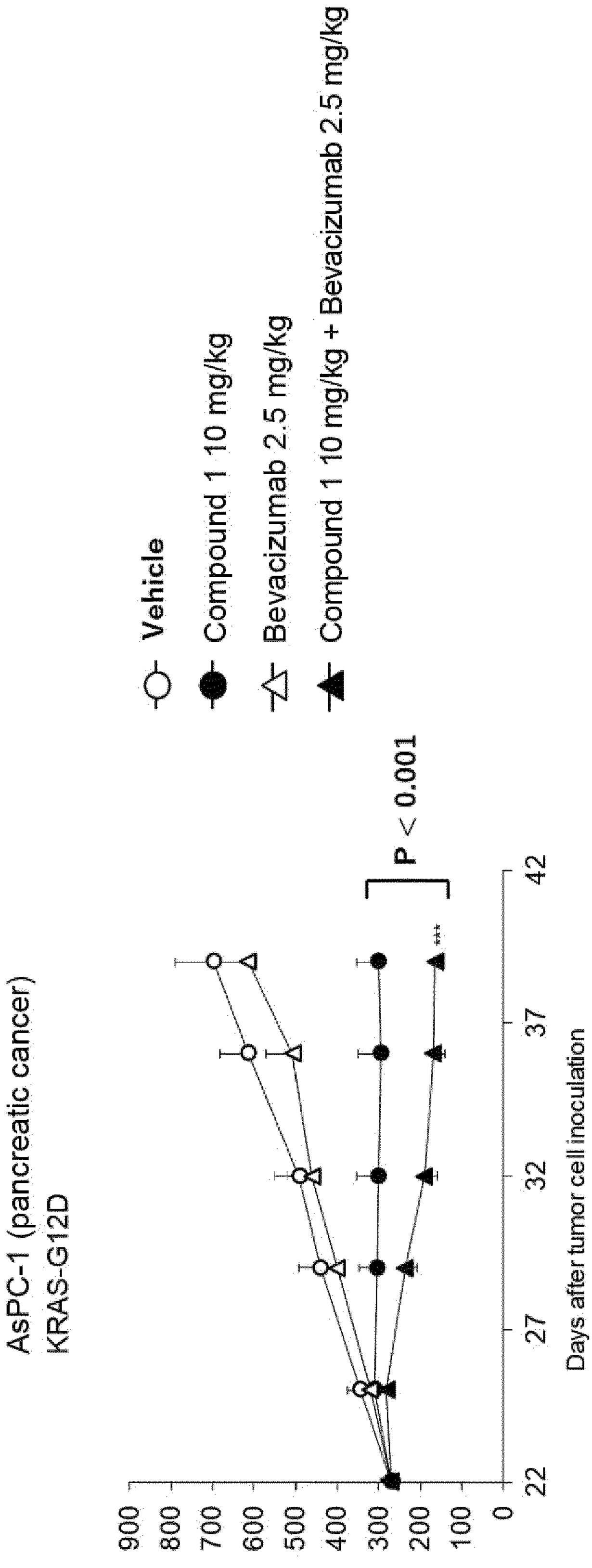
AsPC-1 (pancreatic cancer)
KRAS-G12D
Tumor volume (mm³)
900
800
700
600
500
400
300
200
100
0
22
27
32
37
42
Days after tumor cell inoculation
***
P < 0.001
Vehicle
Compound 1 10 mg/kg
Bevacizumab 2.5 mg/kg
Compound 1 10 mg/kg + Bevacizumab 2.5 mg/kg

Fig.13

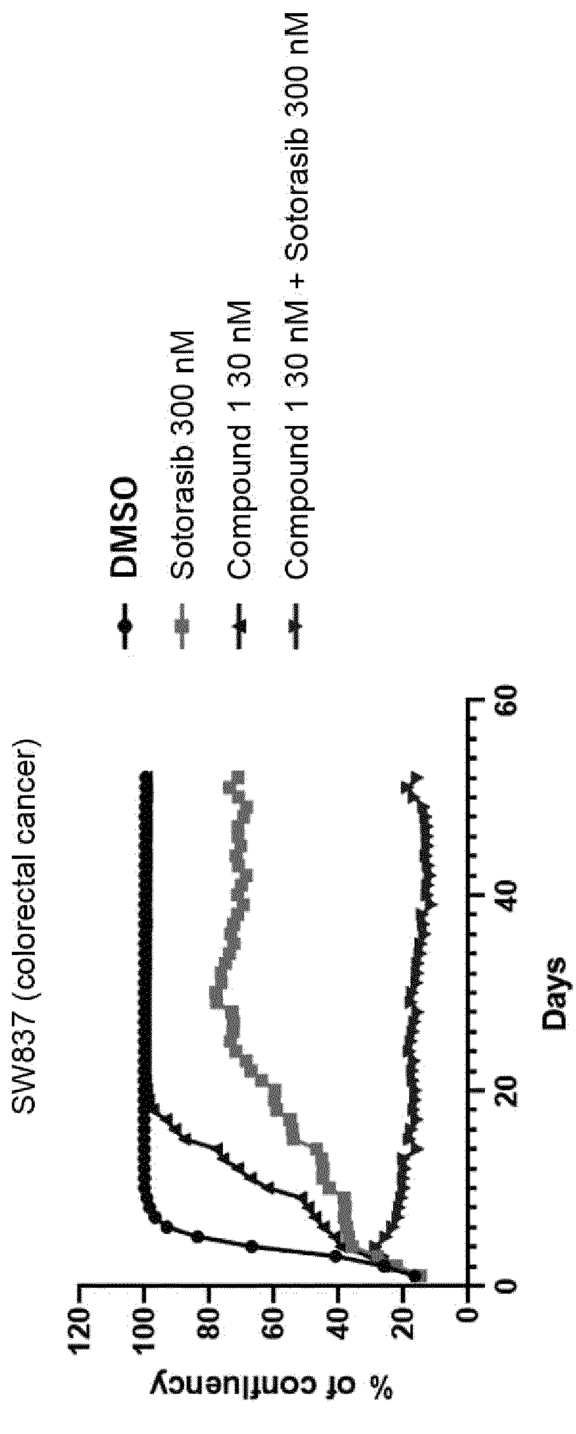
SW837 (colorectal cancer)
% of confluency
120
100
80
60
40
20
0
0
20
40
60
Days
DMSO
Sotorasib 300 nM
Compound 1 30 nM
Compound 1 30 nM + Sotorasib 300 nM

# Fig.14

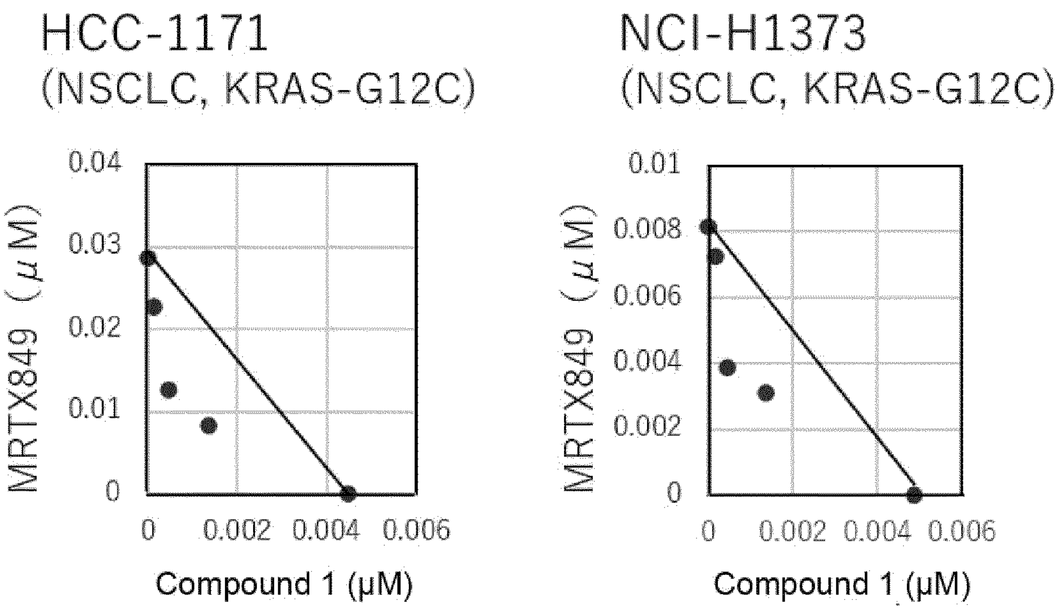
HCC-1171
(NSCLC, KRAS-G12C)
MRTX849 (μM)
0.04
0.03
0.02
0.01
0
0
0.002
0.004
0.006
Compound 1 (μM)
NCI-H1373
(NSCLC, KRAS-G12C)
MRTX849 (μM)
0.01
0.008
0.006
0.004
0.002
0
0
0.002
0.004
0.006
Compound 1 (μM)

**Fig.15**

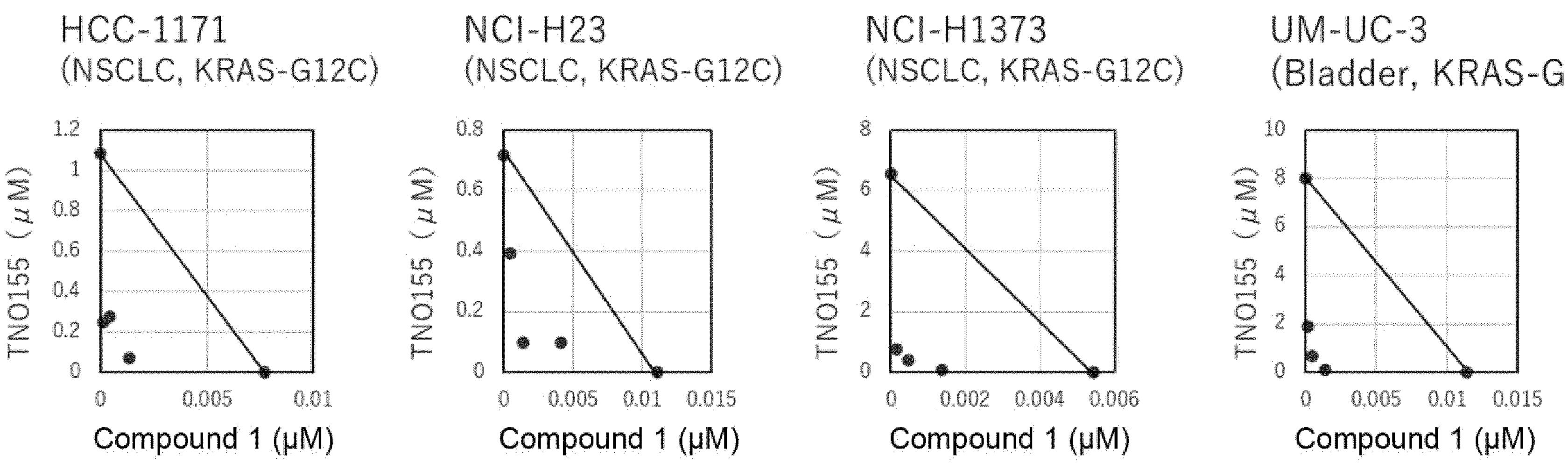
HCC-1171
(NSCLC, KRAS-G12C)
TNO155 (μM)
1.2
1
0.8
0.6
0.4
0.2
0
0
0.005
0.01
Compound 1 (μM)
NCI-H23
(NSCLC, KRAS-G12C)
TNO155 (μM)
0.8
0.6
0.4
0.2
0
0
0.005
0.01
0.015
Compound 1 (μM)
NCI-H1373
(NSCLC, KRAS-G12C)
TNO155 (μM)
8
6
4
2
0
0
0.002
0.004
0.006
Compound 1 (μM)
UM-UC-3
(Bladder, KRAS-G12C)
TNO155 (μM)
10
8
6
4
2
0
0
0.005
0.01
0.015
Compound 1 (μM)

Fig.16

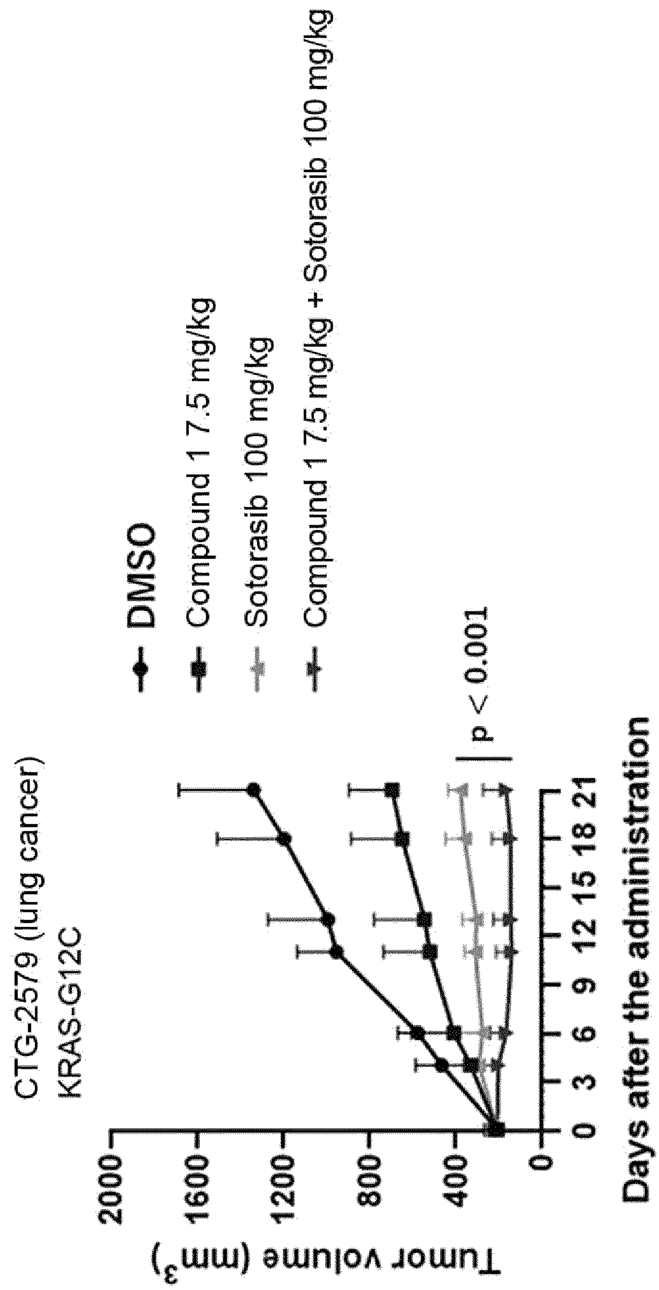
CTG-2579 (lung cancer)
KRAS-G12C
Tumor volume (mm3)
2000
1600
1200
800
400
0
0 3 6 9 12 15 18 21
Days after the administration
p < 0.001
DMSO
Compound 1 7.5 mg/kg
Sotorasib 100 mg/kg
Compound 1 7.5 mg/kg + Sotorasib 100 mg/kg

Fig.17

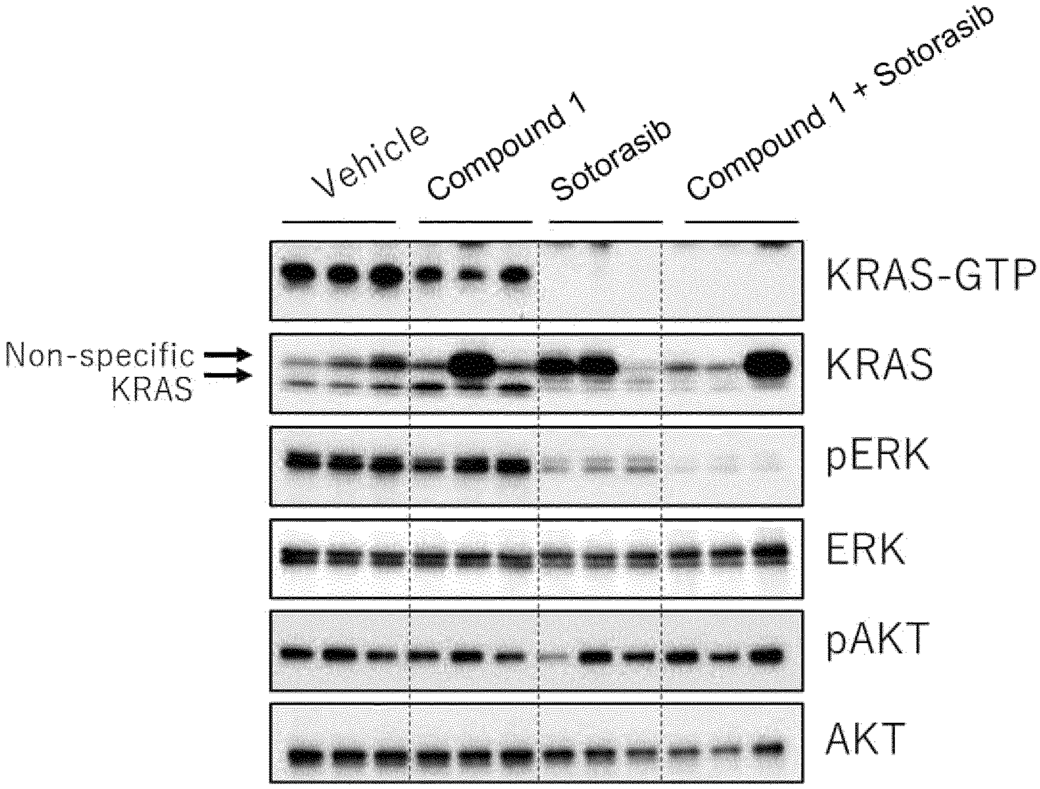
Vehicle
Compound 1
Sotorasib
Compound 1 + Sotorasib
KRAS-GTP
Non-specific
KRAS
KRAS
pERK
ERK
pAKT
AKT

# Fig.18

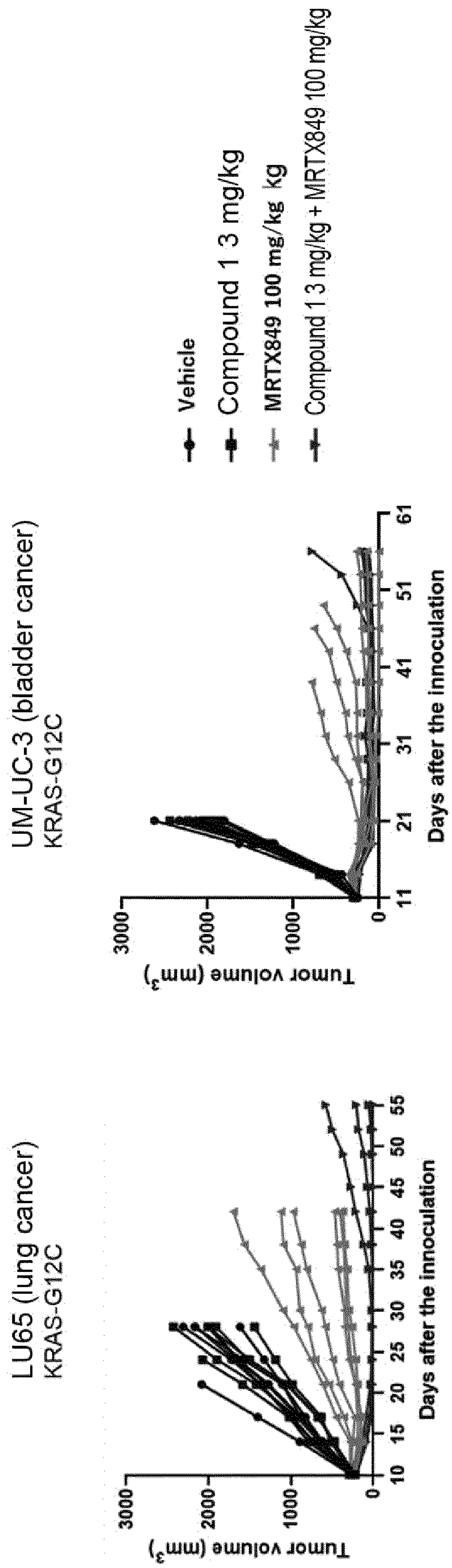
LU65 (lung cancer)
KRAS-G12C
Tumor volume (mm3)
3000
2000
1000
0
10
15
20
25
30
35
40
45
50
55
Days after the innoculation
UM-UC-3 (bladder cancer)
KRAS-G12C
Tumor volume (mm3)
3000
2000
1000
0
11
21
31
41
51
61
Days after the innoculation
Vehicle
Compound 1 3 mg/kg
MRTX849 100 mg/kg kg
Compound 1 3 mg/kg + MRTX849 100 mg/kg

**Fig.19**

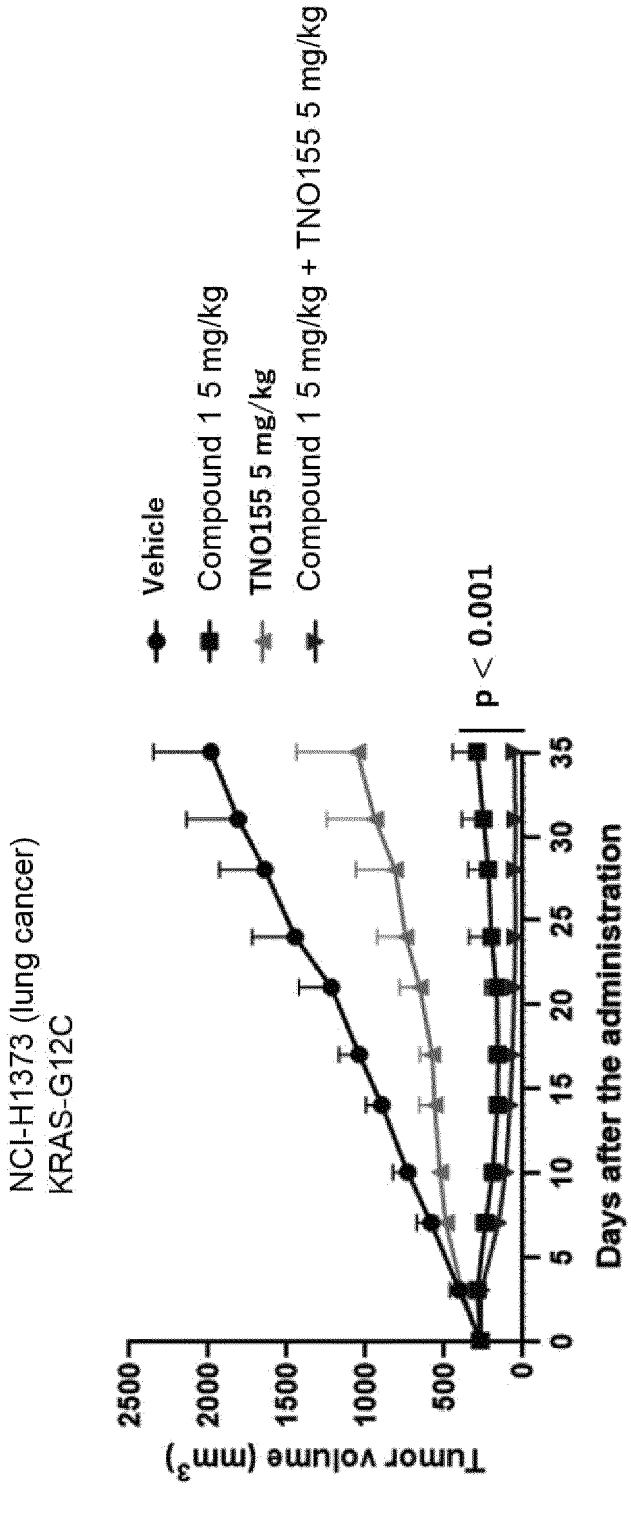
NCI-H1373 (lung cancer)
KRAS-G12C
Tumor volume (mm3)
2500
2000
1500
1000
500
0
0
5
10
15
20
25
30
35
Days after the administration
p < 0.001
Vehicle
Compound 1 5 mg/kg
TNO155 5 mg/kg
Compound 1 5 mg/kg + TNO155 5 mg/kg

Fig.20

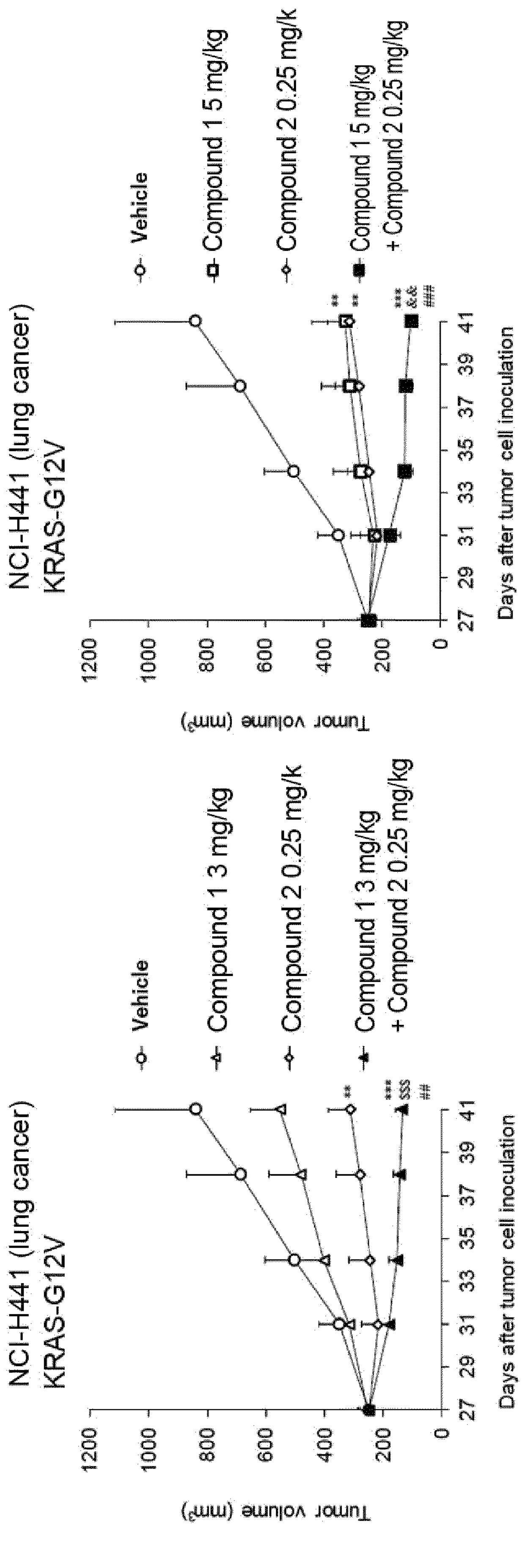
NCI-H441 (lung cancer)
KRAS-G12V
Tumor volume (mm³)
Days after tumor cell inoculation
Vehicle
Compound 1 3 mg/kg
Compound 2 0.25 mg/k
Compound 1 3 mg/kg
+ Compound 2 0.25 mg/kg
**
***
$$$
##
NCI-H441 (lung cancer)
KRAS-G12V
Tumor volume (mm³)
Days after tumor cell inoculation
Vehicle
Compound 1 5 mg/kg
Compound 2 0.25 mg/k
Compound 1 5 mg/kg
+ Compound 2 0.25 mg/kg
**
***
&&
###

*Fig.21*

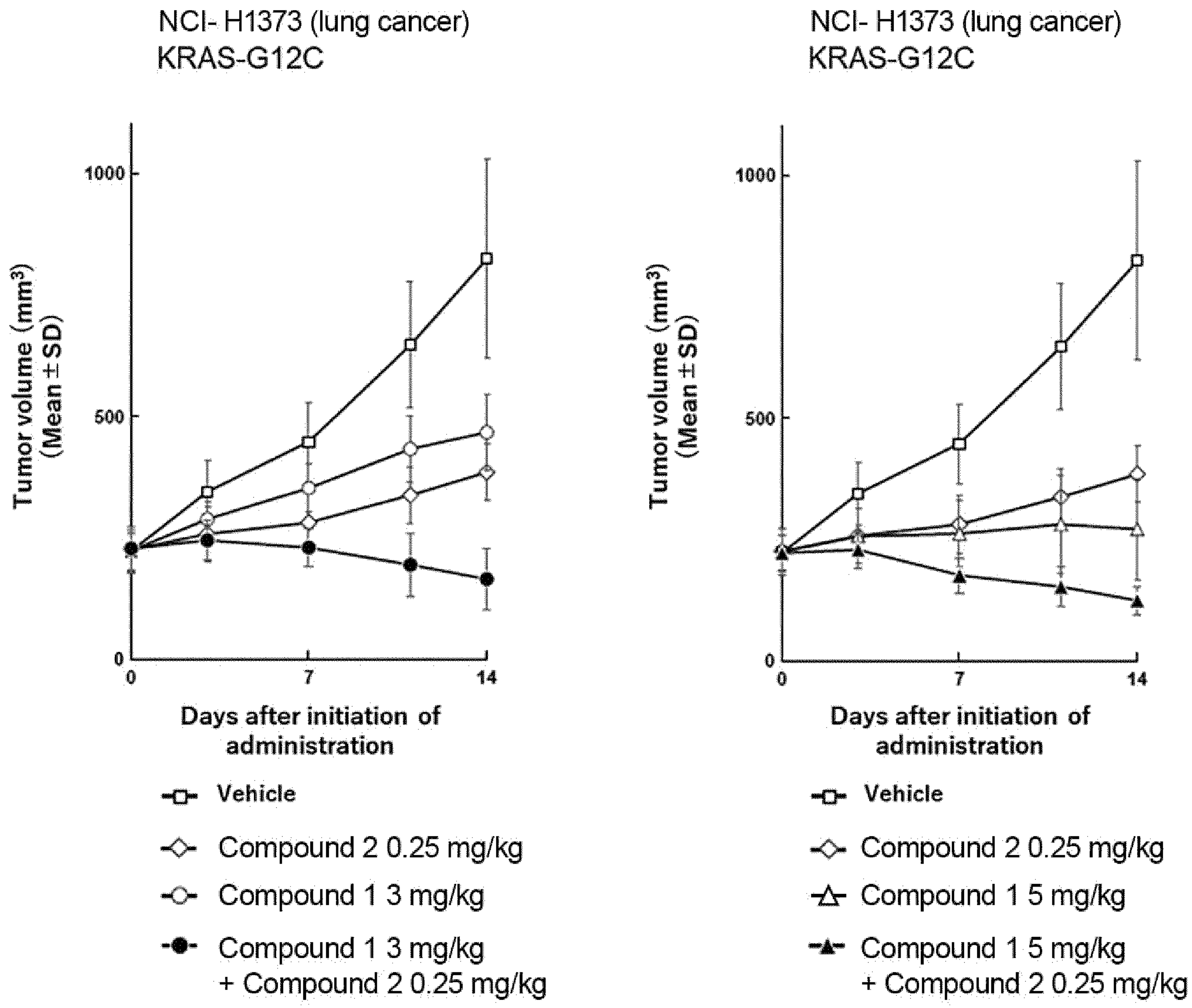
NCI- H1373 (lung cancer)
KRAS-G12C
Tumor volume (mm³)
(Mean ± SD)
1000
500
0
0
7
14
Days after initiation of administration
Vehicle
Compound 2 0.25 mg/kg
Compound 1 3 mg/kg
Compound 1 3 mg/kg + Compound 2 0.25 mg/kg
NCI- H1373 (lung cancer)
KRAS-G12C
Tumor volume (mm³)
(Mean ± SD)
1000
500
0
0
7
14
Days after initiation of administration
Vehicle
Compound 2 0.25 mg/kg
Compound 1 5 mg/kg
Compound 1 5 mg/kg + Compound 2 0.25 mg/kg

# Fig.22

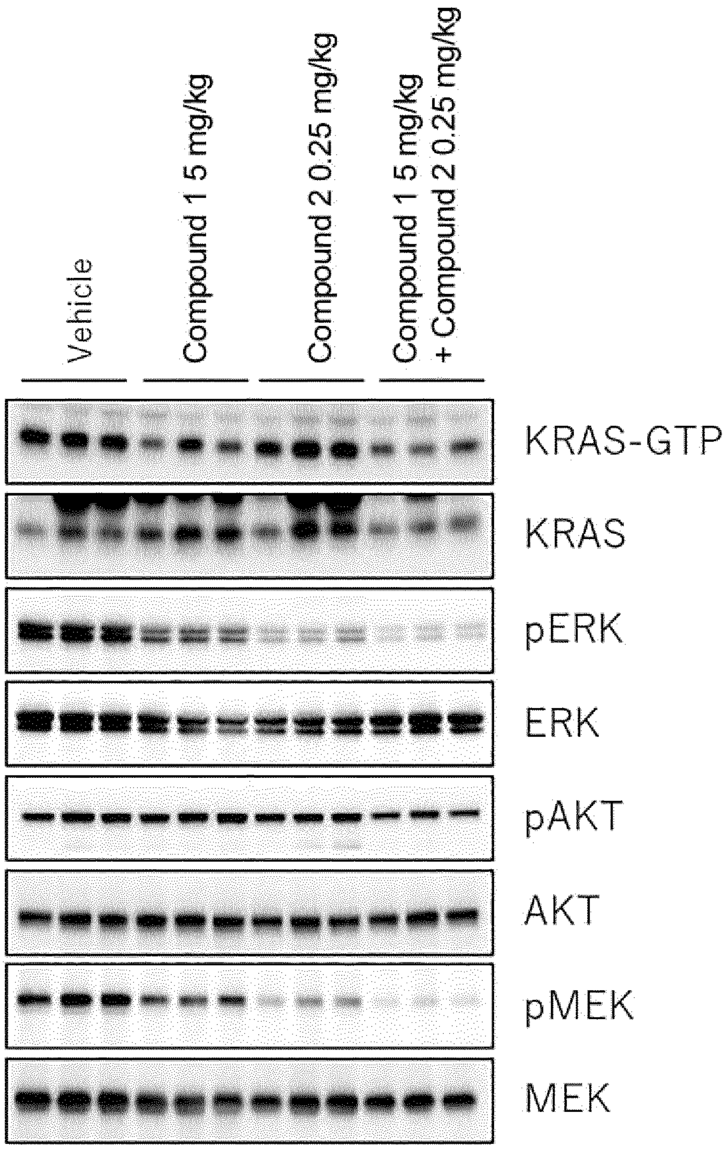
Vehicle
Compound 1 5 mg/kg
Compound 2 0.25 mg/kg
Compound 1 5 mg/kg
+ Compound 2 0.25 mg/kg
KRAS-GTP
KRAS
pERK
ERK
pAKT
AKT
pMEK
MEK

## INTERNATIONAL SEARCH REPORT

International application No.
**PCT/JP2023/001552**

**A. CLASSIFICATION OF SUBJECT MATTER**

***A61K 38/12***(2006.01)i; ***A61K 31/4184***(2006.01)i; ***A61K 31/44***(2006.01)i; ***A61K 31/47***(2006.01)i; ***A61K 31/497***(2006.01)i; ***A61K 31/506***(2006.01)i; ***A61K 31/517***(2006.01)i; ***A61K 31/519***(2006.01)i; ***A61K 31/5377***(2006.01)i; ***A61K 39/395***(2006.01)i; ***A61K 45/00***(2006.01)i; ***A61P 35/00***(2006.01)i; ***A61P 35/02***(2006.01)i; ***A61P 43/00***(2006.01)i; ***C07K 7/02***(2006.01)i; ***C07K 16/28***(2006.01)i

FI: A61K38/12; A61P35/00; A61P43/00 121; A61P35/02; A61K45/00; A61K31/5377; A61K31/517; A61K31/506; A61K39/395 E; A61K39/395 T; A61K39/395 M; A61K31/497; A61K31/47; A61K31/519; A61K31/4184; A61K31/44; C07K7/02; C07K16/28 ZNA

According to International Patent Classification (IPC) or to both national classification and IPC

**B. FIELDS SEARCHED**

Minimum documentation searched (classification system followed by classification symbols)

A61K38/12; A61K31/4184; A61K31/44; A61K31/47; A61K31/497; A61K31/506; A61K31/517; A61K31/519; A61K31/5377; A61K39/395; A61K45/00; A61P35/00; A61P35/02; A61P43/00; C07K7/02; C07K16/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2021/090855 A1 (CHUGAI SEIYAKU KABUSHIKI KAISHA) 14 May 2021 (2021-05-14)<br>claims, paragraphs [0013], [0014], examples, tables 23, 24 | 1-26 |
| Y | JP 2020-105162 A (AMGEN INC.) 09 July 2020 (2020-07-09)<br>claims, paragraphs [0313]-[0380], examples | 1-26 |
| Y | ZARREDAR, H. et al. Combination therapy with KRAS siRNA and EGFR inhibitor AZD8931 suppresses lung cancer cell growth in vitro. J Cell Physiol, 2019, vol. 234, pp. 1560-1566, ISSN 0021-9541<br>abstract | 1-26 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 April 2023** | **25 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/001552**

**C. DOCUMENTS CONSIDERED TO BE RELEVANT**

| Category* | Citation of document, with indication, where appropriate, of the relevant passages | Relevant to claim No. |
|---|---|---|
| Y | TABERNERO, J. et al. KRYSTAL-10: A randomized phase 3 study of adagrasib (MRTX849) in combination with cetuximab vs chemotherapy in patients with previously treated advanced colorectal cancer with KRASG12C mutation. Ann Oncol, 2021, vol. 32, no. S3, p. S121, ISSN 0923-7534<br>column P-71 | 1-26 |
| Y | WO 2020/165732 A1 (NOVARTIS AG) 20 August 2020 (2020-08-20)<br>claims, examples | 1-26 |
| Y | LIU, C. et al. Combinations with Allosteric SHP2 Inhibitor TNO155 to Block Receptor Tyrosine Kinase Signaling. Clin Cancer Res, 2021, vol. 27, no. 1, pp. 342-354, ISSN 1078-0432<br>abstract | 1-26 |
| Y | JACQUELINE, A. et al. SHP2 inhibition as the backbone of targeted therapy combinations for the treatment of cancers driven by oncogenic mutations in the RAS pathway. Cacer Res, 2020, vol. 80, no. 16, Suppl., abstract no. 1943, ISSN 1078-0432<br>abstract | 1-26 |
| Y | WO 2020/205473 A1 (DECERNA PHARMACEUTICALS, INC.) 08 October 2020 (2020-10-08)<br>claims, examples | 1-26 |

Form PCT/ISA/210 (second sheet) (January 2015)

**INTERNATIONAL SEARCH REPORT**

International application No.
**PCT/JP2023/001552**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

“’Annex C/ST.25 text file format” should be read as ‘ST.26 format’”

Form PCT/ISA/210 (continuation of first sheet) (January 2015)

**INTERNATIONAL SEARCH REPORT**
**Information on patent family members**

International application No.
**PCT/JP2023/001552**

| Patent document cited in search report | Publication date (day/month/year) | Patent family member(s) | Publication date (day/month/year) |
|---|---|---|---|
| WO 2021/090855 A1 | 14 May 2021 | EP 4043478 A1<br>claims, paragraphs [0020], [0021], examples, tables 23, 24<br>KR 10-2021-0064234 A<br>KR 10-2022-0081327 A<br>CN 114729006 A<br>JP 2021-119176 A | |
| JP 2020-105162 A | 09 July 2020 | US 2020/0222407 A1<br>claims, paragraphs [0400]-[0464], examples<br>WO 2020/106647 A2<br>EP 3883579 A2<br>CN 113038953 A<br>KR 10-2021-0094570 A | |
| WO 2020/165732 A1 | 20 August 2020 | JP 2022-520079 A<br>claims, examples<br>US 2022/0152026 A1<br>EP 3924053 A1<br>CN 113382774 A | |
| WO 2020/205473 A1 | 08 October 2020 | JP 2022-527108 A<br>claims, examples<br>US 2022/0154189 A1<br>EP 3947681 A1<br>KR 10-2021-0145213 A<br>CN 113924365 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018076369 A
- JP 2021063014 A
- WO 2021090855 A
- RO 4987655
- WO 2021149776 A


### Non-patent literature cited in the description


- *CHEMICAL ABSTRACTS*, 184475-35-2
- *CHEMICAL ABSTRACTS*, 183321-74-6
- *CHEMICAL ABSTRACTS*, 439081-18-2
- *CHEMICAL ABSTRACTS*, 1421373-65-0
- *CHEMICAL ABSTRACTS*, 231277-92-2
- *CHEMICAL ABSTRACTS*, 2172651-73-7, 3
- *CHEMICAL ABSTRACTS*, 1801765-04-7
- *CHEMICAL ABSTRACTS*, 2668298-71-1
- *CHEMICAL ABSTRACTS*, 1801747-42-1
- *CHEMICAL ABSTRACTS*, 56990-57-9
- *CHEMICAL ABSTRACTS*, 2296729-00-3
- *CHEMICAL ABSTRACTS*, 2326521-71-3
- *CHEMICAL ABSTRACTS*, 871700-17-3
- *CHEMICAL ABSTRACTS*, 2677856-11-8
- *CHEMICAL ABSTRACTS*, 874101-00-5
- *CHEMICAL ABSTRACTS*, 606143-52-6
- *CHEMICAL ABSTRACTS*, 284461-73-0
- *CHEMICAL ABSTRACTS*, 444731-52-6
- *CHEMICAL ABSTRACTS*, 557795-19-4
- *CHEMICAL ABSTRACTS*, 319460-85-0
- *CHEMICAL ABSTRACTS*, 755037-03-7
- *Cancer Metastasis Rev*, 2013, vol. 32, 147-162
- *Cancer Discov.*, 2016, vol. 6, 316-329
- *Nat. Rev. Drug Discov.*, 2020, vol. 19, 533-552
- *Molecular Cytogenetics*, 2015, vol. 8, 103